(19) Europäisches Patentamt
European Patent Office
Office européen des brevets

(11) EP 2 327 978 A2

(12) EUROPEAN PATENT APPLICATION

(43) Date of publication:
01.06.2011 Bulletin 2011/22

(51) Int Cl.:
G01N 21/47 (2006.01)
G01N 21/65 (2006.01)
A61B 5/00 (2006.01)

(21) Application number: 10183925.6

(22) Date of filing: 04.04.2003

(84) Designated Contracting States:
DE FR GB

(30) Priority: 05.04.2002 US 370197 P
21.06.2002 US 178062

(62) Document number(s) of the earlier application(s) in accordance with Art. 76 EPC:
03723896.1 / 1 495 309

(71) Applicant: Massachusetts Institute of Technology
Cambridge, MA 02139 (US)

(72) Inventors:
• Motz, Jason T.
Cambridge, MA 02140 (US)
• Galindo, Luis H.
Fitchburg, MA 01420 (US)
• Hunter, Martin
Bradford, Massachusetts 01835 (US)
• Haka, Abigail
Astoria, New Youk 11105 (US)
• Gandhi, Saumil
New York, NY 10025 (US)
• Dasari, Ramachandra
Shererville, Indiana 46375 (US)
• Feld, Michael S.
deceased (US)

(74) Representative: Greenwood, John David et al
Graham Watt & Co LLP
St Botolph's House
7-9 St Botolph's Road
Sevenoaks
Kent TN13 3AJ (GB)

Remarks:
This application was filed on 30-09-2010 as a divisional application to the application mentioned under INID code 62.

(54) Systems and methods for spectroscopy of biological tissue

(57) A probe (70) for measuring tissue comprising a fiber optic probe having a proximal end and a distal end, a delivery optical fiber (76) in the probe that delivers light onto tissue, a collection optical fiber (72) in the probe that collects Raman scattered radiation from tissue and an optical system on the distal end of the probe, the optical system being optically coupled to a distal end of the delivery optical fiber (76) and a distal end of the collection optical fiber (72)r.

Figure 4A

EP 2 327 978 A2

**Description**

GOVERNMENT SUPPORT

This invention was supported, in whole or in part, by grants P41-RR-02594 and R01-HL-64675 from the National Institute of Health. The Government has certain rights in the invention.

CROSS REFERENCES TO RELATED APPLICATIONS

The present application is a continuation-in-part of U.S. Continuation-in-Part Patent Application No. 10/178,062, filed June 21, 2002 which claims the benefit of U.S. Provisional Patent Application No. 60/370,197, filed April 5, 2002. The entire contents of the above applications are incorporated herein by reference in their entirety.

BACKGROUND OF THE INVENTION

Optical methods are increasingly being used for the detection of disease. Near-infrared Raman spectroscopy in particular, because of its chemical specificity, is proving to be a useful tool for both disease diagnosis and the study of disease progression. Over the past decade Raman spectroscopy has been applied to many diseases and biological problems and there have been many advances in-vitro. More recently there have been reports of in-vivo work that however have either been confined to studies of skin or other easily accessible organs, or have used optical fiber configurations that require collection times that are unreasonably long for practical clinical use. The majority of applications require remote sampling via optical fibers, and the size of the probe and fiber bundle is strictly limited by the application. A particular example that current commercial systems cannot provide is the ability to evaluate atherosclerotic lesions in-vivo in real-time, through an angiographic catheter, thus aiding cardiologists in directing the most appropriate treatment in each individual case. These objectives have not been fulfilled by current systems.

In addition, prior art probes for remote Raman sensing, using several different methods for filtering out the fiber spectral background, either exhibit extremely low optical throughput or are too bulky to be used intravascularly. A problem with the prior art designs includes having a 4 cm long stiff tip that prohibits their incorporation into transcutaneous catheters for accessing the coronary arteries. Secondly, in data collected with these probes, a considerable component of the fiber Raman spectrum still remains. Further, data collection times on the order of 30 seconds or longer are typically required for collection of signals with an acceptable signal to noise ratio (SNR).

A need still exists for improved systems and methods which include probes for, for example, Raman spectroscopy that are sized for applications in medicine and provide an improved spectral signature from tissue.

SUMMARY OF THE INVENTION

The system and method of the present invention relates to using spectroscopy, for example, Raman spectroscopic methods for diagnosis of tissue conditions such as vascular disease or cancer. The system and methods of the present invention have several applications: optical breast biopsies and breast analysis through ductoscopy, percutaneous blood analysis and monitoring, vascular stenosis, gastrointestinal cancer evaluation, scanning for dysplasia in the pancreatic duct and skin analyses.

In accordance with a preferred embodiment of the present invention, a system for measuring tissue includes a fiber optic probe having a proximal end, a distal end, and a diameter of 2 mm or less. This small diameter allows the system to be used for the diagnosis of coronary artery disease or other small lumens or soft tissue with minimal trauma. A delivery optical fiber (or fibers) is included in the probe coupled at the proximal end to a light source. A filter for the delivery fibers is included at the distal end. The system includes a collection optical fiber (or fibers) in the probe that collects Raman scattered radiation from tissue, the collection optical fiber is coupled at the proximal end to a detector. A second filter is disposed at the distal end of the collection fibers. An optical lens system is disposed at the distal end of the probe including a delivery waveguide coupled to the delivery fiber, a collection waveguide coupled to the collection fiber and a lens.

The delivery waveguide comprises a rod and the collection waveguide comprises a cylindrical tube, the tube being concentric about the rod. In an alternate preferred embodiment, the delivery waveguide comprises a first tube and the collection waveguide comprises a second cylindrical tube, the second tube being concentric about the first tube. Further the lens includes a ball lens optically coupled to the delivery fiber and the collection fiber.

In a preferred embodiment, the probe further comprises a sleeve that optically isolates the delivery waveguide from the collection waveguide. The sleeve can be metallic, such as palladium, silver or gold. The glass rod tube and sleeve can be attached together with an adhesive. An outer retaining sleeve can attach the distal optics to the fiber optics.

The probe further comprises a first plurality of collection fibers arranged concentrically about the delivery fiber at a first radius, and a second plurality of collection fibers arranged concentrically about the delivery fiber at a second radius that is larger than the first radius.

In accordance with another aspect of the present invention, the probe includes a controller that gates a collection time, the collection time being less than 2 seconds. In one embodiment, the optical lens system has a length less than 10 mm. In a preferred embodiment, the optical lens system has a length of less than 4 mm. The diameter of the distal optical system is preferably in the range of 1-2 mm. The optical lens systems delivers and collects radiation in a radial direction, which can be defined as any off-axis direction. The light source has a wavelength longer than 750 nm with a preferred embodiment using an argon laser pumped Ti : sapphire laser emitting at 830 nm. In an alternate embodiment a diode laser such as a InGaAs laser emitting at 785 nm or 830 nm may be used.

In a preferred embodiment, the radial Raman probe in accordance with the present invention for use in diagnosing atherosclerosis is incorporated in a catheter of the type used for angiography, for example. It includes a balloon for displacing blood and other fluids and to position the catheter in the artery. A preferred embodiment includes a channel for balloon inflation. Further, the catheter system includes the capability for flushing away the blood temporarily with a fluid, for example, saline. One or several optical fibers can be configured so as to direct excitation light in a radial direction, either to the side or at an angle ranging from 45˚ - 90˚. In such a preferred embodiment a balloon disposed on the side is used to contact the fibers adjacent the artery wall, and displace blood or other intervening fluids.

Alternately, the delivery fibers can be arranged to direct light in a circular pattern at an angle to the axis of the probe. The different collection fibers collect light simultaneously from different portions of the circumferential region illuminated. In this embodiment, the probe is enclosed in an inflatable balloon which is inflated before light delivery and/or collection to displace blood and other fluids. In preferred embodiments, the balloon is of a type used in arterial applications, such as, for example, angioplasty, and are made of thin material so as to allow excitation light to pass through to the artery wall, and return Raman light generated in the artery wall to pass through the balloon to the collection fibers.

The present invention includes the diagnostic classification of atherosclerotic plaques in human coronary arteries by quantitative assessment of their morphologic composition using Raman spectroscopy. The rapid and nondestructive nature of Raman spectroscopy provides the opportunity to diagnose coronary artery plaques in-vivo, when applied in a clinical setting using optical fiber technology. So used, the preferred embodiments of the present invention classify an atherosclerotic lesion, and can provide in-vivo quantitative assessment of its morphologic features, such as the presence of foam cells (FC), necrotic core (NC), and cholesterol crystals (CC), which may be used to assess plaque instability and the extent of disease progression, and thereby, the risk of life-threatening complications such as thrombosis and acute plaque hemorrhage. So used, the methods of the present invention may provide insight into as yet poorly understood dynamics in the evolution of atherosclerotic lesions and the effects of lipid-lowering and other therapies.

Chemical composition and morphology, rather than anatomy (degree of stenosis), determine atherosclerotic plaque instability and predict disease progression. In a preferred embodiment, a modification of the Raman spectroscopy reference data can also be used to identify the microscopic morphologic structures comprising the plaque, and the pathological state of the artery can be accurately assessed using a diagnostic algorithm based on the relative contribution of these microscopic morphological structures to the macroscopic arterial Raman spectrum.

In a preferred embodiment eight atherosclerotic classes are used for comparison with previous studies using the principal component analysis (PCA) and chemical reference data. These eight classes are reduced to three classes. On pathologic examination, the presence of FC, NC, and CC are significant predictors of plaque instability and disease progression. The embodiments of the present invention show that Raman spectroscopic analysis of these same morphologic structures can be used to diagnose atherosclerotic lesions in intact coronary arteries, without the need for microscopic examination. This suggests that Raman spectroscopy can provide not only quantitative chemical information, but also quantitative morphologic information regarding atherosclerotic lesion composition, such as the presence of CC, not readily available in current diagnostic imaging techniques such as intravascular ultrasound (IVUS), magnetic resonance imaging (MRI), and angiography.

In a preferred embodiment, the spectral signatures of the cellular and extracellular morphologic components of normal and atherosclerotic arterial tissue in-situ are determined using confocal Raman microspectroscopy. The specific morphologic structures are selected because of their role in normal arterial anatomy (e.g. elastic laminae) and/or atherosclerotic plaque formation (e.g. foam cells, necrotic core, cholesterol crystals). Least-squares minimization of a linear combination of the basis spectra of 12 biochemical components provide information on the biochemical composition of the various morphologic structures. These biochemical components are selected because they were known to be present in high concentration in normal arterial tissue and/or atherosclerotic plaque (e.g. collagen, elastin, and free and esterified cholesterol) or because they are strong Raman scatterers (e.g. β-carotene). Glycosaminoglycans (e.g. hyaluronic acid, chondroitin sulfate, dermatan sulfate, and heparan sulfate), which may contribute 3% of artery dry mass, did not contribute significantly to the biochemical model and reference data fits, most likely because they are weak Raman scatterers (i.e. they have small Raman cross sections), and were excluded from the reference data.

The embodiments of the present invention interpret Raman spectra in terms of morphology. For example, the Raman spectra can be associated with a morphological structure, for example, a foam cell which can be associated with specific chemical compounds. Further, the number of spectra can be reduced, for example, from a large number of chemical spectra to only eight unique spectra associated with morphological structures thereby decreasing the error in the fit. The diagnostics that are available to identify and monitor vulnerable plaque using the optical fiber catheter system of the present invention include the use of chemical composition, information about the morphological structures, thickening of the intimal layer and the thinning of the overlying collagen layer. Preferred embodiments include the determination of the depth .of collagen by measuring the percentage of collagen. Further, the presence of calcification is monitored and any edges are identified and located relative to the collagen as indicators of a potential rupture and blood clot. Further, the reduced fractional fit contributions of collagen fibers in non-calcified plaques is an indication of decreased plaque stability.

The foregoing and other features and advantages of the systems and methods for spectroscopy of in-vivo biological tissue will be apparent from the following more particular description of preferred embodiments of the system and method as illustrated in the accompanying drawings in which like reference characters refer to the same parts throughout the different views. The drawings are not necessarily to scale, emphasis instead being placed upon illustrating the principles of the invention.

BRIEF DESCRIPTION OF THE DRAWINGS

Figure 1A graphically illustrates a comparison of theory, simulations and results for Raman emission data of turbid samples of blood tissue for the radial distribution of the Raman scattered light in accordance with a preferred embodiment of the present invention.
Figure 1B graphically illustrates a comparison of simulations, theory and results for Raman emission data of turbid samples of blood tissue for angular distribution of the Raman scattered light in accordance with a preferred embodiment of the present invention.
Figures 2A-2C are graphical representations of morphological reference data of coronary arteries for a normal coronary artery, non-calcified plaque and calcified plaque, respectively in accordance with a preferred embodiment of the present invention.
Figure 3 is a graphical illustration of Raman morphometry of a coronary artery in accordance with a preferred embodiment of the present invention.
Figure 4A is a longitudinal view of an apparatus including a probe for measuring tissue in accordance with a preferred embodiment of the present invention.
Figure 4B is a transverse view of the probe illustrated in Figure 4A in accordance with a preferred embodiment of the present invention.
Figures 4C and 4D are a longitudinal and transverse view respectively of an alternate embodiment of a probe for measuring tissue with a paraboloidal mirror in accordance with the system of the present invention.
Figure 5 graphically illustrates the transmission characteristics of the excitation and collection fibers incorporating filters with respect to the Raman shift in accordance with a preferred embodiment of the present invention.
Figure 6 graphically illustrates the Raman spectrum of a non-calcified artheroselerotic plaque collected in 1 second with 100 mW excitation power in accordance with a preferred embodiment of the present invention.
Figure 7 graphically illustrates the Raman spectrum of a normal artery in accordance with an in-vitro system preferred embodiment of the present invention.
Figure 8 is a schematic diagram illustrating a system for measuring tissue in accordance with a preferred embodiment of the present invention.
Figure 9 illustrates the excitation light diffusing through tissue in accordance with a preferred embodiment of the present invention.
Figures 10A-10C are graphical representations of the integrated radial distributions, integrated angular distributions and optimized collection efficiency for blood tissue, respectively, in accordance with a preferred embodiment of the present invention.
Figure 11 is a graphical representation of an excitation spot size in accordance with a preferred embodiment of the present invention.
Figure 12 is an illustration of a ray diagram of the distribution of excitation light in tissue in accordance with a preferred embodiment of the present invention.
Figure 13 is an illustration of a ray diagram of the collection efficiency of a probe in accordance with a preferred embodiment of the present invention.
Figure 14 graphically illustrates the collection efficiency of the probe in accordance with a preferred embodiment of the present invention.
Figure 15 is partially sectioned view illustrating a portion of a coronary artery showing a probe in accordance with a preferred embodiment of the present invention.

Figure 16 illustrates a signal from a ball lens as a function of laser power in accordance with a preferred embodiment of the present invention.

Figure 17 graphically illustrates a comparison of data as collected using a probe and an experimental system of a normal aorta in accordance with a preferred embodiment of the present invention.

Figure 18 graphically illustrates a Raman spectrum of normal breast issue examined with a probe in accordance with a preferred embodiment of the present invention.

Figure 19 graphically illustrates a comparison of Raman spectra of a malignant breast tumor as collected using a probe in accordance with a preferred embodiment of the present invention and as predicted by reference data of the present invention.

Figure 20 graphically illustrates a comparison of morphological reference data to calcified aorta data collected with a probe in accordance with a preferred embodiment of the present invention. '

Figures 21A-C illustrate longitudinal views of alternate preferred embodiments of side-viewing probes for measuring tissue in accordance with a system of the present invention.

Figure 21D illustrates a view of a preferred embodiment of a side-viewing probe delivering light imaged onto a portion of tissue and Raman light collected from the tissue in accordance with a preferred embodiment of the present invention.

Figure 22 is a schematic illustration of a combined Raman macrospectroscopy and confocal microspectroscopy system in accordance with a preferred embodiment of the present invention.

Figure 23 graphically illustrates the Raman spectra of eight selected coronary artery morphological structures in accordance with a preferred embodiment of the present invention.

Figures 24A-24C graphically illustrate the results of the fit contribution of seven morphologic structures to the calibration data set and the diagnostic algorithm classification wherein Figure 24A illustrates nonatherosclerotic tissue, Figure 24B illustrates noncalcified atherosclerotic plaque and Figure 24C illustrates calcified atherosclerotic plaque in accordance with a preferred embodiment of the present invention.

Figure 25 illustrates the spectral contribution of $\beta$-carotene in a calibration data set in relation to three diagnostic categories, wherein the carotenoid level is expressed in arbitrary units in accordance with a preferred embodiment of the present invention.

Figures 26A and 26B graphically illustrate the results of the algorithm developed with an initial calibration data set and the results of the prospective validation data set, respectively, in accordance with a preferred embodiment of the present invention.

Figure 27 is a schematic diagram of a system including a confocal Raman microspectrometer in accordance with a preferred embodiment of the present invention.

Figures 28A and 28B are a photomicrograph of internal elastic lamina in a 6-$\mu$m unstained coronary artery section viewed under phase contrast and the Raman spectrum of the internal elastic lamina; respectively, in accordance with a preferred embodiment of the present invention.

Figures 29A and 29B are a photomicrograph of the tunica adventitia with collagen fibers in a 6-$\mu$m unstained coronary artery section viewed under phase contrast and the Raman spectrum of the fibers, respectively, in accordance with a preferred embodiment of the present invention.

Figure 30 graphically illustrates the Raman spectra of four different smooth muscle cells in the tunica media in accordance with a preferred embodiment of the present invention.

Figure 31 graphically illustrates the Raman spectra of four fat cells (adipocytes) in the tunica adventitia in accordance with a preferred embodiment of the present invention.

Figures 32A and 32B are a photomicrograph of foam cells in an intimal athersclerotic plaque in a 6-$\mu$m unstained coronary artery section viewed under phase contrast and a Raman spectra of the foam cells and necrotic core, respectively, in accordance with a preferred embodiment of the present invention.

Figure 33 graphically illustrates the Raman spectra of cholesterol crystals in intimal atherosclerotic plaques in accordance with a preferred embodiment of the present invention.

Figures 34A and 34B are a photomicrograph of the calcification in the necrotic core of an intimal atherosclerotic plaque in a 6-$\mu$m unstained coronary artery section viewed under phase contrast and the corresponding Raman spectra in accordance with a preferred embodiment of the present invention.

Figure 35 graphically illustrates a Raman basis spectra of the 12 biochemicals used for linear fitting to the morphologic spectra in accordance with a preferred embodiment of the present invention.

Figures 36A-36H provide a graphical comparison between observed data and reference data of spectra of the different morphological structures in the coronary artery in accordance with a preferred embodiment of the present invention.

Figures 37A-37H graphically illustrate the biochemical composition of each morphologic structure in accordance with a preferred embodiment of the present invention.

Figure 38A graphically illustrates the results for Raman emission data of turbid samples of artery tissue for the radial distribution of the Raman scattered light in accordance with a preferred embodiment of the present invention.

Figure 38B graphically illustrates the results for Raman emission data of turbid samples of artery tissue for angular distribution of the Raman scattered light in accordance with a preferred embodiment of the present invention.

Figures 38C-38E are graphical representations of integrated radial distributions, integrated angular distributions and optimized collection efficiency of artery tissue, respectively, in accordance with a preferred embodiment of the present invention.

Figures 39A-39C characterize the spatial distribution of Raman light emitted from normal aorta wherein Figure 39B shows the measured (circles) discrete radial distribution $B_1(r)$ and a multi-Gaussian fit (line) to the data, as a function of distance from the excitation beam, the radial collection efficiency ($\eta_1(r)$, circles) is plotted in Figure 39C, along with a least-squares fit (line) confirming a Gaussian profile in accordance with a preferred embodiment of the present invention.

Figures 40A and 40B characterize the integrated angular distribution ($\eta_2(\theta)$, (circles) illustrated in Figure 40B, and the theoretical $\sin^2(\theta)$ distribution for a Lambertian source (line), wherein theory and experiment agree well for the range of angles measured in accordance with a preferred embodiment of the present invention.

Figure 41 illustrates the efficiency of the Raman probes in accordance with a preferred embodiment of the present invention wherein the angular (dashed line) and radial (thin line) collection efficiencies as a function of radius are shown and the product of these curves is the total collection efficiency $\eta_T(r)$ (thick line).

Figure 42 illustrates the simulation results of the Raman probe excitation spot size wherein a slight focusing occurs 1mm from the ball lens with no scattering (circles and solid line), but an immediate divergence occurs when the probe is in contact with a scattering medium (squares and dashed line) in accordance with a preferred embodiment of the present invention.

Figure 43 illustrates a schematic diagram of the Raman spectroscopy system used for experimental testing of the Raman probe in accordance with a preferred embodiment of the present invention.

Figure 44 illustrates the Raman spectrum of $BaSO_4$ collected with the single-ring probe demonstrating the efficiency of the filter module in accordance with a preferred embodiment of the present invention wherein there is minimal evidence of fiber background in this spectrum.

Figure 45 illustrates the results of the tissue phantom studies showing signal collection as a function of transport length wherein the intensity of the perchlorate signal of interest (circles and solid lines) are plotted along with the fiber background (squares with dashed lines) lines of constant absorption are drawn to demonstrate the effects of signal collection with increased scattering in accordance with a preferred embodiment of the present invention.

Figures 46A and 46B illustrate the comparison of traditional open-air optics Raman system with the Raman probe, wherein the raw data is shown in Figure 46A, demonstrating slightly increased collection from the Raman probe along with the remaining fiber background, removal of fiber background and tissue fluorescence results in identical spectra Figure 46B except for the peaks at 750 cm$^{-1}$ just below 1600 cm$^{-1}$ atom probe tip components in accordance with a preferred embodiment of the present invention.

Figures 47A and 47B illustrate the Raman spectra of normal breast tissue and a malignant breast tumor, respectively, wherein data is shown as dots with the corresponding model fit (line), and the residual is plotted below on the same scale.

Figures 48A and 48B illustrate a clinical probe having a total diameter of less than 3 mm in accordance with a preferred embodiment of the present invention.

Figures 49A and 49B illustrate clinical data for the normal artery, intimal fibroplasia, wherein Figure 49A is the Raman spectra acquired and Figure 49B illustrates the corresponding histology in accordance with a preferred embodiment of the present invention.

Figures 50A-50C illustrate clinical data for atheromatous plaque wherein Figure 50A illustrates the Raman spectra and Figures 50B and 50C the corresponding histology in accordance with a preferred embodiment of the present invention.

Figures 51A and 51B illustrate clinical data acquired for calcified plaque wherein Figure 51A illustrates the Raman spectra and Figure 51B the corresponding histology in accordance with a preferred embodiment of the present invention.

Figures 52A-52C illustrate clinical data acquired for ruptured plaque wherein Figure 52A illustrates the Raman spectra and Figures 52B and 52C the corresponding histology in accordance with a preferred embodiment of the present invention.

Figures 53A-53C illustrate clinical data acquired for calcified plaque with thrombus wherein Figure 53A illustrates the Raman spectra and Figures 53B and 53C the corresponding histology in accordance with a preferred embodiment of the present invention.

Figure 54 illustrates a diagram of a side-viewing probe in accordance with a preferred embodiment of the present invention.

Figures 55A-55C illustrate the effects of blood on signal collection, wherein the figures illustrate the spectra of artery with no blood, artery with blood and blood alone, respectively, in accordance with a preferred embodiment of the present invention.

Figure 56 illustrates a schematic diagram of an endoscopic system having a Raman probe in accordance with a preferred embodiment of the present invention.

Figure 57 is a flow chart illustrating the methods to acquire data for in vivo Raman spectral diagnosis in accordance with a preferred embodiment of the present invention.

Figure 58 is a flow chart illustrating the processing of the data used in a real-time analysis Raman system in accordance with a preferred embodiment of the present invention.

Figures 59A-59D illustrate Raman images of normal breast duct [(A)-(C)] with corresponding serial stained section (D). Each image represents the contribution of a specific morphological element to the region being studied. (A) collagen; (B) cell cytoplasm; (C) cell nucleus in accordance with a preferred embodiment of the present invention.

Figure 60 illustrates the basis spectra used in the morphological model of the breast. (A) cell cytoplasm; (B) cell nucleus; (C) fat; (D) β-carotene; (E) collagen; (F) calcium hydroxyapatite; (G) calcium oxalate; (H) cholesterol-like; (I) water in accordance with a preferred embodiment of the present invention.

Figure 61 illustrates the Raman spectra of four types of cells observed in normal or diseased human breast tissue. (A) fibroblast (normal stroma); (B) epithelial cell (fibrocystic disease); (C) epithelial cell (normal duct); (D) malignant cell in accordance with a preferred embodiment of the present invention.

Figure 62 illustrates comparison of commercially available [(A), (C)] and morphologically derived [(B), (D)] Raman spectra observed in cells. (A) DNA (Sigma); (D) cell nucleus (breast tissue); (C) actin (Sigma); (D) cell cytoplasm (breast tissue) in accordance with a preferred embodiment of the present invention.

Figure 63 illustrates comparison of (A) purified collagen and (B) morphologically derived collagen in accordance with a preferred embodiment of the present invention.

Figure 64 illustrates comparison of (A) purified triolein and (B) morphologically derived fat in accordance with a preferred embodiment of the present invention.

Figure 65 illustrates the spectrum of necrotic core ('cholesterol-like') fitted with a cell cytoplasm, cell nucleus, fat, cholesterol linoleaate and cholesterol in accordance with a preferred embodiment of the present invention.

Figure 66 illustrates the spectra of breast deposits. (A) calcium oxalate dehydrate; (B) calcium hydroxyapatite; (C) β-carotene in accordance with a preferred embodiment of the present invention.

Figure 67 illustrates Raman spectra collected from the extracellular matrix of five patients in accordance with a preferred embodiment of the present invention.

Figures 68A-C illustrate the quality of the model's fit to macroscopic breast tissue samples: normal (•) fit with model (-), fibrosis and adenosis. Below each spectrum is plotted the residual of the fit (with the zero line drawn). The percentages given at the side represent the fit coefficients of the basis spectra, normalized to sum to one (fit coefficient of water is not included in summation) in accordance with a preferred embodiment of the present invention.

Figures 69A-C further demonstrate the quality of the model's fit to macroscopic breast tissue samples: fibrosis + cysts (•) fit with model (-), fibroadenoma and infiltrating ductal carcinoma (residual plotted below) in accordance with a preferred embodiment of the present invention.

Figure 70 is a schematic representation of the confocal Raman microscopy instrumentation system in accordance with a preferred embodiment of the present invention.

Figures 71A-C illustrate a, specimen radiograph and b, phase contrast image taken from a section of the same sample. c, Raman spectrum of a type I calcification arising in association with secretions in the lumen of a duct cyst in a focus of fibrocystic disease in accordance with a preferred embodiment of the present invention. The region from which the Raman spectrum was acquired is highlighted by a box.

Figures 72A-72C illustrate a, specimen radiograph, and b, phase contrast image taken from a section of the same sample. c. Raman spectrum of a type II calcification in a malignant breast lesion in accordance with a preferred embodiment of the present invention. The region from which the Raman spectrum was acquired is highlighted by a box.

Figure 73 illustrates the results of a diagnostic algorithm for type II microcalcifications based on the scores of three PCs (▲, benign; ○, malignant) in accordance with a preferred embodiment of the present invention.

Figure 74 illustrates the ROC curve, which illustrates the ability of Raman spectroscopy to separate microcalcifications occurring in benign and malignant breast lesions. A simulated ROC curve of two indistinguishable populations, represented by the dashed line, is included for comparison in accordance with a preferred embodiment of the present invention.

Figures 75A and 75B illustrate a, PC spectrum 5. b, PC spectrum 5 (solid line) overlaid with the mean spectrum from all type II microcalcifications (dotted line) to illustrate broadening of the 960 cm$^{-1}$ peak (arrow) in accordance with a preferred embodiment of the present invention.

Figure 76 illustrates the PC spectrum 2 exhibiting a large, second derivative-like feature around 960 cm$^{-1}$ (arrow)

in accordance with a preferred embodiment of the present invention.

Figure 77 illustrates the PC spectrum 3 exhibiting positively directed protein features such as the peak at 1445 cm$^{-1}$, the Amide I vibration at 1650 cm$^{-1}$, and the phenylalanine feature at 1004 cm$^{-1}$ (arrows) in accordance with a preferred embodiment of the present invention.

Figures 78A-78G illustrate Raman images (A-E) of HT29 cells with corresponding phase contrast image (F). Raman spectra are fit with phosphatidyl choline (A), DNA (B), cholesterol linoleate (C), triolein (D), and morphologically-derived cell cytoplasm (E) spectra to produce chemical maps of the cells. G shows the spectrum (•) acquired from within the box indicated in image E along with the corresponding fit (-) and residual (below, with zero line drawn). The fit contributions of each model element are listed to the side in accordance with a preferred embodiment of the present invention.

Figures 79A-79G illustrate phase contrast images (A and B) of a mildly atherosclerotic artery, with the internal elastic lamina (IEL) and collagen fibers highlighted in B. Also shown are the Raman images of collagen (C), cholesterol (D), internal elastic lamina (E), foam cells and necrotic core (F), and smooth muscle cells (G). Key morphological features, such as the fenestration of the internal elastic lamina can be observed in accordance with a preferred embodiment of the present invention.

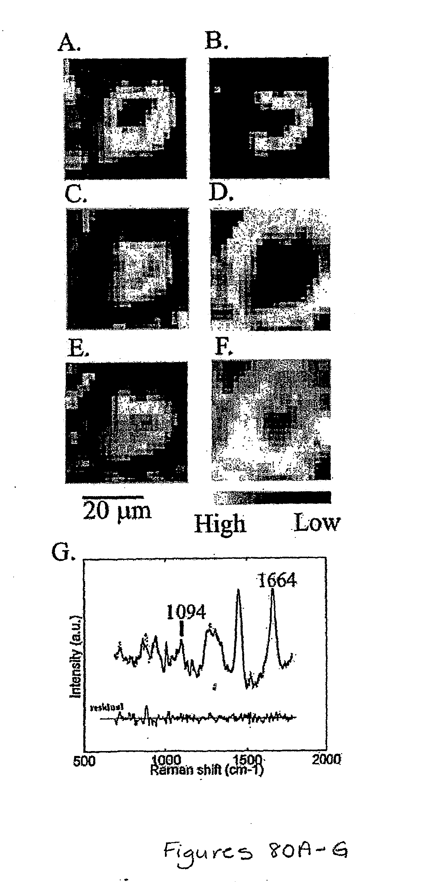

Figures 80A-80G illustrate Raman images of normal breast duct based on ordinary least-squares fitting of morphologically-derived components: cell cytoplasm (A), cell nucleus (B), fat (C), and collagen (D). Images E and F plot the intensity of single bands: the DNA phosphate (1094 cm$^{-1}$) and the protein-based amide I (1664 cm$^{-1}$) peaks respectively. Demonstration of the fitting of a morphologically-based model (·) to the spectrum of an individual pixel (located in a region with cellular content) in a Raman image (-) is shown in G. The residual of the fist is plotted below the spectrum is plotted (with the zero line drawn) in accordance with a preferred embodiment of the present invention.

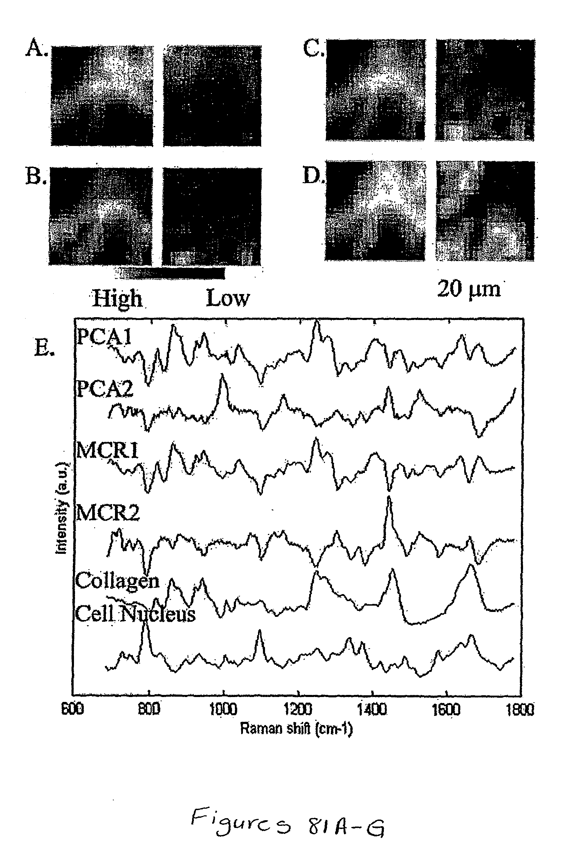

Figures 81A-81E illustrate the comparison of four different methods for analyzing Rainan images of a region with multiple ductal units, separated by collagen. The images produced by the fit coefficients of the first two principal components are shown in A. B shows the two corresponding images produced by multivariate curve resolution (MCR). C shows images based on Euclidean distance, using the collagen (left) and cell nucleus (right) spectra from the morphological model. The images in D are produced using the fit coefficients produced by ordinary least-squares fitting with the morphological model, only collagen (left) and cell nucleus (right) are shown, but the complete model was used. E shows the basis vectors used to create the images, from top to bottom: the first two principal components, the corresponding spectra produced by multivariate curve resolution, the morphologically-derived spectrum of collagen and the morphologically-derived spectrum of the cell nucleus. The last two spectra were used in both the Euclidean distance measurements and morphological modeling in accordance with a preferred embodiment of the present invention.

Figures 82A and 82B illustrate (A) Raman image (same as Figure 81D, left) with third row indicated by white line and (B) heights for corresponding fit coefficients for the indicated row obtained using the four different models: PCA (Δ), MCR (□), Euclidean distance (○), and morphological model (X) in accordance with a preferred embodiment of the present invention.

## DETAILED DESCRIPTION OF THE INVENTION

The present invention is directed to systems and methods for using Raman spectroscopy of tissue. A predicate for developing systems and methods for in-vivo applications using angiographic catheters to aid cardiologists in directing the appropriate treatment is the development of optical fiber probes for Raman spectroscopy capable of delivering low energy laser light to, and efficiently collecting the resulting Raman spectral signature from, in-vivo tissue. The probes in preferred embodiments are small, and use micro-optical design principles.

Methods for performing Raman spectroscopy for diagnosis and treatment of tissue are described in U.S. Patent No. 5,615,673 issued on April 1, 1997, in U.S. Patent No. 5,304,173 issued on April 19, 1994, in International Publication No. WO 92/15008, published on September 3, 1992 and in International Publication No. WO 96/29925 published on October 3, 1996, the entire teachings of all the references are incorporated herein by reference.

There are at least two difficulties to be overcome in producing such probes. The first is due to the spectral background signal generated in the delivery and collection fibers themselves, which may be orders of magnitude larger than the signal from the tissue site being examined. This background signal includes Raman light from the fused silica core, fluorescence from impurities and dopants used to design fibers of a particular numerical aperture (NA), as well as signal from various jacket materials. Laser light in the delivery fibers generates an intense fiber background due to the long path length traversed in the fibers, typically three to four meters. This fiber spectrum is scattered from the tissue surface and is collected, along with the tissue Raman spectrum, by the collection fibers, often masking the tissue Raman signal which is generated from only approximately 1 mm of sample due to the relatively short penetration of light into tissue.

In addition, laser light backscattered from the tissue is also collected by the collection fibers, and this scattered laser light produces an additional fiber spectrum originating in the collection fibers, which further compromises the quality of the tissue spectrum reaching the detector. In addition to obscuring and distorting the spectrum of interest, the intense fiber background adds a level of shot-noise to the signal and this noise can often be larger than the tissue Raman bands. Analyzing both delivery and collection fibers indicates that they both produce approximately equal amounts of this fiber spectral background. In a preferred embodiment, two different filters are used to suppress the undesired fiber background, one for delivery and one for collection. Further, it is desirable to terminate the delivery fibers with a short wavelength pass or band-pass filter that transmits the laser excitation light while blocking the longer wavelength spectral background generated in the delivery fibers. In a preferred embodiment, the collection fibers can be preceded by a long wavelength pass filter or notch filter which transmits the tissue Raman spectrum while blocking laser light backscattered from the tissue. Any filters used also perform the appropriate function over a range of angles corresponding to the acceptance angle (NA) of the fibers they are coupled to.

The second difficulty is related to signal collection. This has two components, the first of which pertains to the inherently weak nature of the Raman effect. Approximately only one out of every billion excitation photons are converted into a Raman photon. In a preferred embodiment, a high-throughput optical probe apparatus collects signals with sufficient signal-to-noise ratio (SNR) to be useful in a clinically realistic timeframe. To be clinically useful and commercially viable, a preferred embodiment collects high SNR spectra in approximately 1-2 seconds. The second component also addresses optimization of collection which is further compromised by absorption and scattering in the tissue which results in causing the light to be widely diffused over large areas and angles.

In a preferred embodiment the collection ability of an optical system is limited by its throughput, approximately given by the product of area of collection (A) and solid angle ($\Omega$) (A$\Omega$-product). The A$\Omega$ product is conserved throughout the system. In typical Raman spectroscopy systems, throughput is determined by the spectrograph/CCD collection detection system. In a preferred embodiment, the spectrograph is f/1.8 (NA=0.278) corresponding to a solid angle of $\Omega$=0.242sr, with a maximal slit height of 16 mm. To achieve sufficient spectral resolution for biological Raman spectroscopy a 0.2 mm slit width is used. Therefore, the maximal area of collection is A=3.2 mm$^2$, resulting in a theoretical maximal throughput of A$\Omega$=0.77 mm$^2$-sr. In a preferred embodiment a CCD detector is used to ensure that the effective Raman source generated in the tissue by the incident excitation light, no matter how bright, is optimally collected. The light is considered to be emitted over a large area and $4\pi$ solid angle but is limited by the collection angle $2\pi$. Therefore the optimal trade-off between collection solid angle and area is determined in preferred embodiments of the present invention.

In a preferred embodiment system the spectrograph/CCD is replaced by a higher throughput system. For example, one such arrangement consists of a series of dichroic beam-splitters, filters and photodiodes. The filter wavelengths are determined to optimize multivariate spectral analysis with the minimum number of wavelengths. The exact number of wavelengths and bandwidths of the detector element depend on the spectral features of the chemical/morphological structures to be sensed. Such a system results in much greater throughput than the prior art spectrograph/CCD systems and is smaller, cheaper and does not require cooling, further eliminating bulk and expense.

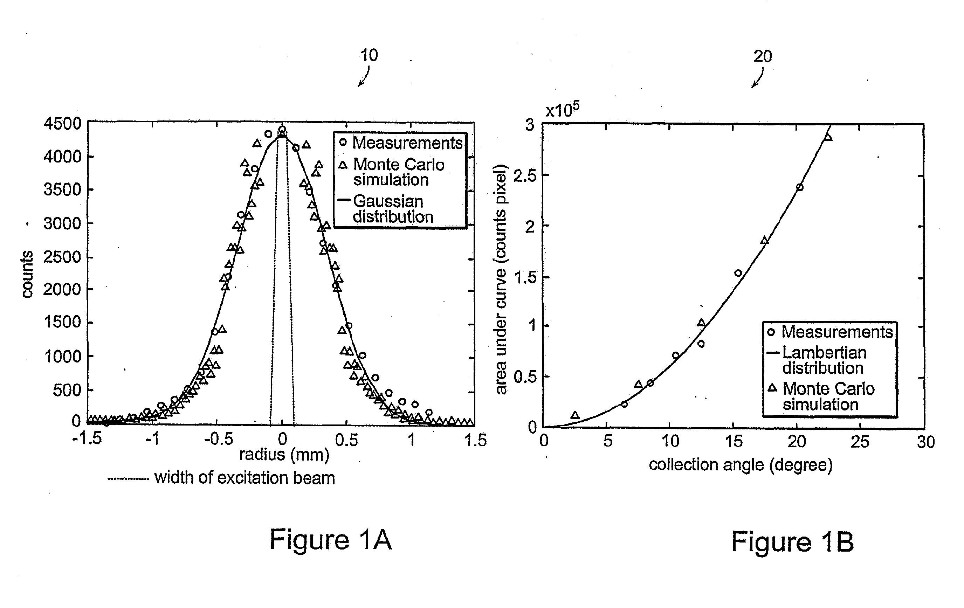

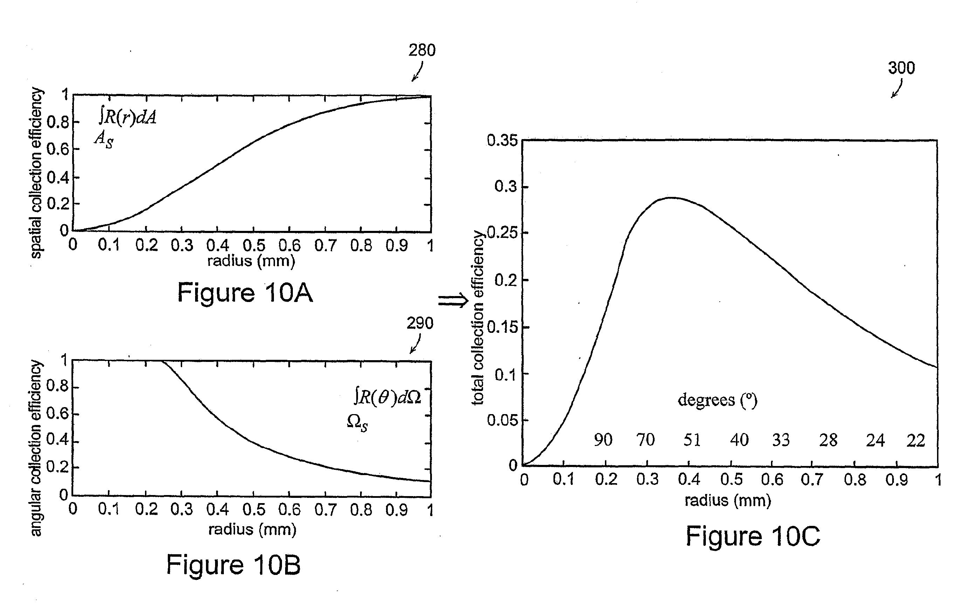

Figures 1A and 1B graphically illustrate a comparison of theory, simulations and results for Raman emission data of turbid samples of blood tissue for radial and angular distribution, respectively, of the Raman scattered light in accordance with a preferred embodiment of the present invention. Biological tissue is a collection of similar cells and the intercellular substances surrounding them. The four basic tissues in the body include epithelium tissue; connective tissues including blood, bone, and cartilage; muscle tissue; and nerve tissue. Most tissues with the exception of the cornea, are turbid, as they exhibit a high degree of elastic scattering, due to microscopic structures and refractive index variations contained therein and thus light entering such tissue is greatly diffused. Thus, the samples are characterized as turbid samples in Figures 1A, 1B, 10A-10C, 38A-38E. In a preferred embodiment, simulations such as, for example, Monte Carlo simulations are performed to predict the spatial and radial distribution of both the excitation and the Raman scattered light. In a preferred embodiment, the radial distribution is approximately Gaussian as shown in Figure 1A, while the angular distribution is Lambertian as shown in Figure 1B. Using these parameters and the optical throughput theorem which includes the conservation of the product of area and solid angle of the light being transmitted through an optical system, the optimal collection area and angles are determined. It should be recognized that the product A$\Omega$ is a constant and choosing the optimal combination of A and $\Omega$ is important as shown in Figures 10A-10C. In a preferred embodiment collection parameters of approximately 0.35 mm radius and 55˚ are optimal for blood tissue. The optimal collection parameters for artery tissue are approximately 0.4 mm radius and 20˚. The results of the analyses are then incorporated into an optical design program such as, for example, Zemax program to determine appropriate optics for maximal signal collection.

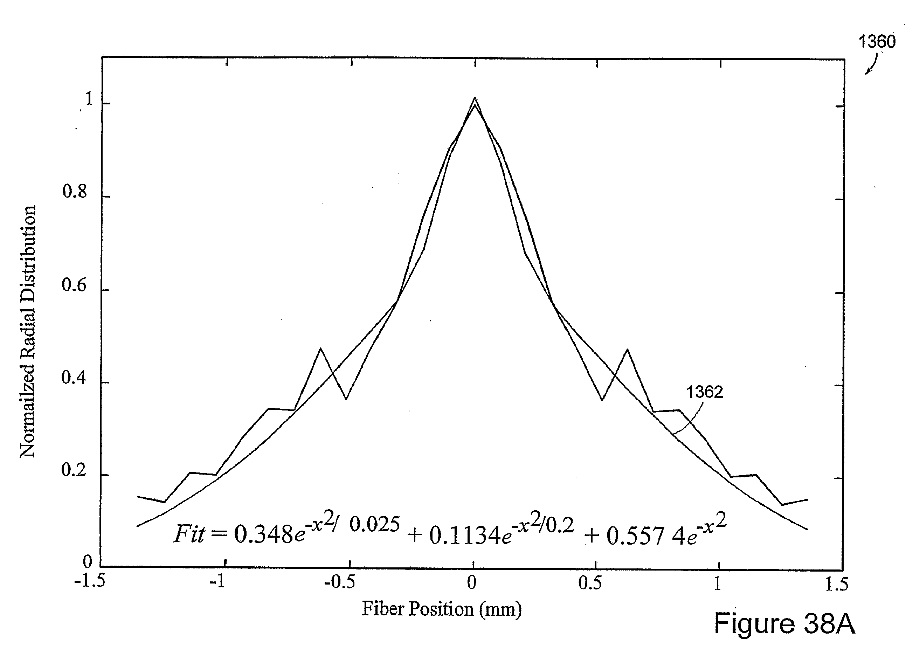

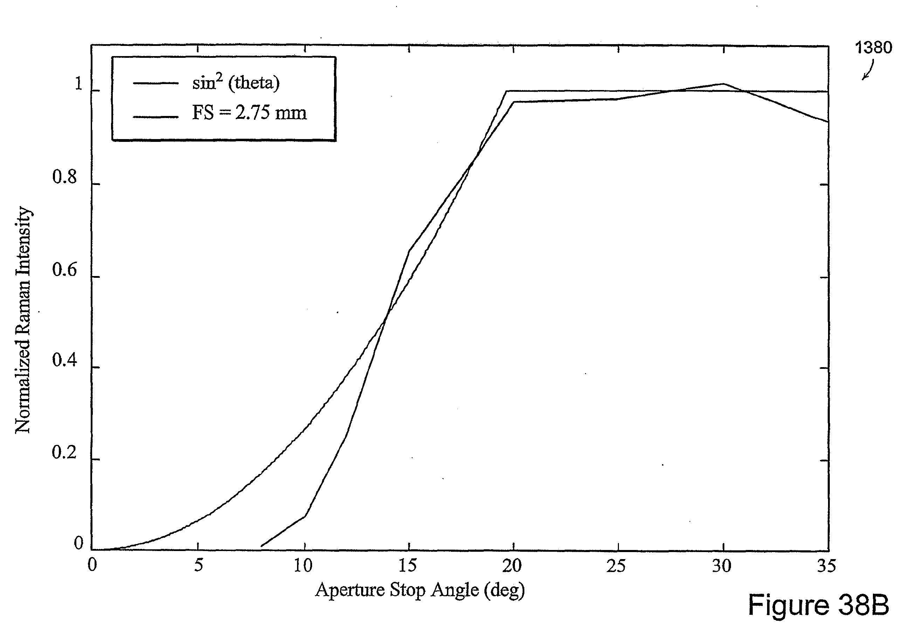

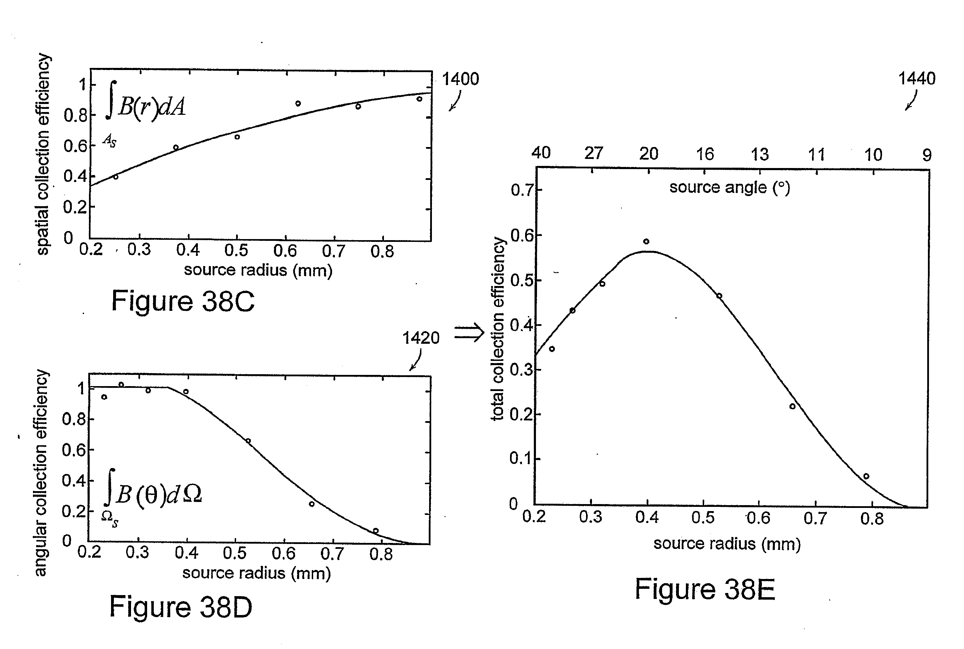

Figure 38A graphically illustrates the results for Raman emission data 1360 of turbid samples of artery tissue in contrast to blood tissue described with respect to Figure 1A for the radial distribution of the excitation and Raman scattered light in accordance with a preferred embodiment of the present invention. The curve 1362 illustrates the fit using a three Gaussian fit. Further, Figure 38B graphically illustrates the results for Raman emission data 1360 of turbid samples of artery tissue for angular distribution of the excitation and Raman scattered light in accordance with a preferred embodiment of the present invention. Similar to Figures 10A-10C which illustrated distributions and collection efficiency for blood tissue; Figures 38C-38E are graphical representations of integrated radial distributions, integrated angular distributions and optimized collection efficiency for artery tissue, respectively, in accordance with a preferred embodiment of the present invention.

Optical elements are used to transfer the light collected from the tissue to the distal end of optical fibers in the probe. The proximal end of the fiber bundle is then re-shaped to match the shape, area, and NA of the spectrograph. These procedures are followed so as not to decrease light transmission efficiency, and provide effective coupling. The choice of collection fiber NA and collection fiber diameter is determined by the spectrometer NA, the desired spectral resolution, and considerations of matching optics, as well as the limitation set by filter acceptance angle. The trade-offs for the system include the spectrometer chosen, and the desired resolution determines a slit width. At the output end the collection fibers are arranged in a straight line, which is imaged onto the entrance slit by the matching optics. Considering the throughput theorem, the requirement on the collection fibers includes that the product of fiber NA and diameter equal the product of spectrometer NA and slit width. If a fiber is chosen which satisfies the stronger condition that the fiber diameter equals the slit width and the fiber NA equals the spectrometer NA, the necessity of using matching optics is eliminated and the probe is directly coupled into the spectrometer. If only the product requirement can be satisfied then matching optics are needed. In an alternate embodiment, spectrometers use curved slits, and the output end of the collection fibers can be modified to match any slit shape. An upper limit on the number of collection fibers is defined by the height of the fiber array image that is less than the slit height or CCD chip, whichever is less. However a smaller limitation may be set by the space available in the collection tip.

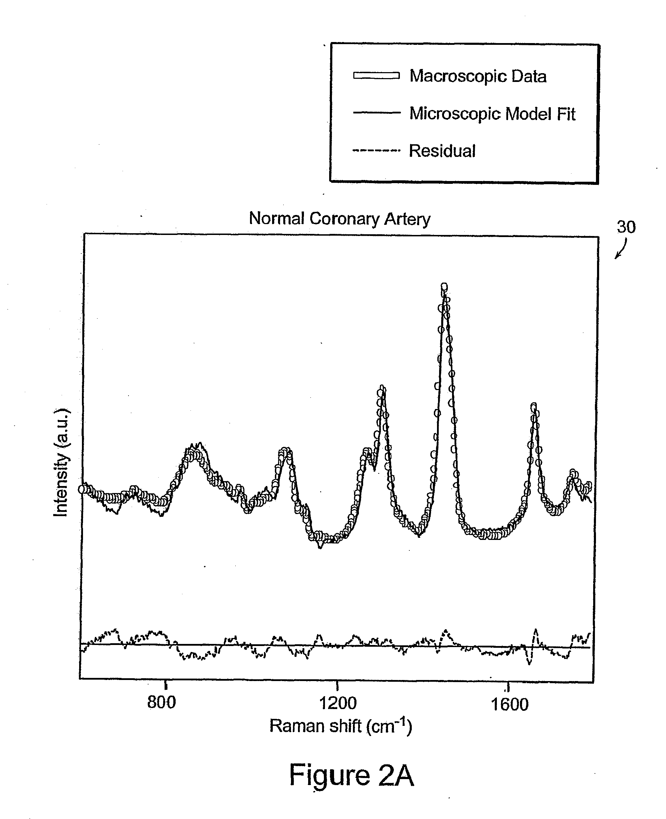

Figures 2A-2C are graphical representations 30, 40, 50 of the morphological models and references of the coronary artery in accordance with a preferred embodiment of the system. The studies use biochemical composition in determining plaque stability and plaque progression. The morphological factors are discussed in "Raman microspectroscopy of human coronary atherosclerosis: Biochemical assessment of cellular and extracellular morphologic structures in-situ" by Hendrik P. Buschman et al, as published in Cardiovascular Pathology 10 (2001) 69-82 and "Diagnosis of human coronary atherosclerosis by morphology-based Raman spectroscopy" by Hendrik P. Buschman et al, as published in Cardiovascular Pathology 10 (2001) 59-68, the entire teachings of which are incorporated herein by reference.

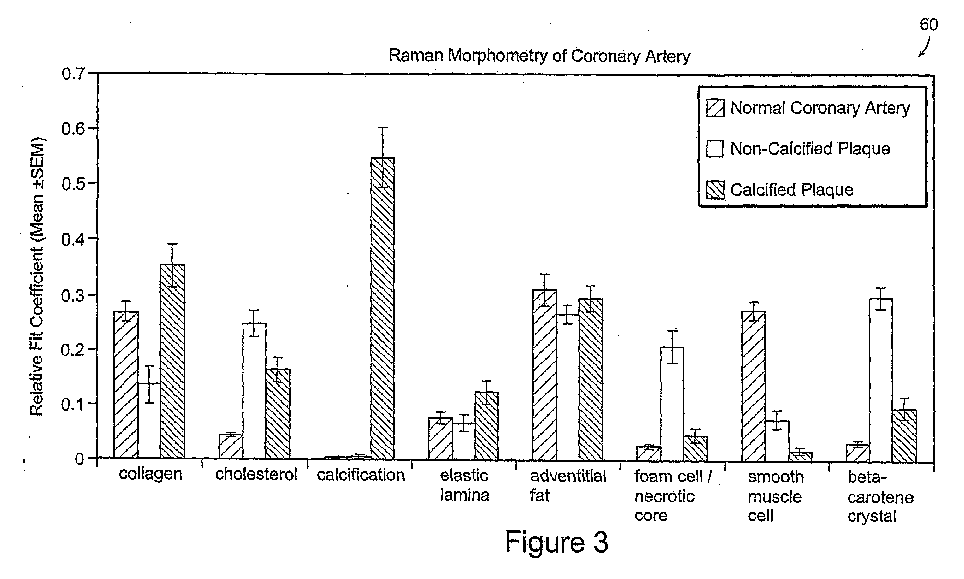

Figure 3 is a graphical illustration 60 of Raman morphometry of a coronary artery in accordance with a preferred embodiment of the present invention. The relative fit coefficients are plotted against different conditions in the normal artery, artery having non-calcified plaque and calcified plaque.

In accordance with preferred embodiments for intravascular applications all of the parameters such as, for example, but not limited to optical filtering and high-throughput optics designed to collect from diffuse sources is accomplished without increasing the diameter of the tip, or compromising its flexibility. Many prior art commercial probes are designed to be used with 785 nm excitation. The methods of the present invention include the recognition that the fluorescence background generated in tissue with 785 nm excitation is at least four times greater than that generated with 830 nm excitation. Operating at 785 nm necessitates longer data acquisition times that is prohibitive for in-vivo applications. The longer the wavelength of operation, the better in terms of fluorescence background. In a preferred embodiment, the use of 830 nm is governed by the fundamental long wavelength limit (1100 nm) of the silicon based charge coupled device (CCD) detectors which is governed by the silicon band gap. Alternate preferred embodiments, can use 785 nm or 1064 nm excitation light with appropriate detector technology.

A preferred embodiment of the present invention includes an optical fiber Raman probe which removes the optical fiber background, limits the length of the rigid distal tip to less than a few mm and the diameter to about two mm, for example, to facilitate use in coronary artery catheterization, employs 830 nm excitation and, maximizes signal collection from diffuse sources in order to allow data collection times of a few seconds or less.

A preferred embodiment includes a rod and tube configuration in which the rod and tube of optical filter modules are coated separately which is easier than coating a single disc having two separate coatings: one in the center to filter the excitation light, and one at the edges to filter the collected light. These embodiments are preferable to coating individual fibers because the filter can adhere better due to the increased surface area. In addition, a two-tone disc is preferable to coating a single disc because it is difficult to deposit concentric coatings on a small diameter with a smooth circular interface without gaps or overlapping regions. Further, it is difficult to place three meter fiber lengths in deposition coating chambers. Each filter can include a stack of dielectric thin films. Such thin film filters can be fabricated by Research Electro-Optics Inc., Boulder, Colorado.

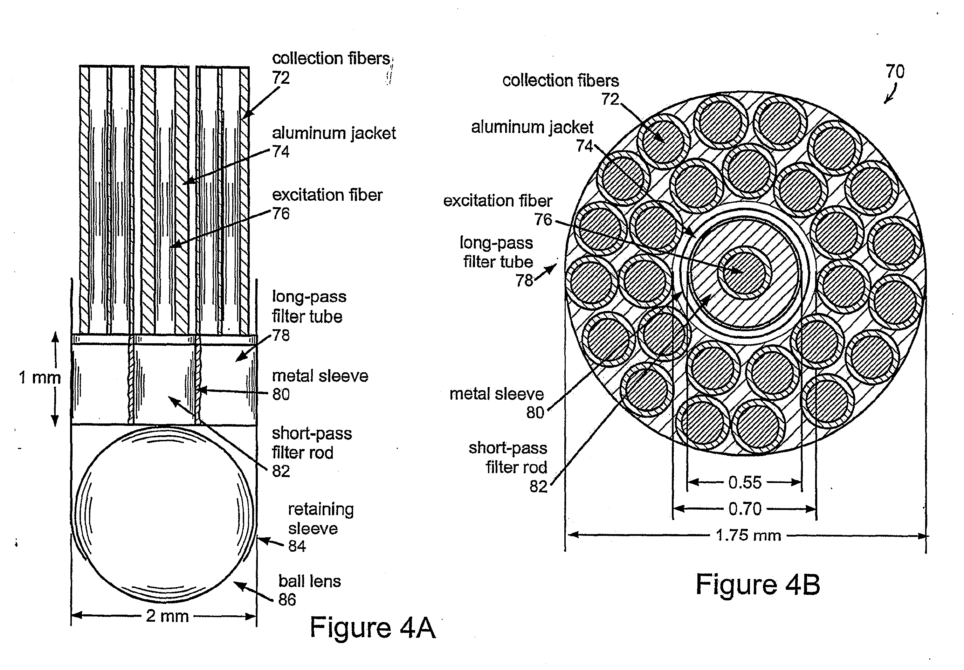

Figures 4A-4B show a longitudinal and transverse view, respectively, of a preferred embodiment apparatus including a Raman probe. The apparatus 70 includes a two piece multiple, for example, dual wavelength micro-optical dielectric filter module for minimizing and preferably eliminating fiber Raman background in the delivery and collection fibers. This module consists of a rod 82 carrying the excitation dielectric filter coating on one plane face, fitted into the tube 78 carrying the collection dielectric coating on one plane face of the tube. Rods and tubes are used in the embodiment that are made of either sapphire or fused silica which are separately coated with their respective filters prior to assembly. The rod is wrapped or coated with a thin sheet of metal 80 to provide optical isolation between the components. The module is then placed at the distal end of the probe between the fiber bundles and a lens system for collimating the light beams having a lens 86 such as, for example, a ball lens. The lens collects light from high angles and a large area effectively overlapping excitation and collection regions. The ball lens can be fabricated and supplied by Edmund Industrial Optics, New Jersey. In a preferred embodiment, sapphire lenses that are coated with anti-reflection coatings and having an appropriate index for angular acceptance, for example, 1.77 is fabricated by MK Photonics, Albuquerque, New Mexico. Although it is expensive to obtain high quality interference filters at this scale, the cost of the filters is independent on the number of pieces coated, thus it is possible to coat many filters at once, thereby reducing the construction cost of each probe. Furthermore, through additional coating runs, the filter size can be adjusted to create smaller diameter probes for various applications. In a preferred embodiment, the filters are deposited on sapphire or quartz rods and tubes for proper registration with fibers.

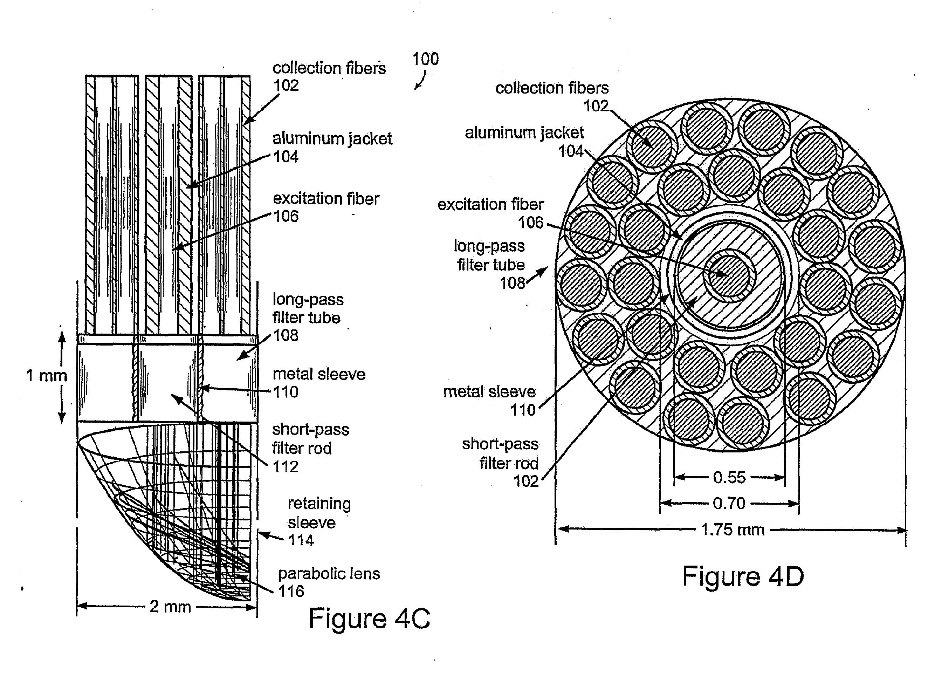

Figures 4C and 4D show a longitudinal and transverse view, respectively, of an alternate preferred embodiment having a paraboloidal mirror disposed in the lens system. The collection angle can be in the range of 0 to approximately 55° with a collection diameter of approximately 1mm. The paraboloidal mirror collects light from a wider angle and a larger area.

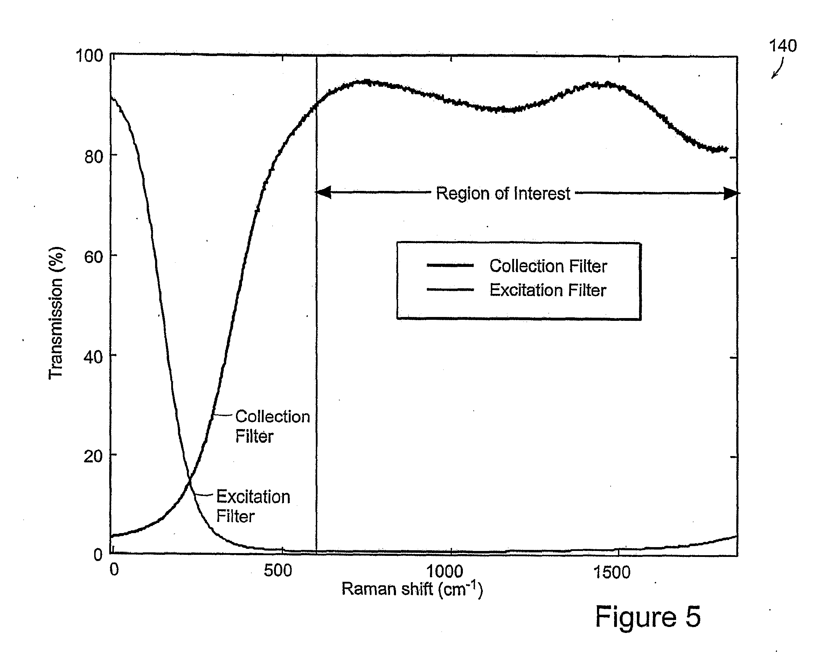

Transmission characteristics of the excitation and collection fibers incorporating these filters are shown in Figure 5 wherein 0 cm$^{-1}$ = 830 nm.

In accordance with preferred embodiments, the choice of fiber diameter and numerical aperture (NA), is dictated by the following considerations, for example, that the fiber Raman signal (produces unwanted background) is proportional to the square of the NA, and independent of the fiber diameter, that low NA is better, and that diameter has no effect.

For the excitation fiber, using a lower NA fiber is useful, however there are issues to contend with. At the input end it makes coupling the energy into the fiber more difficult. In a preferred embodiment, when exciting with a laser with a low beam divergence, reasonable care in mounting the fiber and the matching optics avoids this problem. At the output end the beam is more confined. This makes the filter construction simpler and more efficient, but illuminating a larger area in order to minimize the potential of tissue damage due to confining the power of the incident beam to a smaller area (spot) can also be important. However, even a smaller diameter spot of laser excitation light incident on the tissue spreads to cover a larger area typically ½ - 1 mm diameter because of the aforementioned elastic scattering turbidity, thus mitigating this consideration. In a preferred embodiment a larger diameter fiber, or a distributed array of smaller fibers is used. Preferred embodiments balance the fact that low NA fibers typically exhibit an increased spectral background caused by dopants used in the core and cladding of the fiber to reduce the NA, and hence, use a modest core size and NA for the excitation fiber.

For the collection fibers the situation is different. The Raman energy collected is proportional to the square of the NA. Therefore, from a signal-to-background analysis there is an advantage in using high NA collection fibers the size of which is limited by the spectrograph NA. Here, the best choice of fiber NA and fiber diameter is determined by the spectrometer NA, the desired spectral resolution, and considerations of matching optics, as well as the limitation set by filter acceptance angle. In a preferred geometry, one or a few number of delivery fibers are used as the energy of the laser source can be efficiently coupled into the delivery fiber/fibers. However, a greater number of collection fibers is important to increase the area of collection as shown in Figure 4B. The area for collection is maximized since it is important to optimize collection of Raman light. Taking all these considerations into account, it is best to use as much of the available cross-sectional area of the optical fiber probe for collection fibers, keeping the number and diameter of the delivery fiber(s) to a minimum.

Preferred embodiments include the following trade-offs. For the spectrometer chosen, the desired resolution determines a slit width. Considering the throughput theorem again, the requirement on the collection fibers is that the product of fiber NA and diameter equal the product of spectrometer NA and slit width. If it is possible to choose a fiber which satisfies the stronger condition that the fiber diameter equals the slit width and the fiber NA equals the spectrometer NA, the necessity of using matching optics is eliminated and the probe can be directly coupled into the spectrometer. If only the product requirement can be satisfied then matching optics are needed. At the output end the collection fibers are arranged in a straight line, which is imaged onto the entrance slit by the matching optics. Occasionally spectrometers use curved slits; the output end of the collection fibers can be modified to match any slit shape. An upper limit on the number of collection fibers is that the height of the fiber array image be less than the slit height or CCD chip, whichever is less. However a smaller limitation may be set by the space available in the collection tip.

In a preferred embodiment, the fiber section of the probe includes a single central excitation fiber with an NA of 0.22 and a core diameter of 200 μm. The buffer of the fiber is matched to the diameter of the excitation filter rod, to facilitate proper fiber/filter registration, and has an aluminum jacket to provide optical isolation from the collection fibers. The 200 μm core diameter collection fibers are arranged in two different geometries in two alternate embodiments. The first embodiment consists of two concentric rings of 10 and 17 fibers for the inner and outer ring, respectively. The second embodiment has a single ring of 15 collection fibers. Although the second design has a slightly reduced collection efficiency, it is more flexible and still able to collect a high SNR spectra in short exposure times. The collection fibers all have an NA of 0.26 so that they are f/#-matched to the spectrograph for optimal throughput as illustrated in Figures 4A-4D. The diameter of the probe, in a preferred embodiment is less than 2 mm for access to coronary arteries.

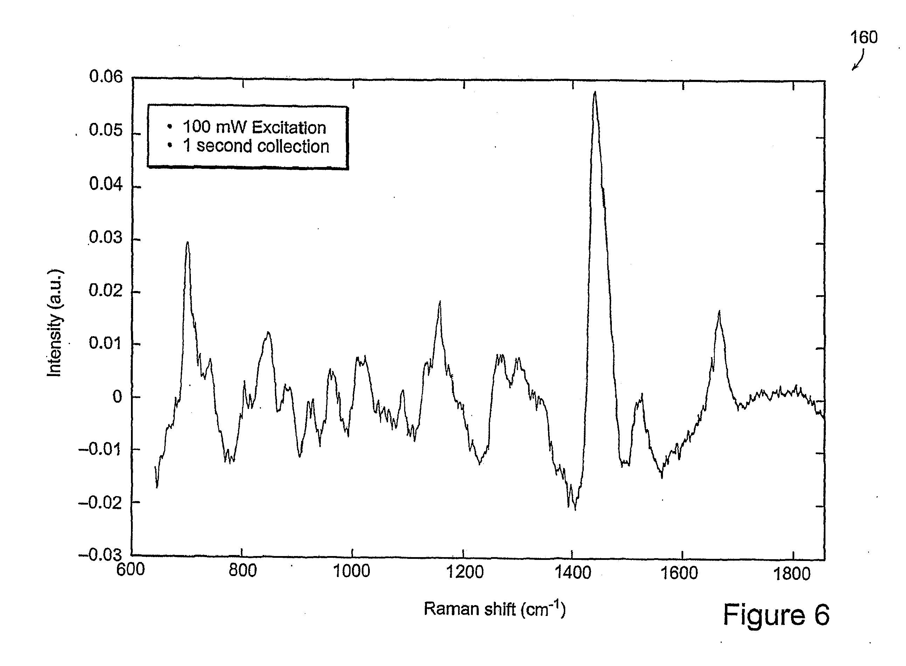

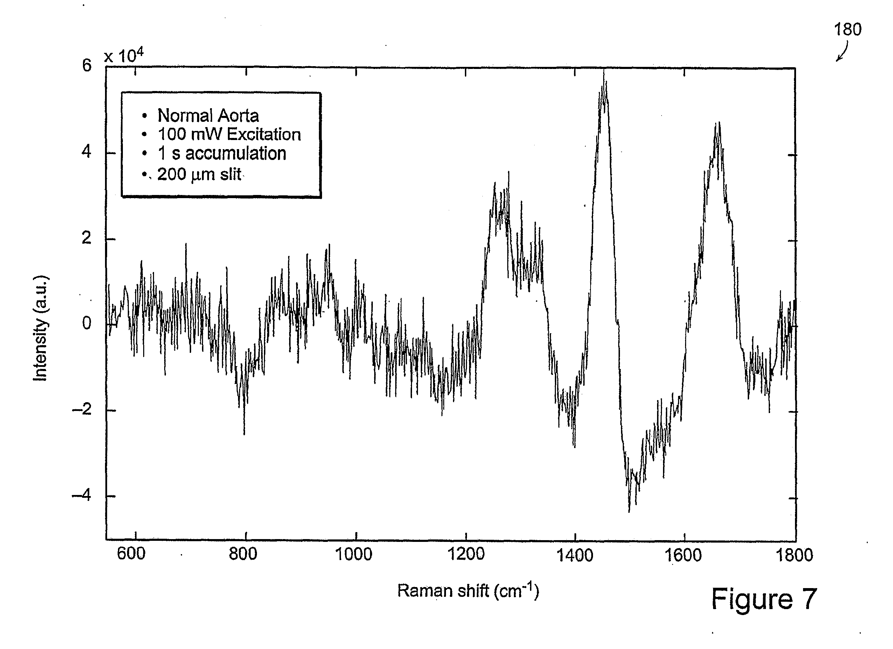

A preferred embodiment provides flexibility with respect to the particular choice of optics for high-throughput collection so that a variety of optical elements can be used to collect the desired AΩ-product. In a preferred embodiment, a ball lens provides highly efficient collection for front viewing optical fiber probes that closely match calculated collection over a radius of 0.35 mm for blood tissue (0.4 mm for artery tissue) while still collecting over large angles as shown in Figures 1A-1B and 4A-4B. Collection efficiencies greater than 30% are achieved if a small space is maintained between the sample and lens, greater than 10% when in contact with tissue, the likely and more reproducible in-vivo geometry. An example of the high quality spectra obtained with a preferred embodiment with the probe in contact with tissue is presented in Figure 6 which shows the Raman spectrum of a non-calcified atherosclerotic plaque collected in 1 second with 100 mW excitation power. In contrast, Figure 7 shows the spectrum of a normal artery taken with another preferred embodiment system in 10 seconds with the same excitation power.

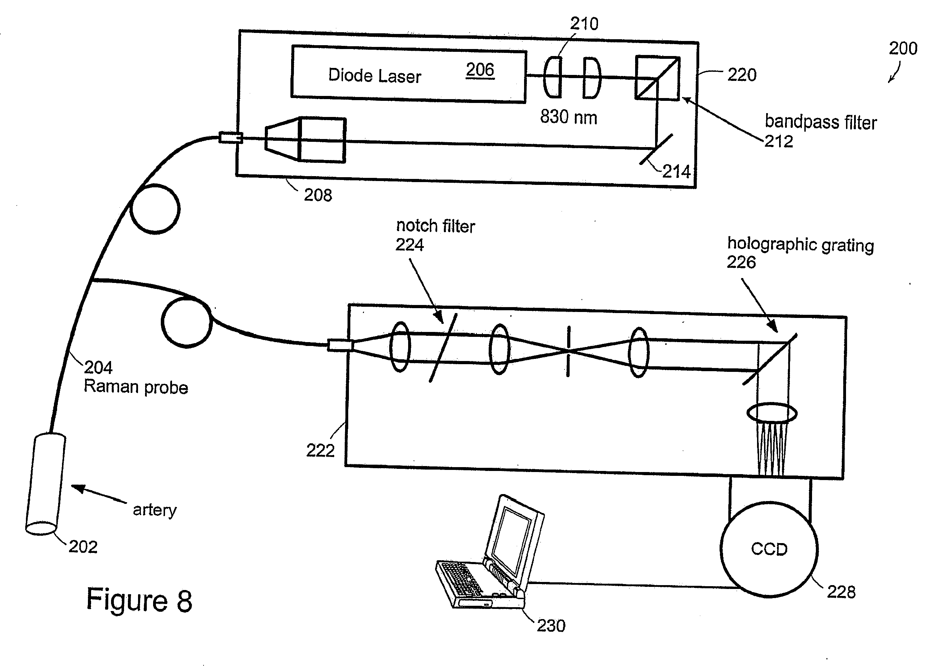

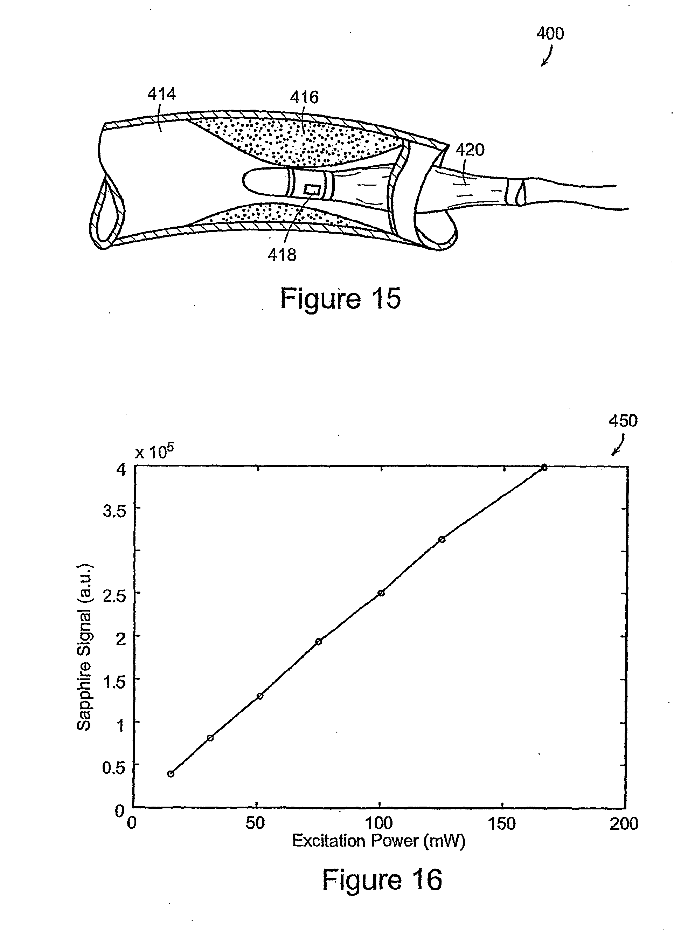

Figure 8 is a schematic diagram illustrating a system for measuring tissue in accordance with a preferred embodiment of the present invention. A light source 206 emitting at a wavelength longer than 750 nm, such as an argon pumped Ti : sapphire laser system or a diode laser is used. The diode laser may be an InGaAs laser emitting at 785 nm or 830 nm, such as, for example, fabricated by Process Instruments, Salt Lake City, Utah. The laser output is band pass-filtered and is coupled into the delivery optical fibers which are included in the probe 204. The probe 204 is inserted into an artery 202 to diagnose and possibly treat the buildup of, for example, plaque in the artery. Figure 15 is a partially sectioned view of a portion of coronary artery showing a probe in accordance with a preferred embodiment of the system of the present invention. The system may include a guidewire, and a guide catheter for threading through the large arteries. In a preferred embodiment, in an artery that is partially blocked by fatty material 416, the guide wire is first extended into the artery followed by the catheter which includes the balloon 420. A probe assembly is housed in the tip of the catheter and has a collection window 418. Once the balloon has entered the artery 414, the probe assembly provides a surgeon with a cross-sectional view of the artery. The balloon temporarily blocks blood flow providing a clear field of view, stabilizes the probe and minimizes effects of cardiac motion. Fluoroscopic markers may be used in preferred embodiments. The light is incident on the tissue and Raman-scattered light from the tissue is collected by the collection optical fibers. The collected light is notch-filtered and projected onto an entrance slot of a spectrophotometer. The notch filter removes Rayleigh - scattered laser light. Inside the spectrograph, a grating disperses light onto a CCD detector 228. The CCD interface and data storage and processing is provided in a computer such as a personal computer. A program such as Winspec Software provided by Princeton Instruments can be used to connect the CCD interface to the personal computer which performs the data processing and storage function. In alternate embodiments, the Labview program by National Instruments, Austin, Texas, is used to connect the CCD interface to the personal computer. Raman signals are read from the CCD, collected by the computer and stored on a computer readable media for later analysis or may be used for real time analysis in a clinical setting.

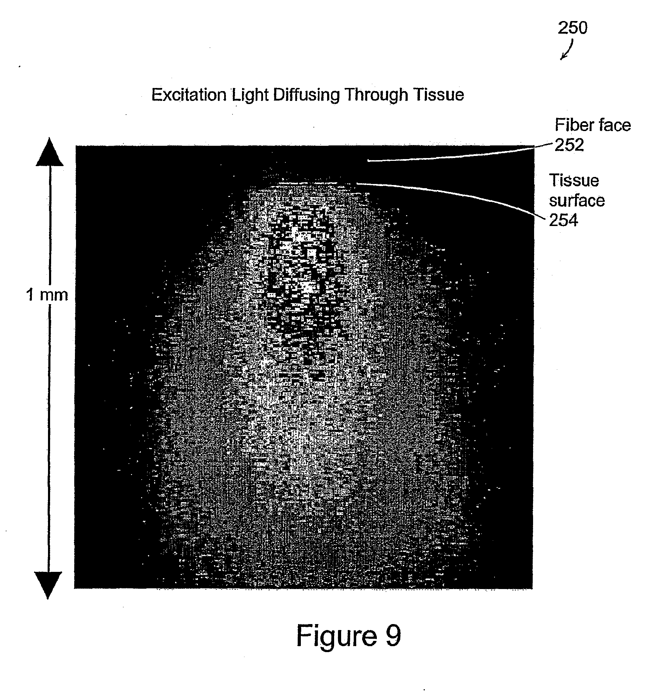

Figure 9 illustrates the excitation light diffusing through tissue in accordance with a preferred embodiment of the present invention. Figures 10A-10C are graphical representations of the integrated radial distributions, integrated angular distributions and optimized collection efficiency, respectively, for blood tissue in accordance with a preferred embodiment of the present invention. Figure 10C illustrates the collection efficiency by varying A and Ω illustrated in Figures 10A and 10B, respectively, but keeping the product AΩ constant and equal to that of the spectrograph. By the throughput theorem, the étendue is conserved where étendue is the product of area and solid angle. The radial and angular distributions are integrated and their product determines the optimization curve 300. The collection optics are designed to perform at the maximum of the efficiency curve 300. Similar graphical illustrations for artery tissue are provided in Figures 38C-38E.

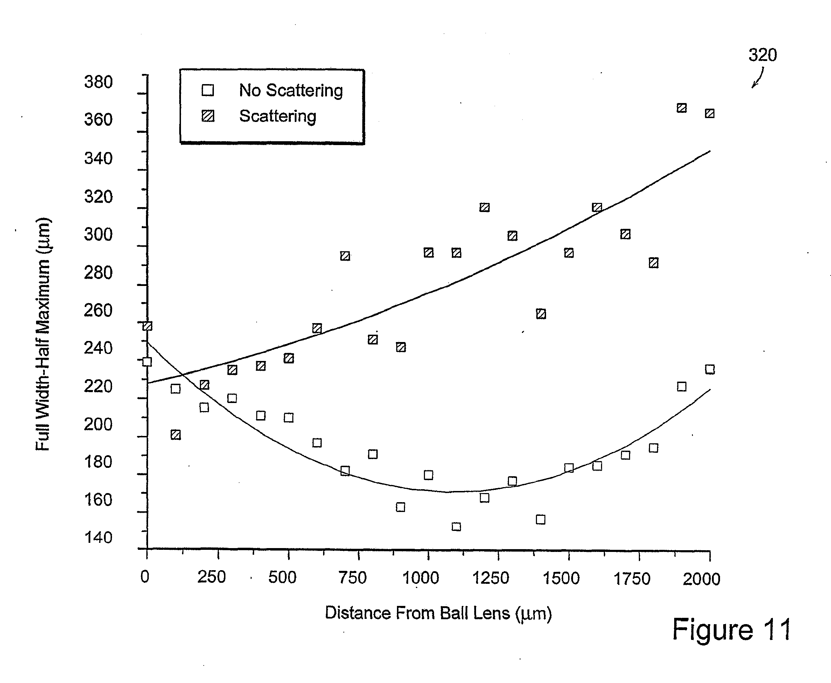

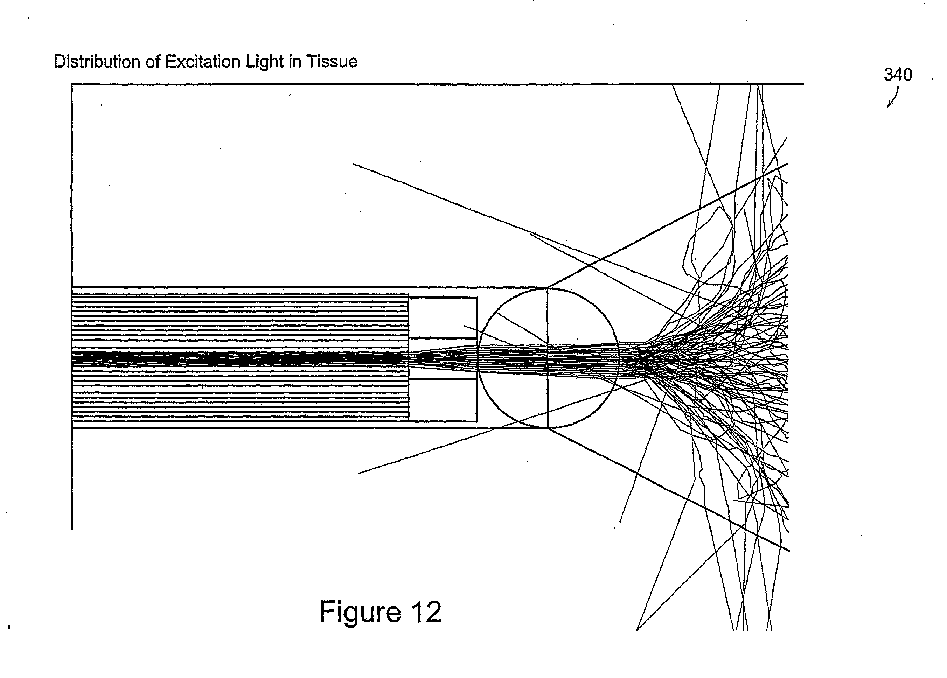

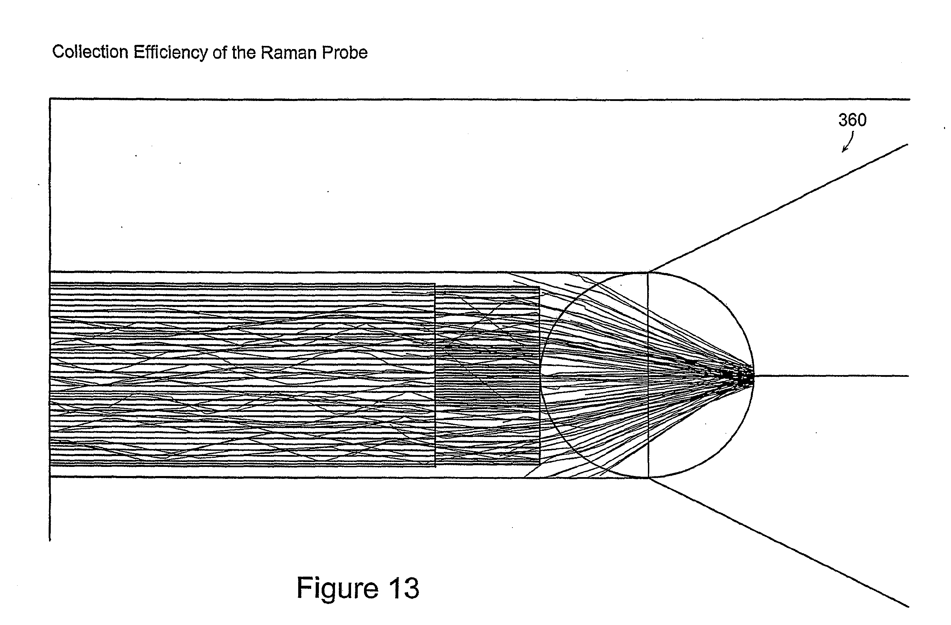

Figure 11 is a graphical representation 320 of an excitation spot diameter in accordance with a preferred embodiment of the present invention. Figure 12 is an illustration of a ray diagram 340 of the distribution of excitation light in tissue in accordance with a preferred embodiment of the present invention. Figure 13 is an illustration of a ray diagram 360 of the collection efficiency of a probe in accordance with a preferred embodiment of the present invention.

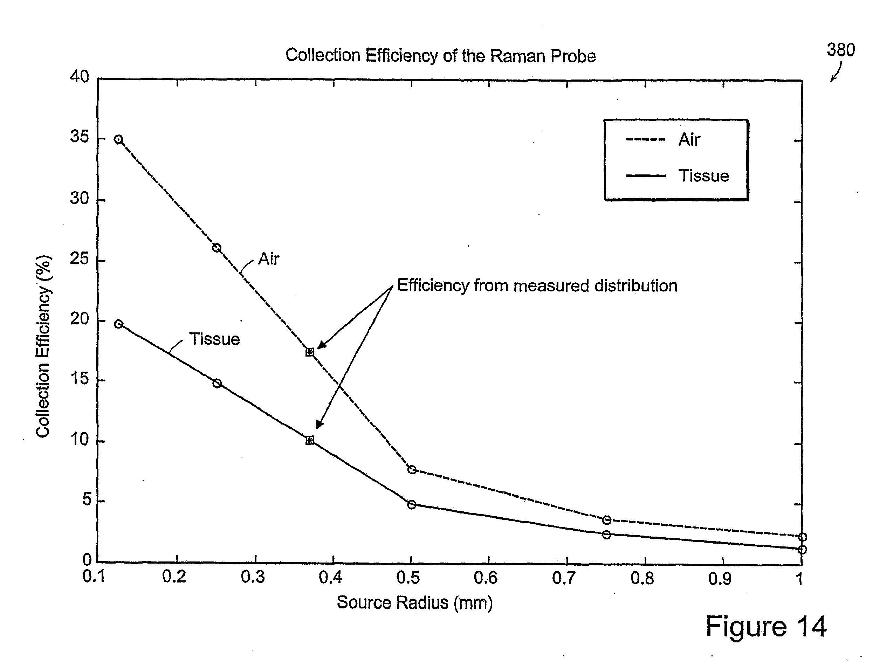

Figure 14 graphically illustrates the collection efficiency of the probe in accordance with a preferred embodiment of the present invention. The efficiency from a measured distribution for tissue and air is illustrated. All components of the probe are constructed of medical grade materials that can withstand standard cold gas, ethylene oxide sterilization. Alternate sterilization methods as provided by Steris Corporation of Ohio can be used such as, for example, a low temperature sterile processing system. The filter module in the probe tip is assembled and attached to the fiber bundle using high purity sodium silicate as an index matching cement. The advantages of sodium silicate as an index matching cement in Raman spectroscopy are unique and its utility goes beyond the present application. The advantages include producing no interfering Raman spectrum, having an index of refraction close to that of fused silica, thereby greatly reducing the reflection losses from mating surfaces, having a low optical absorption in the near IR, so it introduces no appreciable absorption losses, having cementing properties that facilitate the assembly of the small optical components involved, and it is an article accepted in commerce with uses in many industrial applications.

Sodium silicate is a ternary compound, created by mixing various combinations of water, silicon dioxide and sodium hydroxide, in the alternative sodium oxide. The optical and mechanical properties of the end product can be adjusted by varying these ratios. The other alkali silicates have similar properties, for example, lithium silicate, potassium silicate and can also be used in certain applications.

It is important not to have any adherents between the ball lens and the filters so that there is no index matching that can compromise the lensing effect provided by the curvature of the lens. The lens is secured with a crimped retaining sleeve and sealed with medical grade epoxy to prevent fluid from leaking into the probe tip in accordance with a preferred embodiment of the present invention.

The modular nature of the preferred embodiment probe is very versatile and can accommodate many optical embodiments. Additional embodiments for side-viewing probes as well as other front viewing embodiments for alternate applications are included in the systems of the present invention. For example the use of an angled and mirrored ball lens, a prism, or a micro-optical paraboloidal mirror allows efficient side-viewing probes. A tapered tip allows incorporation into needle probes for optical breast biopsies and a slightly smaller diameter in an alternate preferred embodiment allows breast analysis through ductoscopy. Other potential uses are for skin analysis, transcutaneous blood analyte monitoring, and gastrointestinal cancer evaluation.

Figure 16 illustrates a signal 450 from a ball lens and function of laser power in accordance with a preferred embodiment of the present invention. The front-viewing Raman probe uses a sapphire ball lens to focus the excitation light and to collect the Raman signal from the tissue. The Raman spectrum from the sapphire lens can be used as an internal standard to calibrate collected signals relative to the excitation laser power, thereby obtaining intensity information. This intensity information is not typically exploited in biological Raman spectroscopy, but can provide enhanced diagnostic power. The graphical plot is generated using data taken with a preferred embodiment Raman probe and a clinical Raman system and represents the magnitude of the signal from the sapphire lens as a function of excitation power while the probe is held in the air. It is indicative of a natural internal standard for measuring power delivered to tissue using a preferred embodiment of the present invention.

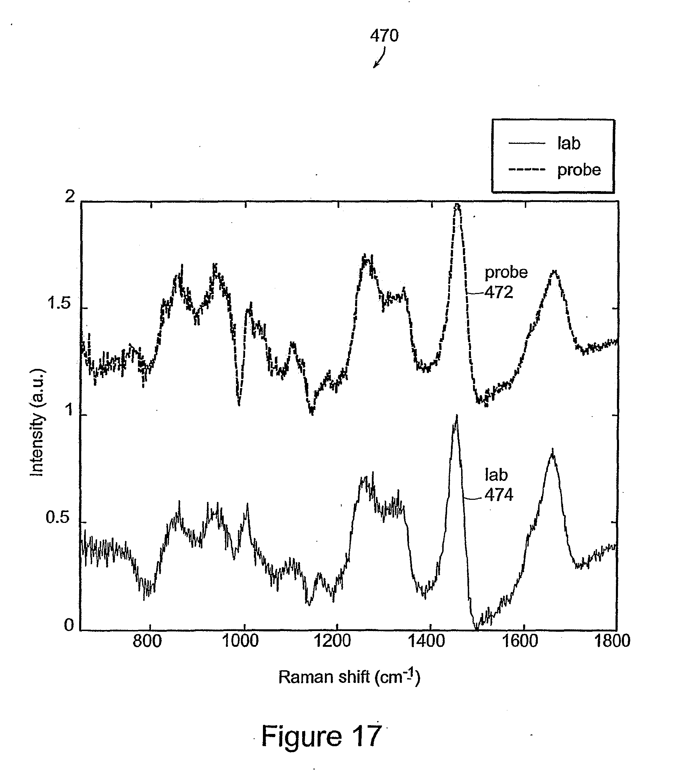

Figure 17 graphically illustrates a comparison of data from a normal artery collected using a preferred embodiment probe 472 and an experimental system 474. To demonstrate and verify the function of the Raman probe, similar types of tissue are examined. It is demonstrated that similar spectra are obtained, and the signal-to-noise ratio (SNR) of the spectra is also examined, which is an indication of system performance as greater SNR translates to a system having less noise and indicates better performance.

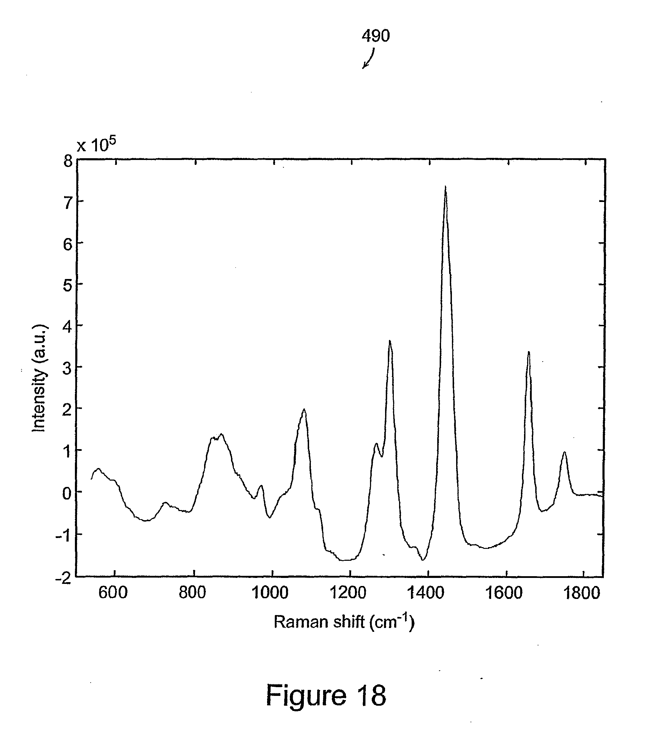

Figure 18 graphically illustrates a Raman spectrum 490 of normal breast tissue examined with a probe in accordance with the present invention. Tissues other than an artery have been examined to demonstrate that the probes have multiple uses for disease diagnosis. The probes can be used in ductoscopy procedures for early diagnosis of breast cancer.

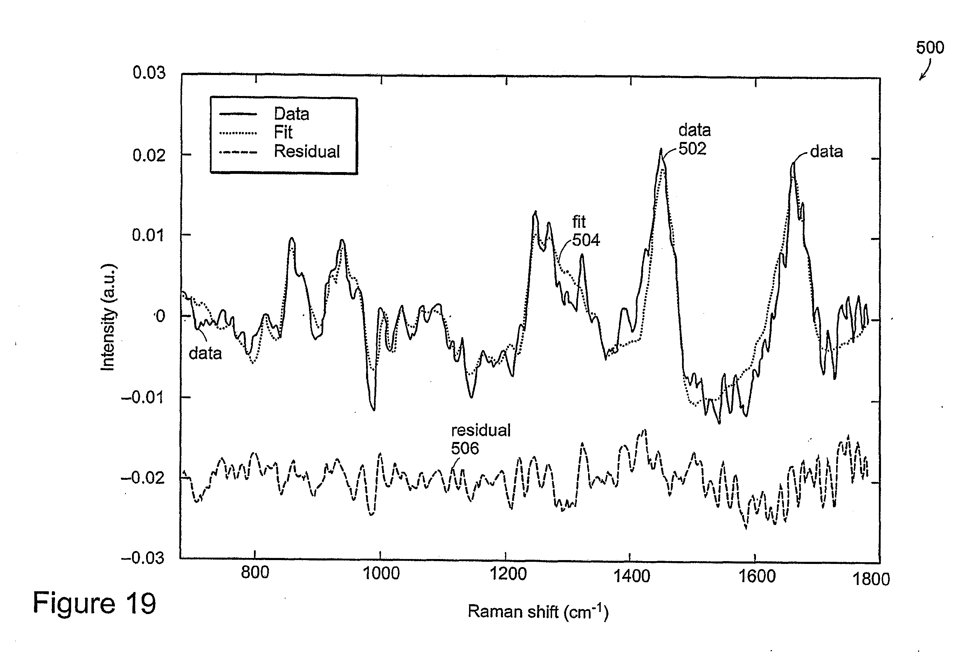

Figure 19 graphically illustrates a comparison of Raman spectra of a breast tumor which is diagnosed as being malignant in accordance with a preferred embodiment of the present invention and as predicted by reference data of the present invention. The data 502 as collected using a preferred embodiment probe is compared to data 504 generated by reference data. The reference data coefficients are tabulated in Table 1.

Table 1

| Component | Coefficient (%) |
|---|---|
| Calcium oxalate | 11 |
| Calcium hydroxyapatite | 14 |
| Cholesterol | 2 |
| Water | 0 |
| β-carotene | 0 |
| Fat | 15 |
| Collagen | 45 |
| Nucleus | 0 |
| Cytoplasm | 12 |

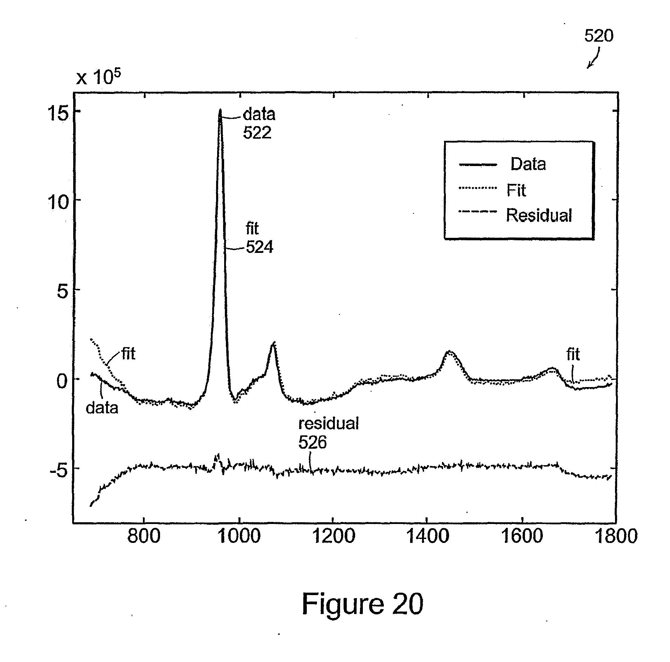

Figure 20 graphically illustrates a comparison of morphological reference data to calcified aorta data collected using a probe in accordance with a preferred embodiment of the present invention. Figure 20 illustrates the Raman spectrum 522 of a calcified aorta obtained with the Raman probe and a clinical Raman system in 1 second with 100 mW excitation power. A fit with morphological reference data is shown as spectrum 524. The residual spectrum 526 is plotted below on the same scale. The lack of features in the residual indicate a high level of agreement between the observed data and referenced data, proving that the reference data in accordance with preferred embodiments of the present invention developed with the experimental in-vitro system can be applied to data taken with the preferred embodiment Raman probes. Also of note is that there are no features corresponding to any probe background in the residual spectrum 526 indicating that any remaining, unfiltered background noise can be accurately removed. Further, the Raman spectra obtained from diseased artery does not diffuse as much in comparison to spectra obtained from normal artery, thus providing a spectra with a better level of signal-to-noise ratio (SNR). It should be noted that the SNR is affected by both Raman cross-sections of the tissue and the distribution of light.

In a preferred embodiment, the non-axial Raman probe in accordance with the present invention for use in diagnosing atherosclerosis is incorporated in a catheter of the type used for angiography, for example. It includes a balloon for displacing blood and other fluids and to position the catheter in the artery. A preferred embodiment includes a channel for balloon inflation. Further, the catheter system includes the capability for flushing away the blood temporarily with a fluid, for example, saline. One or several optical fibers can be configured so as to direct excitation light in a non-axial direction, either to the side or at an angle ranging from 45° - 90°. In such a preferred embodiment a balloon disposed on the side is used to contact the fibers adjacent the artery wall, and displace blood or other intervening fluids.

Alternately, the delivery fibers can be arranged to direct light in a circular pattern at an angle to the axis of the probe. The different collection fibers collect light simultaneously from different portions of the circumferential region illuminated. In this embodiment, the probe is enclosed in an inflatable balloon which is inflated before light delivery and/or collection to displace blood and other fluids. In preferred embodiments, the balloon is of a type used in arterial applications, such as, for example, angioplasty, and are made of thin material so as to allow excitation light to pass through to the artery wall, and return Raman light generated in the artery wall to pass through the balloon to the collection fibers.

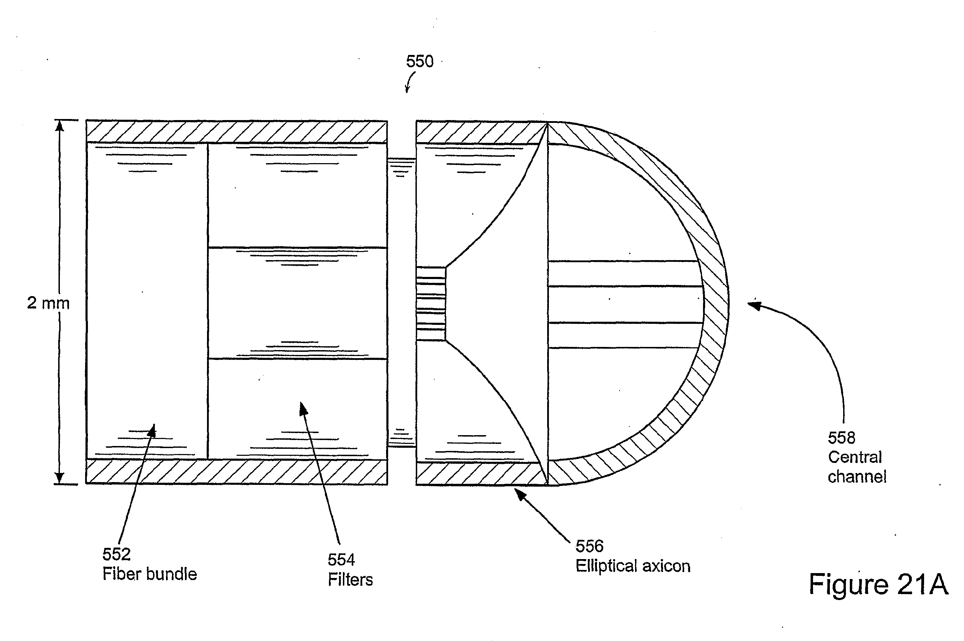

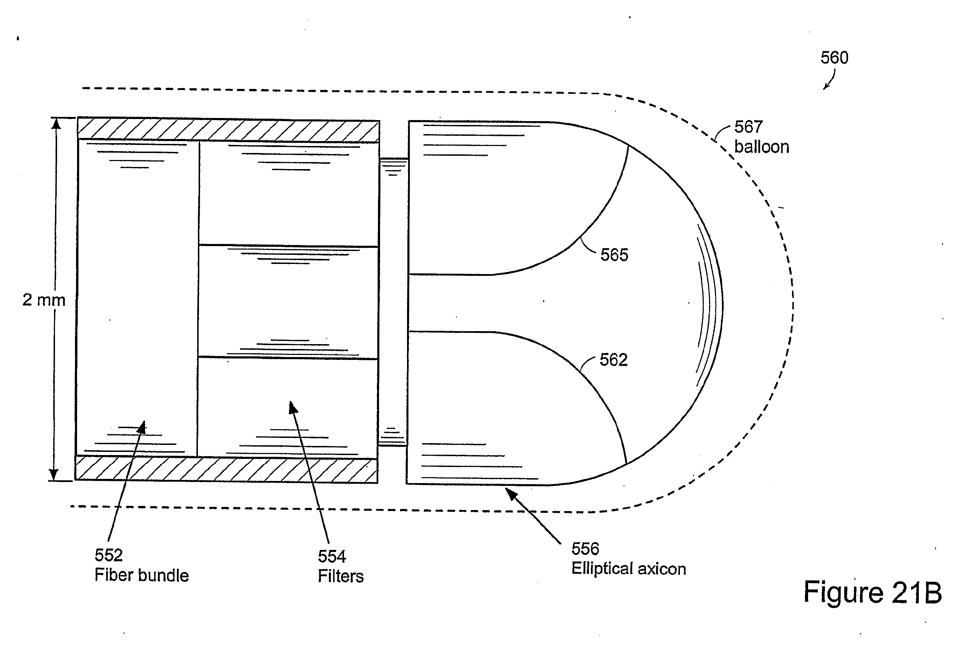

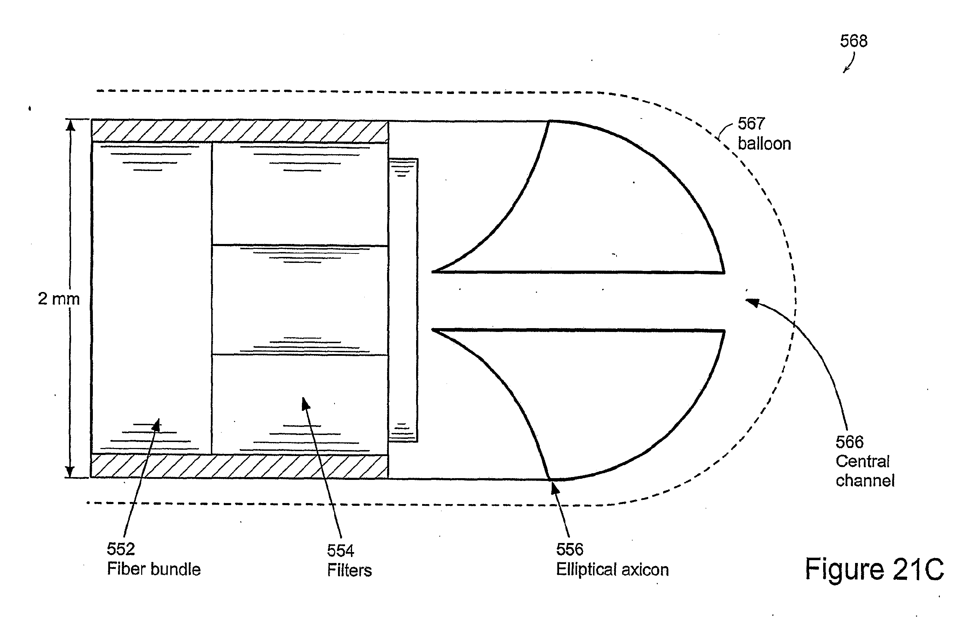

Figure 21A illustrates a longitudinal view 550 of an alternate preferred embodiment of the side-viewing probe for measuring tissue in accordance with a system of the present invention. The embodiment includes a modified axicon 556 in which the surfaces of the angled sides are made elliptical. Figures 21B and 21C are preferred alternate embodiments including at least two different radii of curvature on the angled surface to provide circumferential imaging. Circumferential imaging can be obtained in an embodiment by providing beams ranging from approximately 45° - 90° angle and rotating the probe to get a circumferential image. Alternatively, delivery fibers can provide light to the tissue and image, such as, for example, six images are collected in collection fibers to get a circumferential image. In one preferred embodiment, the volume between the filters 554 and the angled portion of the axicon 556 comprises solid glass, preferably sapphire wherein the redirection of light occurs via total internal reflection.

In the alternate preferred embodiment as illustrated in Figure 21B the angled surfaces 562 of the axicon 556 are mirrored which allow for reflections. The laser light is directed radially or non-axially onto the tissue 564. Further, the surface 565 is elliptical and fabricated using sapphire. The volume between the filters 554 and the axicon 556 may either be filled or empty. This embodiment as illustrated in Figure 21C provides an open central channel 558, 566 that can be used for flushing fluid, for example, saline into the artery or to inflate a balloon to temporarily block blood flow while data for a spectrum is being acquired. If a central channel 558, 566 is used then the probe includes placing several excitation fibers around the circumference of the central channel. The foci of the axicon can be adjusted. The rod-in-tube geometry of filters described in previous embodiments are modified to a tube-in-tube geometry, i.e., a central tube for the excitation filters with a hole in the middle for the central channel and an outer tube for the collection filters.

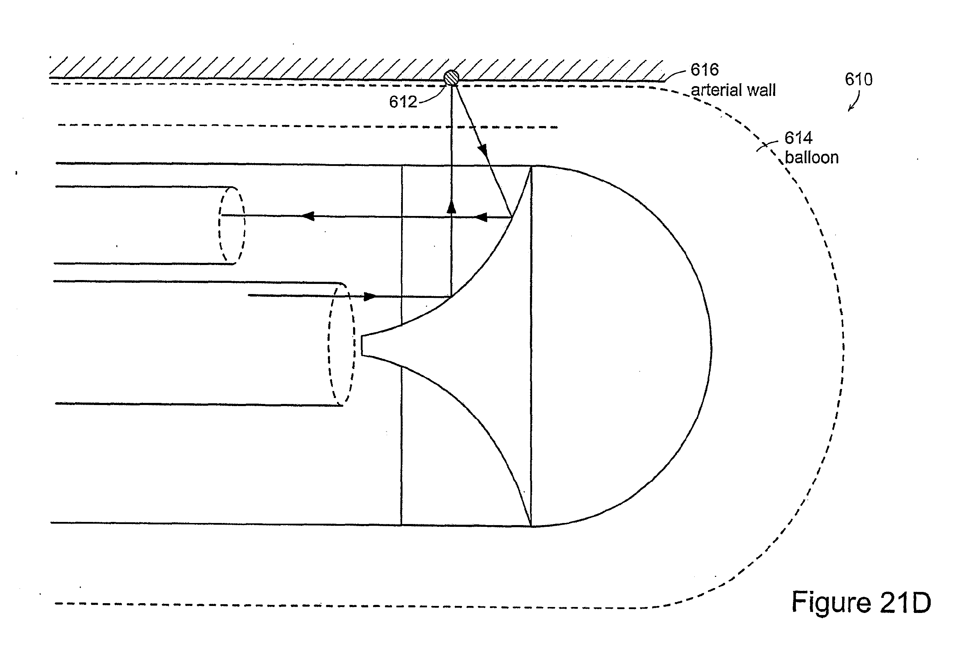

Figure 21D illustrates a view of a preferred embodiment of a side-viewing probe, or non-axial viewing probe, and catheter 610 delivering light onto a portion of tissue 612 and Raman light collected from the tissue and reimaged on a point at a second surface in accordance with a preferred embodiment of the present invention. The side-viewing probe includes an inflatable balloon 614 or flexible wire installed adjacent the side-looking element across from its viewing aperture. Upon balloon inflation or wire inflexion, the aperture is pressed into contact with the artery wall 616 displacing blood and/or establishing a well-defined collection geometry.

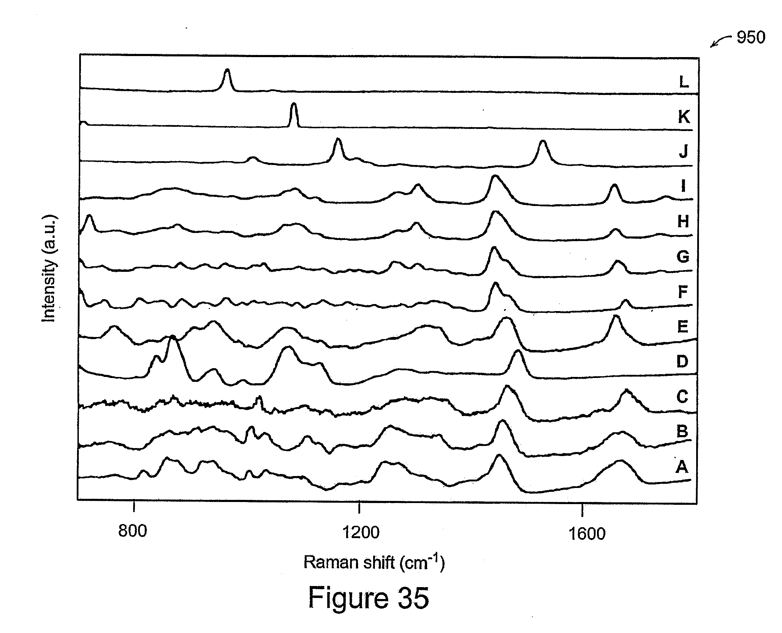

As discussed briefly hereinbefore, recent studies have shown that chemical composition and morphology, rather than anatomy (degree of stenosis), determine atherosclerotic plaque instability and predict disease progression and the risk of life-threatening complications such as thrombosis and acute plaque hemorrhage. For example, the presence of cholesterol esters may soften the plaque, whereas crystalline-free cholesterol may have the opposite effect. Raman spectroscopy can identify cholesterol esters from free cholesterol as illustrated in Figures 35F and 35G. Prior art clinical diagnostic techniques provide accurate assessment of plaque anatomy, but have limited capability to assess plaque morphology in-vivo. Further, prior art diagnostic imaging techniques such as intravascular ultrasound (IVUS), MRI, and angiography provide predominantly anatomic information about the extent of luminal stenosis, but yield only limited information about lesion composition. Coronary angiography, still the "gold standard" for diagnosing coronary artery disease, shows the degree of luminal stenosis, but provides no chemical or morphologic information about the plaque. In fact, unstable atherosclerotic plaques are often "silent" on angiography. IVUS, the most accurate and quantitative technique currently in clinical use, uses the reflection of acoustical waves delivered by an intravascular catheter to probe tissue density and provide imaging information. It has advanced the understanding of atherosclerosis significantly by demonstrating extensive atherosclerosis in coronary arteries that appear normal on angiography. However, although IVUS can identify the presence of an atheroma core, it cannot specifically identify foam cells (FC) or cholesterol crystals (CC) and does not provide any chemical information. MRI has the advantage of being a noninvasive technique, and uses radio waves generated by applying a magnetic field gradient to again probe tissue density and provide imaging information. Like IVUS, it can be used to analyze anatomy and, to a lesser extent, morphology. However, conventional proton MRI techniques used clinically largely ignore and often suppress the chemical shift information. Thus, currently plaque morphology and chemical composition can only be assessed by microscopic examination of excised tissues after endarterectomy or atherectomy.

The preferred embodiment of the present invention includes a method for a morphology-based diagnosis of atherosclerosis in the coronary arteries using Raman spectroscopy that can potentially be performed in-vivo using optical fiber technology. In a preferred embodiment, Raman tissue spectra are collected from normal and atherosclerotic coronary artery samples in different stages of disease progression ($n$ = 165) from explanted transplant recipient hearts ($n$ = 16). Raman spectra from the elastic laminae (EL), collagen fibers (CF), smooth muscle cells (SMC), adventitial adipocytes (AA) or fat cells, foam cells (FC), necrotic core (NC), cholesterol crystals (CC), β-carotene containing crystals (β-C), and calcium mineralizations (CM) are used as basis spectra in a linear least squares-minimization (LSM) model to calculate the contribution of these morphologic structures to the coronary artery tissue spectra. The preferred embodiment includes a diagnostic sequence of instructions that uses the fit-contributions of the various morphologic structures to classify 97 coronary artery samples in an initial calibration data set as either nonatherosclerotic, calcified plaque, or noncalcified atheromatous plaque. The sequence of instructions correctly classifies 64 (94%) of 68 coronary artery samples prospectively. Raman spectroscopy provides information about the morphologic composition of intact human coronary artery without the need for excision and microscopic examination. Thus, a preferred embodiment uses Raman spectroscopy to analyze the morphologic composition of atherosclerotic coronary artery lesions and assess plaque instability and disease progression in-vivo.

The present invention includes acquiring quantitative morphologic information regarding lesion composition from coronary arteries by Raman spectroscopy using a modification of mathematical reference data. This morphologic information can be used for diagnostic purposes. The chemical and morphologic information obtained by Raman spectroscopy can be the basis of a diagnostic assessment of human coronary artery disease.

In principal, both quantitative chemical and morphologic information regarding atherosclerotic lesion composition can be obtained from the same Raman spectrum. A preferred embodiment of the present invention analyzes coronary artery tissue by modeling of Raman tissue spectra using the spectra of morphologic structures rather than biochemical components as a basis set. Basis spectra for the reference data are obtained from morphologic structures commonly observed in the normal artery wall and in atherosclerotic plaque, including collagen fibers (CF), the internal and external elastic laminae (EL), smooth muscle cells (SMC), adventitial adipocytes (AA) or fat cells, foam cells (FC), necrotic core (NC), cholesterol crystals (CC), β-carotene containing crystals (β-C), and calcium mineralizations (CM). These basis spectra can then be used to linearly fit the spectra of an initial calibration set of coronary artery specimens. Using the fit-contributions of the various morphologic structures, an algorithm is included in a preferred embodiment that classifies the arteries as atherosclerotic or nonatherosclerotic as in a biochemical model. The diagnostic performance of the preferred embodiment can be tested by applying morphology-based reference data, to a second, prospective, validation set of coronary arteries.

In a preferred embodiment, tissue preparation includes obtaining from explanted recipient hearts, within 1 hour after heart transplantation, human coronary artery samples ($n$ = 200) from 16 patients, exhibiting different stages of atherosclerosis. Seven patients had heart failure due to dilated cardiomyopathy and nine due to severe ischemic heart disease. Immediately after dissection from the explanted heart, the artery segments were rinsed with neutral-buffered saline solution, snap-frozen in liquid nitrogen, and stored at - 85°C until use. The artery samples were collected in two sets, the first containing 113 (calibration set) and the second, 87 samples (prospective validation set).

These artery samples can be and were used for macroscopic and microscopic Raman spectroscopy studies. For the macroscopic study, the samples (97 and 68, from the first and second sets, respectively) were warmed passively to room temperature, cut open longitudinally, placed in an aluminum holder with the lumen side upwards, and examined under x10 magnification for selection of the region to be evaluated. After spectroscopic examination, each spot interrogated was marked with a small spot of colloidal ink, and fixed in 10% neutral-buffered formalin.

For the collection of the Raman spectra using a microspectrometer unstained, transverse tissue sections (6-8 μm) were cut from the coronary artery samples. Four sections of each sample were mounted on glass microscope slides and stained for light microscopic examination, whereas serial unstained transverse sections were mounted on $BaF_2$ or $MgF_2$ flats (International Scientific Products, Tarrytown, NY and Spectra-Tech, Stamford, CT), kept moist with phosphate buffered saline (pH 7.4), and transferred to the microscopic stage for spectroscopic experiments. No coverslip was used. Under white light illumination, the major morphologic structures were selected and recorded on videotape under x 10 and x 63 magnification.

The formalin-fixed macroscopic tissue samples were processed, paraffin-embedded, and cut through the marked locations in 5-μm thick sections, stained with hematoxylin and eosin, and examined by two experienced cardiovascular pathologists. The tissue sections were classified according to the updated Systemized Nomenclature of Human and Veterinary Medicine (SNoMed). The samples in both the calibration and validation data sets were diagnosed as either (1) normal ($n$ = 12 and 1), (2) intimal fibroplasia ($n$ = 61 and 25), (3) atherosclerotic plaque ($n$ = 3 and 0), (4) atheromatous plaque ($n$ = 6 and 16), (5) calcified atherosclerotic plaque ($n$ = 1 and 3), (6) calcified atheromatous plaque ($n$ = 7 and 13), (7) calcified fibrosclerotic plaque (n=5 and 10), or (8) calcified intimal fibroplasia ($n$ = 2 and 0, respectively). Because some of these categories had small sample numbers, the eight categories were condensed into three diagnostic classes for the development of a diagnostic algorithm: Class I, nonatherosclerotic tissue (Categories 1 and 2; $n$ = 73 and 26); Class II, noncalcified atherosclerotic plaque (Categories 3 and 4; $n$ = 9 and 16); and Class III, calcified atherosclerotic plaque (Categories 5-8; $n$ = 15 and 26).

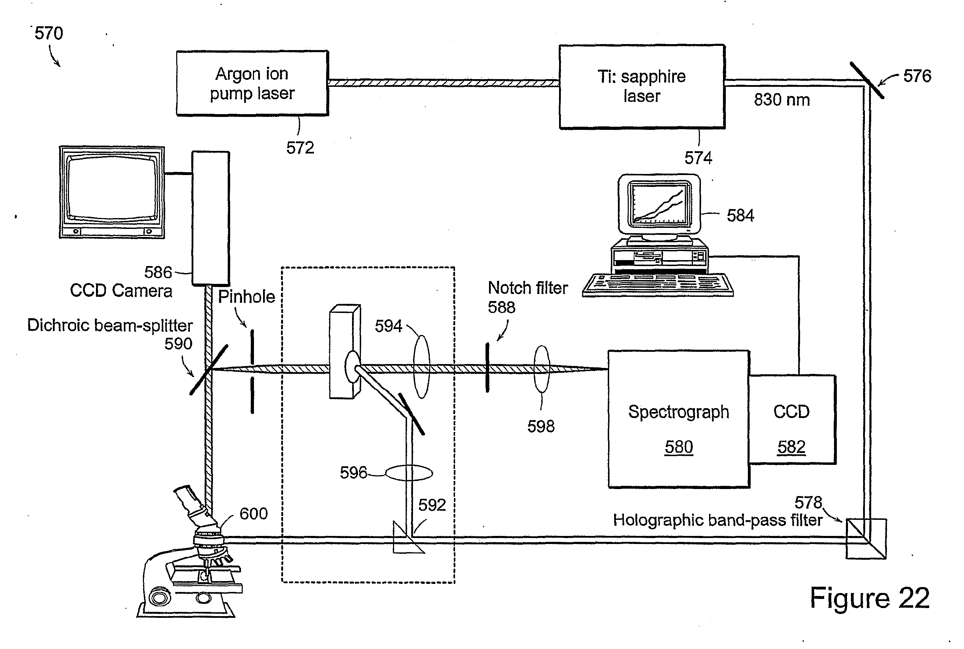

The macroscopic and microscopic Raman spectra were obtained using the Raman spectroscopy system shown in Figure 22. Near-infrared (NIR) laser light (830 nm) is generated by an $Ar^+$ -pumped Ti:sapphire laser system 572, 574 (Coherent Innova 90/Spectra Physics 3900S, Coherent, Santa Clara, CA). The laser output is band pass-filtered (Kaiser Optical Systems HLBF, Ann Arbor, MI) and, by insertion of a prism 578, either projected onto the tissue sample in the macroscopic setup, or projected into a confocal microscope 600 and focused onto the tissue section with a x 63 infinitely corrected water immersion objective (Zeiss Achroplan, NA 0.9). In the macroscopic setup, Raman-scattered light from the tissue (sampling volume 1 - 2 mm$^3$) is collected with a lens, Notch-filtered 588, and focused onto the entrance slit of a Chromex 250IS/SM spectrophotometer (Chromex, Albuquerque, NM). In the microscope setup (sampling volume approximately 2 x 2 x 2 $\mu$m$^3$), the Raman light scattered from the tissue is collected with the same objective that is used to focus light onto the sample, passed through a pinhole (giving the system its confocal characteristic), Notch-filtered, and projected onto the entrance slit of the spectrophotometer. Inside the spectograph 580, a grating disperses light onto a deep-depletion CCD detector 582 (Princeton Instruments, Princeton, NJ) cooled to -110°C. The CCD interface (ST130, Princeton Instruments), along with data storage and processing, is rendered on a personal computer 584.

For the macroscopic measurements, the laser power is 350 mW, and the signal collection time is in the range 10 - 100 s. For the microscopic measurements, the laser power is 80 - 120 mW, and the signal collection time is 60 - 360 s, and the Raman spectra is collected in the range between 400 and 2000 cm $^{-1}$ (resolution 8 cm $^{-1}$).

Each spectrum is frequency-calibrated and corrected for chromatic variations in spectrometer system detection. A fourth-order polynomial is fit to each spectrum and subtracted from the spectrum to correct for remaining tissue fluorescence. The macroscopic tissue spectra can be modeled in the 680 - 1800 cm$^{-1}$ Raman shift range as a linear combination of the morphologic structure basis spectra by LSM. This Raman shift range is chosen, because this range contains the most spectral information.

The morphologic structure Raman spectra can be normalized with respect to their maximum peak intensity. All spectra in the two data sets can be modeled accurately with the final set of eight morphologic basis spectra. The Raman spectral reference data calculated the fractional fit-contribution of seven of the morphologic structures. The eighth structure, β-carotene, is an intense Raman scatterer that often contributes to coronary artery Raman spectra, but is present only in low concentrations. For this reason, its spectrum is included in the spectral reference, but no fractional fit-contribution is calculated.

In calcified atherosclerotic plaques, CM often occupy large volumes of the tissue examined by Raman spectroscopy. To obtain information about the remaining noncalcified regions, and to compare the morphologic structure fractional fit-contributions among the different disease classes, the spectra of calcified plaques are renormalized, neglecting the contribution of calcium mineralization, and the morphologic structure fractional fit-contributions of the noncalcified regions (denoted by $X_{NCR}$) is calculated.

The relative fit-contribution of each morphologic structure to the spectra in the calibration data set is used to develop the algorithm or sequence of instructions to classify the tissue into one of the three diagnostic classes. The method of logistic regression can be used to generate a discriminant score, $R_I$, based on a linear combination of relative fit-contributions ($C_l$) of each morphologic structure $l$ as $R_i = \alpha_i + \beta_{1i}C_1 + \beta_{2i}C_2 + ...$ *with* $\alpha_i$ being a constant and $\beta_{li}$ an adjustable coefficient for each morphologic structure. This method is chosen over discriminant analysis, because logistic regression does not make any assumptions about the normalcy of the fit-coefficients.

Using maximum likelihood estimation with an analytical tool, for example, the software package STATA (Release 5.0, Stata, College Station, TX), the probability that an artery sample $j$ is nonatherosclerotic ($P_{jI}$), or contains a noncalcified atherosclerotic plaque ($P_{jII}$), or contains a calcified atherosclerotic plaque ($P_{jIII}$) is determined as

$$P_{jI} = \frac{1}{1 + e^{Rj1} + e^{Rj2}} \qquad\qquad (1)$$

$$P_{jII} = \frac{e^{Rjt}}{1 + e^{Rj1} + e^{Rj2}}, and \; P_{jIII} = 1 - P_{jII} - P_{j1} \tag{2}$$

which sum to one. Furthermore, using a likelihood-ratio test on the initial calibration data set, it can be determined which morphologic structure relative fit-contributions are significant for diagnosis, and what diagnostic thresholds for these relative fit-contributions correctly classify the most samples. The algorithm so developed can then be used to prospectively classify the artery samples in the second validation data set.

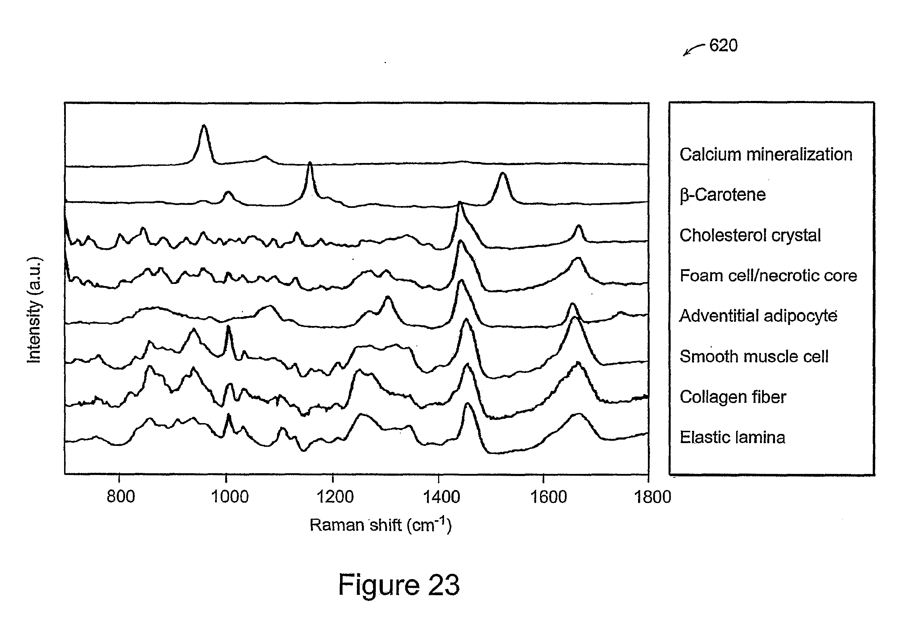

To determine the level of error in the reference data, it is necessary to analyze the signal/noise ratio (SNR) of the spectra being used. Because the microscopic Raman artery spectra of the morphological reference data can be collected for arbitrarily long times, they are virtually noise-free as illustrated in Figure 23 which graphically illustrates the Raman spectra of eight selected coronary artery morphological structures in accordance with a preferred embodiment of the present invention. Therefore, the limiting source of error in the reference is due to noise in the macroscopic spectra of the intact arteries. The in-vitro system is shot noiselimited, and therefore, the noise for any given sample is equal to the square root of the signal. Following standard multivariate analysis techniques, the concentration error is proportional to the noise in the spectrum.

$$E = N \times B \tag{3}$$

where $B = P^T(PP^T)^{-1}$, is the calibration vector for the morphologic basis spectrum of interest, and N is the noise in the sample.

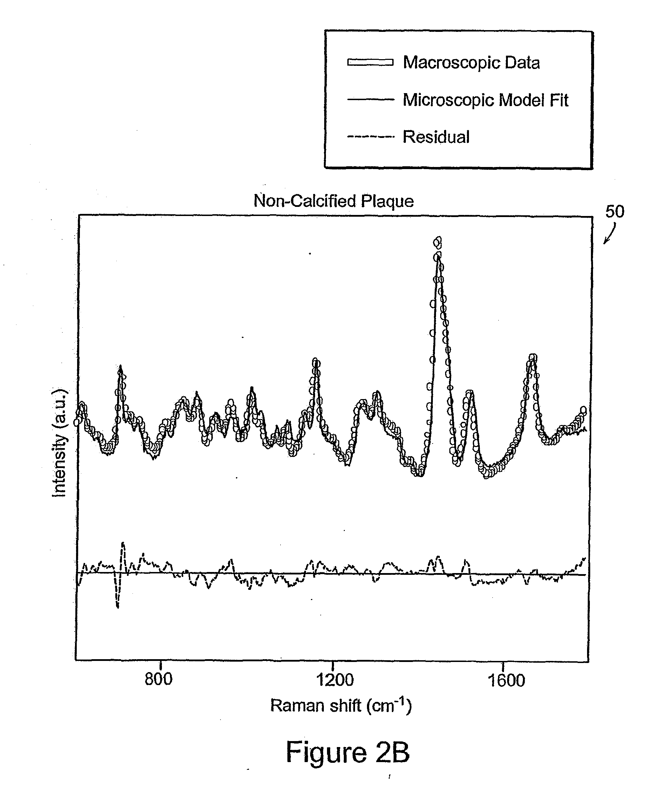

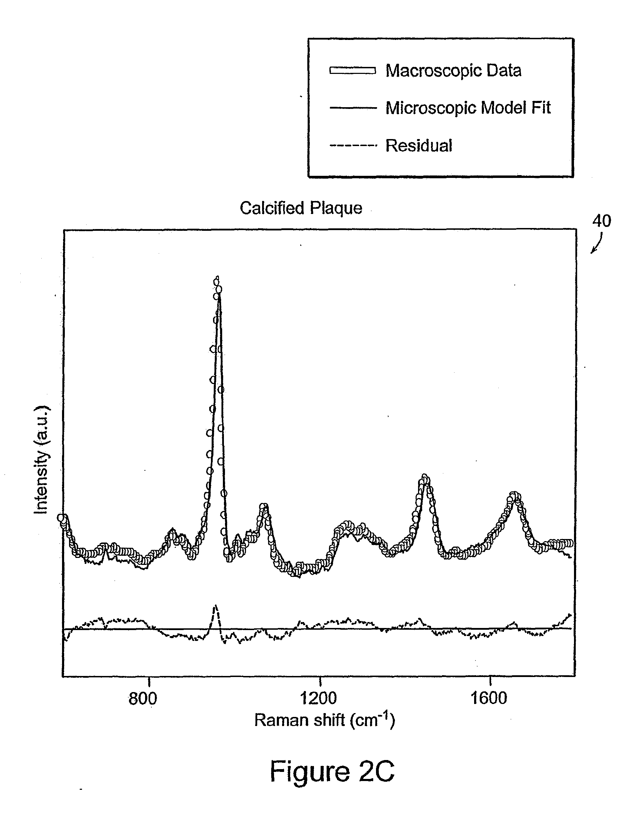

Figures 2A-2C described hereinbefore show macroscopic Raman spectra collected from coronary artery samples representing each of the three diagnostic classes (normal coronary artery (Figure 2A), noncalcified atherosclerotic plaque (Figure 2B), and calcified atherosclerotic plaque (Figure 2C)), together with LSM reference data. The solid lines are the macroscopic spectra and the dotted lines are indicative of the reference data. Residual (data minus the fit) are shown on the same scale. For all spectra, the calculated fit agrees well with the measured spectrum, which suggests that the morphologic basis spectra are a reasonably complete representation of the Raman spectra of the macroscopic tissue samples.

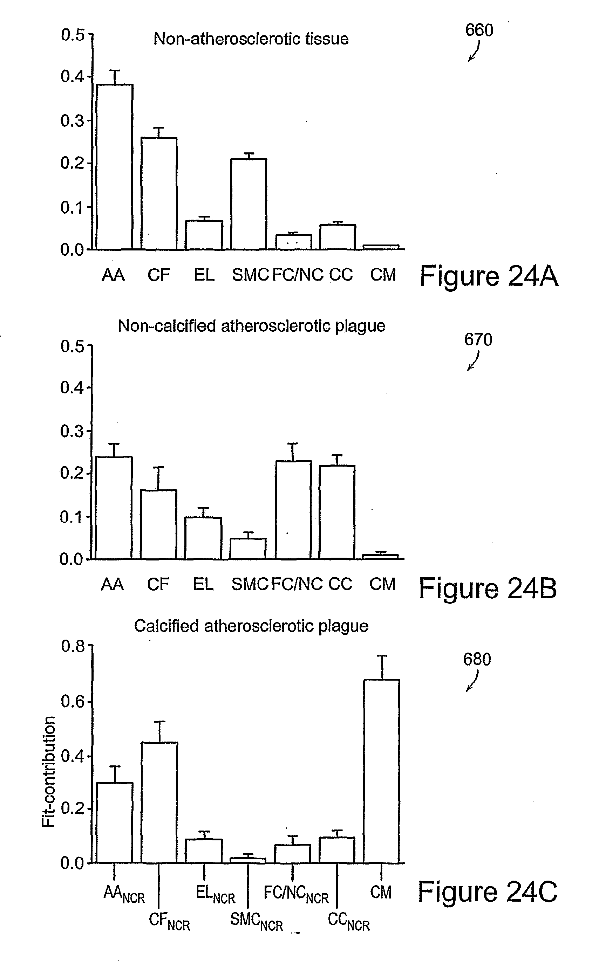

The Raman spectra of all 97 coronary artery samples in the calibration data set, which were classified by a pathologist into one of the three diagnostic classes, can be analyzed in the same way. The mean $\pm$ standard error of the mean for the relative fit-contribution of all eight selected morphologic structures in nonatherosclerotic tissue (I), noncalcified atherosclerotic plaque (II), and calcified atherosclerotic plaque (III) are shown in Figures 24A-24C, respectively. These figures clearly show that Raman spectroscopic modeling is able to detect morphologic changes in coronary artery tissue. The morphologic Raman reference data showed, as expected, that nonatherosclerotic tissue consisted mainly of AA, CF, EL, and SMC (Figure 24A). In nonatherosclerotic artery, the intima is thin and therefore, the contribution of the adventitial layer (which contains a relatively large amount of adipose tissue) to the spectroscopically examined tissue volume is large, because the NIR laser light penetrates through the entire vessel wall. In noncalcified and calcified atherosclerotic plaque, the morphologic Raman reference data revealed a dramatic change of the morphologic composition with progression of disease. In noncalcified atherosclerotic plaques, where the initima is thickened, the AA contribution decreased, whereas the contribution of FC/NC and CC increased (Figure 24B) due to accumulation of lipids in the plaque. Raman spectra of calcified atherosclerotic plaques are dominated by the CM contribution (Figure 24C). The contribution of $AA_{NCR}$ and $CF_{NCR}$ in calcified atherosclerotic plaque is larger than that of AA and CF in noncalcified atherosclerotic plaque. The reduced $CF_{NCR}$ in non-calcified plaques is an indication of decreased plaque stability.

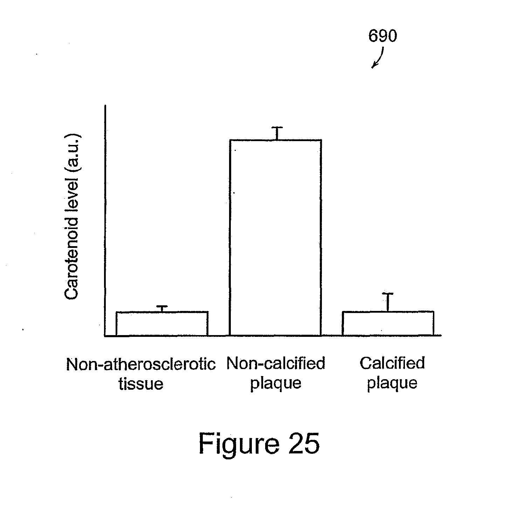

Although the concentration of β-carotene in arterial tissue is low, the modeling outcome showed large differences in the contribution of carotenoids among the disease classes as illustrated in Figure 25. The largest contribution is found in noncalcified atherosclerotic plaques, since β-carotene is a lipophilic substance that dissolves easily in the NC.

Using logistic regression, it is determined that an optimal separation of the data into three diagnostic classes can be obtained using the fit-contributions of CM and $FC/NC_{NCR} + CC_{NCR}$, with P< .0001 using a likelihood-ratio test. In addition, the likelihood ratio test determined that no improvement in classification resulted from inclusion of any of the remaining morphologic structures ($P<$ .05). The discriminant scores are determined to be $R_{j1}$ = -420.4 + 1870.0 x ($FC/NC_{NCR} + CC_{NCR}$) -6094.3 x CM, and $R_{j2}$ = -8.3 + 23.3 x ($FC/NC_{NCR} + CC_{NCR}$) + 47.6 x CM.

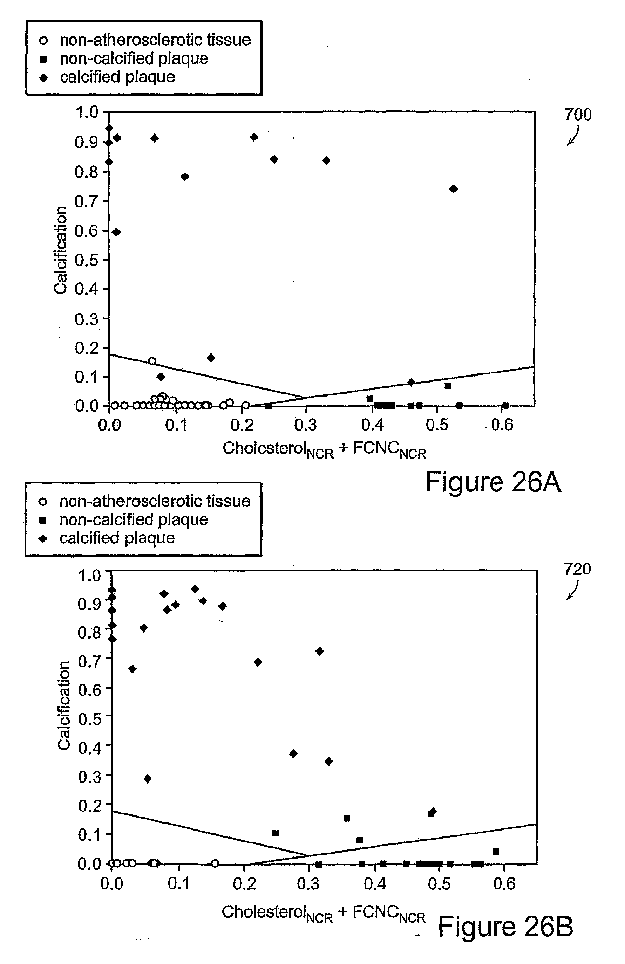

The fit-contributions of CM and $FC/NC_{NCR} + CC_{NCR}$ of each artery sample can be plotted in a decision diagram as illustrated in Figure 26A, using the corresponding $R_1$ and $R_2$ values. The border separating the regions of nonatherosclerotic tissue and noncalcified atherosclerotic plaque is given by PI = PII, which is a line described by the equation CM = -0.07 + 0.31 x ($FC/NC_{NCR} + CC_{NCR}$). The border separating the regions of nonatherosclerotic tissue and calcified atherosclerotic plaque is given by PI = PIII, and has the equation CM = 0.17 - 0.48 x (FC/NC$_{NCR}$ + CC$_{NCR}$). The line separating the regions of noncalcified atherosclerotic plaque and calcified atherosclerotic plaque is given by PII = PIII, and has the equation CM = -0.07 + 0.30 x (FC/NC$_{NCR}$ + CC$_{NCR}$). For 95 of the 97 (98%) samples in the initial calibration data set, the decision determined by the Raman-based diagnostic algorithm correlated with that of the pathologist.

This algorithm was also used prospectively in a preferred embodiment to classify the artery samples of the second validation data set into one of the three diagnostic classes as illustrated in Figure 26B. Prospectively, the algorithm result agreed with that of the pathologist for 64 of 68 (94%). Comparison of the pathologic and Raman spectroscopic diagnoses for both data sets is shown in Table 2.

Table 2

| Comparison of Pathologic Diagnosis with the of the Morphology-based Raman Diagnostic Algorithm | | | | |
|---|---|---|---|---|
| Raman diagnosis | | | | |
| Pathology diagnosis | I | II | III | Total |
| Calibration data set | | | | |
| I | 72 | 0 | 1 | 73 |
| II | 0 | 9 | 0 | 9 |
| III | 1 | 0 | 14 | 15 |
| Total | 73 | 9 | 15 | 97 |
| Prospective data set | | | | |
| I | 26 | 0 | 0 | 26 |
| II | 0 | 12 | 4 | 16 |
| III | 0 | 0 | 26 | 26 |
| Total | 26 | 12 | 30 | 68 |

The classes are (1) nonatherosclerotic tissue, (II) noncalcified plaque, and (III) calcified plaque.

Because the in-vitro Raman system used for collecting macroscopic artery spectra is shot-noise limited, the NIR techniques used in acquiring the data have resulted in extremely high SNR spectra. The average peak-to-peak noise is less than 0.04 counts on normalized spectra. Calculation of error on the fit-coefficients of diagnostic morphologic components yield a three standard deviation (SD) error of 0.041 for CM, and a three-SD error of 0.036 for FC/NC$_{NCR}$ + CC$_{NCR}$.

In another preferred embodiment, thirty-five coronary artery samples were taken from 16 explanted transplant recipient hearts, and thin sections were prepared. Using a high-resolution confocal Raman microspectrometer system with an 830-nm laser light, high signal-to-noise Raman spectra were obtained from the following morphologic structures: internal and external elastic lamina, collagen fibers, fat, foam cells, smooth muscle cells, necrotic core, β-carotene, cholesterol crystals, and calcium mineralizations. Their Raman spectra can be modeled by using a linear combination of basis Raman spectra from the major biochemicals present in arterial tissue, including collagen, elastin, actin, myosin, tropomyosin, cholesterol monohydrate, cholesterol linoleate, phosphatidyl choline, triolein, calcium hydroxyapatite, calcium carbonate, and β-carotene.

The results show that the various morphologic structures have characteristic Raman spectra, which vary little from structure to structure and from artery to artery. The biochemical model describes the spectrum of each morphologic structure well, indicating that the most essential biochemical components are included in the reference data. Furthermore, the biochemical composition of each structure, indicated by the fit contributions of the biochemical basis spectra of the morphologic structure spectrum, are very consistent. Thus, the Raman spectra of various morphologic structures in normal and atherosclerotic coronary artery may be used as basis spectra in a linear combination model to analyze the morphologic composition of atherosclerotic coronary artery lesions.

Raman spectroscopy has great potential for biochemical analysis of tissue on both the macroscopic and microscopic scale. One of the great advantages of this method is its ability to provide information about the concentration, structure, and interaction of biochemical molecules in their microenvironments within intact cells and tissues (i.e. in-situ), nondestructively, without homogenization, extraction, or the use of dyes, labels, or other contrast-enhancing agents. In addition, Raman spectroscopy can be performed in-vivo using optical fiber technology as described hereinbefore.

Using the predicate that morphologic factors may be as important as biochemical composition in determining plaque stability and progression, a preferred embodiment of the present invention includes the morphology-based diagnosis of atherosclerotic lesions in arterial tissue using Raman spectroscopy. To that end, in-situ Raman spectra of individual cellular and extracellular components of normal and atherosclerotic human coronary artery tissue were obtained in-vitro using confocal Raman microspectroscopy described hereinbefore. The biochemical composition of these microscopic morphologic structures were then determined by modeling their Raman spectra using a linear combination of basis Raman spectra of biochemicals in a similar way as used previously for intact tissue. Analogous to the macroscopic Raman spectroscopy biochemical analyses, these macroscopic Raman spectroscopy morphologic analyses can ultimately be used in a diagnostic algorithm to assess atherosclerotic plaque stability and disease progression in-vivo. Human coronary artery samples (n=35), exhibiting varying stages of atherosclerosis, were obtained from explanted recipient hearts (n=16) within 1 hour of heart transplantation. Immediately after dissection from the explanted hearts, the artery samples were rinsed with neutral buffered saline, snap frozen in liquid nitrogen, and stored at -85C.

Frozen coronary artery samples were mounted on a cryostat chuck with Histoprep (Fisher Diagnostics, Orangeburg, NY). Thin transverse tissue sections (6-8 $\mu$m) for light microscopy and Raman microspectroscopy were cut using a cryostat/microtome (International Equipment, Needham Heights, MA). Four sections of each sample were mounted on glass microscope slides and stained with hematoxylin and eosin. Serial unstained sections were then mounted on $BaF_2$ or $MgF_2$ flats (International Scientific Products, Tarrytown, NY, and Spectra-Tech., Stamford, CT), kept moist with phosphate buffered saline (pH 7.4), and transferred to the microscope stage for spectroscopic experiments performed at room temperature. No coverslip was used for spectroscopic measurements. If spectra were collected from a large number of morphologic structures, each section was replaced by a freshly cut section after approximately 2 hours to avoid biochemical changes in the tissue as a result of enzymatic degradation. No significant changes were seen in the Raman spectra within this 2 hour period of study. The morphologic structures examined were in normal arteries: collagen fibers in the various layers of the arterial wall, internal and external elastic laminae, medial smooth muscle cells, and adventitial fat cells, and in intimal atherosclerotic lesions: collagen fibers in the fibrous cap, foam cells, necrotic core, cholesterol crystals, $\beta$-carotene-containing crystals, and calcium mineralizations.

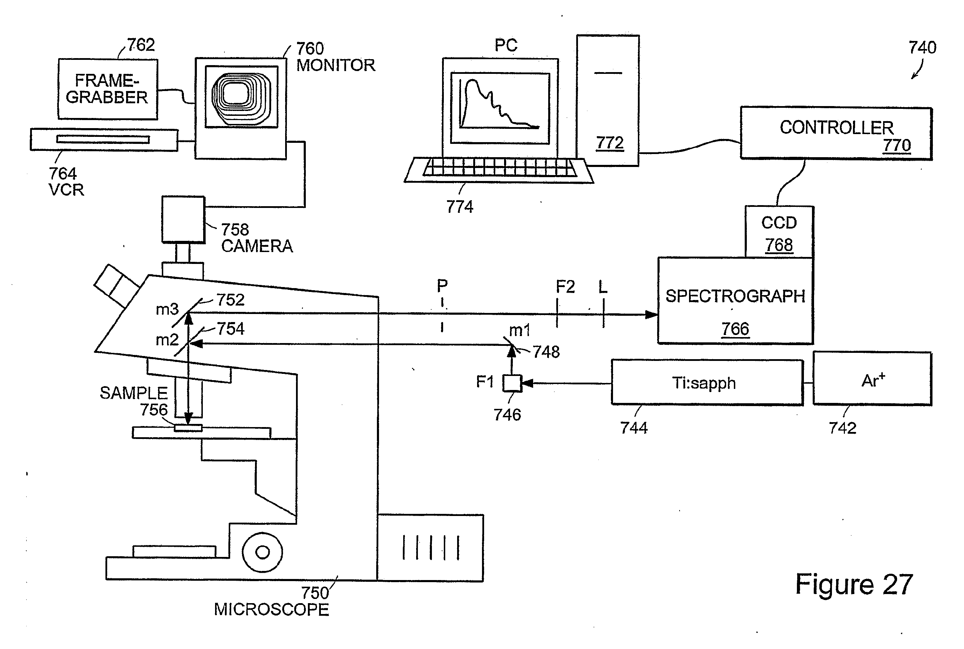

A schematic representation of the system in accordance with the preferred embodiment of the present invention is shown in Figure 27. All Raman spectroscopic measurements are carried out using a confocal configuration in order to suppress signal Raman light from features that are in peripheral surfaces other than the region of interest of the selected morphologic structure. The observation and analysis used a microscope (Zeiss Axioskop 50, Zeiss, Thornwood NY), fitted with a phase contrast system and a stage controller (Prior Scientific Instruments, Cambridge, MA). Initial examination of the sample was performed with phase contrast microscopy at 10x magnification (Zeiss Achroplan objective). Detailed examination and microspectroscopy were performed with 63x infinitely corrected water immersion objective (Zeiss Achroplan, NA 0.9). The phase contrast tissue examination and morphologic structure selection for microspectroscopy were recorded using a CCD color video camera 758 (Sony, Cambridge, MA) attached to the microscope 750 and stored on video tape (VCR) 764 from which frames were digitized (PCVision-plus, Imaging Technologies, Bedford, MA).

Near-infrared (830 nm) laser light was generated by an $Ar^+$ laser 742 -pumped Ti:sapphire laser system 744 (Coherent Innova 90/Spectra Physics 3900S, Coherent, Santa Clara, CA). The laser output was band pass filtered 746 (F1) (Kaiser Optical Systems HLBF, Ann Arbor, MI) and focused onto the sample using an adjustable mirror (m1) 748, and a dichroic beamsplitter (m2) 754, with a laser power on the sample 756 of 50-100 mW. Light emitted from the tissue sample was collected by the same objective, passed through the beamsplitter and passed through a pinhole (P: 100 $\mu$m diameter) by a removable mirror (m3) 752. This mirror was used to direct either light emitted from the sample to the spectrometer/CCD system, or white light images to the video camera system. The light directed to the CCD/spectrometer is then Notch-filtered to reject Rayleigh scattered light (F2; Kaiser Optical Systems HSNF) and focused with an achromatic lens (L) into a Chromex 250IS/SM spectrograph-monochromator (Chromex, Albuquerque, NM). The spectrograph 766 includes a grating dispersed light onto a back illuminated deep-depletion CCD detector 768 (Princeton Instruments, Princeton, NJ) cooled to -100°C. The CCD interface (ST130 Princeton Instruments) was connected to a personal computer 774 using Winspec software (Princeton Instruments, version 1.4.3), which performed data processing and storage. At least three Raman spectra (sampling time between 10 and 100 s) over a range of 100-2000 $cm^{-1}$ (8 $cm^{-1}$ resolution) were obtained from each site selected.

The method to estimate the light collection or sampling volume of the confocal Raman microspectrometer uses a small (1-2 $\mu m^3$) collection volume to insure adequate resolution to collect Raman spectra from small or thin microscopic structures, such as individual collagen fibers. In short, polystyrene beads of 1.0 $\mu$m diameter (Polysciences, Warrington, PA) were moved through the focused laser beam, and the Raman signal was collected as a function of the bead position relative to the center of the laser focus. The step resolution of the microscope stage in the horizontal plane was 1 $\mu$m. Vertical displacement proceeded in 1.1 $\mu$m steps. The position is optimized to obtain the maximal Raman signal of the bead. Lateral resolution is determined by alternately measuring the Raman signal of the central position and one of eight positions in the *X* or *Y* direction from the center of the bead using 1- or 2-$\mu$m steps. The intensity of the strong

1004 cm$^{-1}$ polystyrene Raman band is measured as a function of the distance to the laser focus in both the planar directions and the axial direction. The result for each direction is then fitted with a Gaussian function, and the diameter of the focused beam is determined from the full width at half-maximum intensity (FWHM). For both lateral directions, the diameter is about 1 $\mu$m while the axial direction is 2 $\mu$m. The sampling volume is calculated to be about 2 $\mu$m$^3$.

Data analysis of Raman spectra from morphologic structures is performed with Microcal Origin software (version 4.10, Clecom, Birmingham, UK). This analysis consists of cosmic ray removal, wavenumber shift calibration using the spectral features of toluene (Mallinckrodt Specialty Chemicals, Paris, KY) and correction for chromatic variation in the filter/spectrometer/CCD detector system with a calibrated tungsten light source. The tissue spectra is then corrected for BaF$_2$ or MgF$_2$ background contribution by subtraction of the appropriate spectrum, and corrected for tissue fluorescence by subtraction of a fourth-order polynomial that is fitted to the spectrum by least-squares minimization (LSM).

Each morphologic structure spectrum is modeled in the Raman shift range of 700-1800 cm$^{-1}$, using a simple linear combination reference to generate fractional fit contributions (C$_1$) for each of the 12 biochemical components, as

$$r_{total} = C_1 r_1 + C_2 r_2 + C_3 r_3 ... \qquad\qquad (4)$$

where r is the Raman spectrum. The 700-1800 cm$^{-1}$ Raman shift range is chosen because this range contains most spectral information.

Reagent grade commercial chemicals (Sigma, St. Louis, MO), are used to obtain the Raman spectra, for use as basis spectra, of the 12 biochemical components, including proteins (collagen type III, elastin, actin, myosin, and tropomyosin), unesterified cholesterol (cholesterol monohydrate), cholesterol esters (cholesterol linoleate), phospholipids (phosphatidyl choline), triglycerides (triolein), carotenoids ($\beta$-carotene), and calcium salts (calcium hydroxyapatite and calcium carbonate). These 12 biochemical components are selected as the most common Raman active biochemical species found in normal arterial tissue and atherosclerotic plaque. Additionally, a similar set of biochemical constituents has provided good fit of the reference data to the observed spectrum in previous macroscopic tissue studies. The Raman spectra from these chemicals is recorded in a similar way as the Raman spectra from the morphologic structures.

The reference data components cannot be scaled on chemical weight, since the actual concentration of the biochemicals in the various morphologic structures in unknown. Therefore, the intensity of the spectral feature at 1440-1455 cm$^{-1}$ (representing the bonding of CH$_2$ bonds in protein and lipid) is set to unity. The Raman spectra of $\beta$- carotene, calcium carbonate, and calcium hydroxyapatite, which lack spectral features in this region, are set to unity with respect to spectral features at 1159, 1080, and 961 cm$^{-1}$, respectively. This reference thus provides information about the relative fit contribution of these chemical components to the Raman spectra of the various morphologic structures. The fit contribution of each biochemical component is expressed as a fraction of the maximum (i.e. 1).

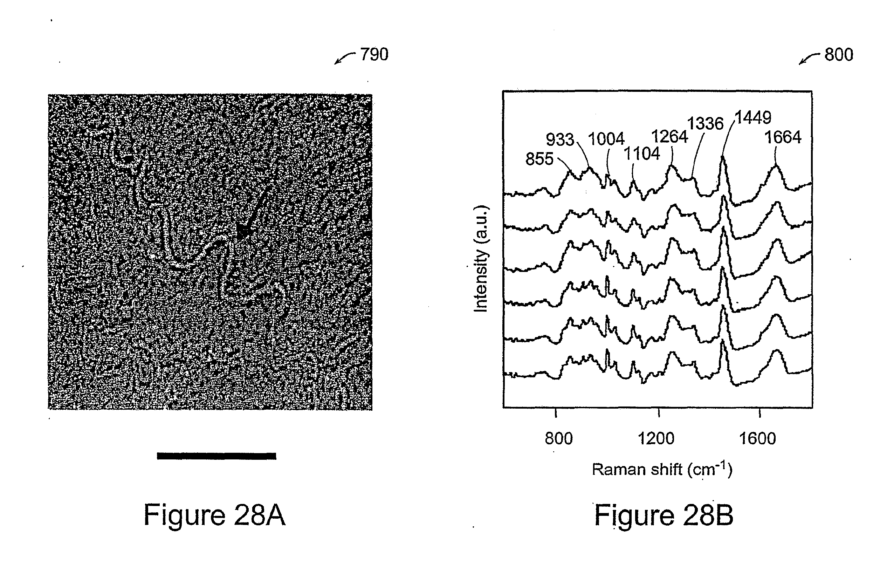

Figure 28A is a photomicrograph of an unstained coronary artery section showing the internal elastic lamina viewed under phase contrast. This structure is examined at a total of 54 sites in 21 coronary artery samples. In nine of these samples, were collected spectra from the external elastic lamina. In Figure 28B, the Raman spectra of six different internal elastic laminae are shown. The bands at 1664 and 1264 cm$^{-1}$ are attributable to the amide I and III vibrations, respectively, of structural proteins such as elastin and collagen. The intense band at 1449 cm$^{-1}$ can be assigned to the CH$_2$ and CH$_3$ bending mode of proteins, while the 1004 cm$^{-1}$ band is due to phenylalanine. The bands at 1336 and 1104 cm$^{-1}$ are attributable to desmosine/isodesmosine, and are specific for elastin. The bands at 933 and 855 cm$^{-1}$ can be assigned to the C-C stretching mode of proline and are present in collagen. These results indicate that internal elastic lamina contains both elastin and collagen. Furthermore, on visual inspection, these spectra show very little variation from structure to structure, indicating that the biochemical composition of internal elastic lamina is very consistent within and between coronary artery samples. Spectra obtained from the external elastic lamina are identical to those obtained from the internal elastic lamina.

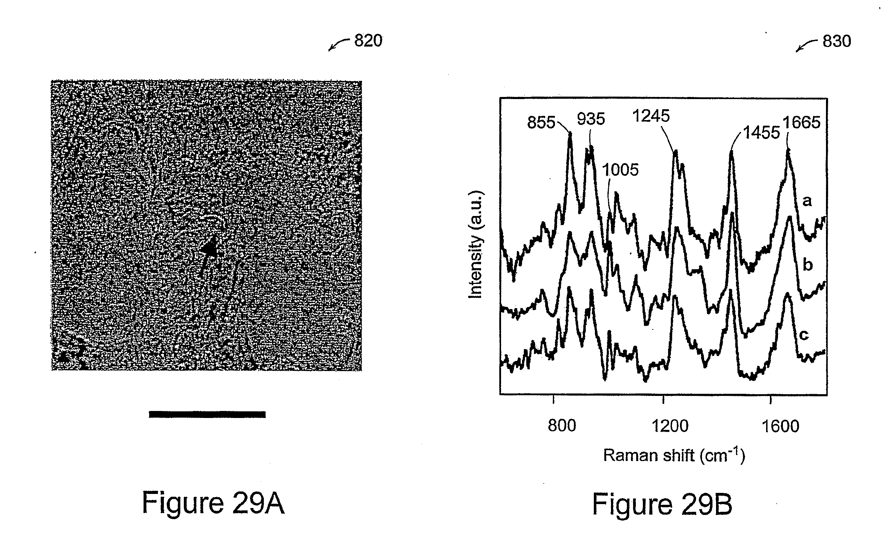

Figure 29A is a phase contrast photomicrograph showing collagen fibers (length approximately 10 $\mu$m, diameter approximately 2 $\mu$m) in the connective tissue of the tunica adventitia. In total, 17 collagen fibers in 10 samples were studied. Figure 29B shows the Raman spectra from collagen fibers from three different artery samples. Again, on visual inspection, these spectra collected from the adventitia (a, b) show little variation from fiber to fiber within or among coronary artery samples, and are identical to those taken from collagen fibers in the fibrous cap (c) of intimal atherosclerotic lesions. These spectra are also very similar to those from the elastic laminae, except for the absence of desmosine and isodesmosine bands (specific for elastin). The collagen fiber spectra also contain a pronounced hydroxyproline contri- bution (855 cm$^{-1}$) specific for collagen.

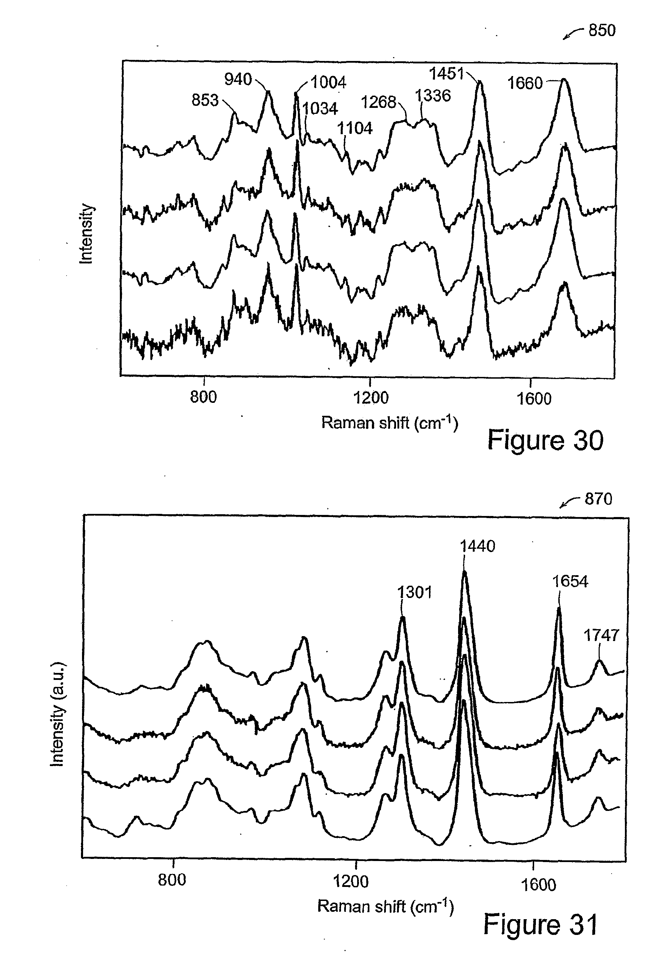

Figure 30 shows four Raman spectra recorded from smooth muscle cells in the tunica media in normal and atherosclerotic coronary artery samples in accordance with a preferred embodiment of the present invention. In total, 32 spectra were recorded from 10 coronary artery samples. On visual inspection, no significant differences were observed between spectra taken from individual smooth muscle cells. The main spectral features in the smooth muscle cell spectra are similar to - those observed in the elastic laminae and collagen fiber spectra, and are dominated by protein bands at 1660 and 1268 cm$^{-1}$ (amide I and III vibrations, respectively), 853, 940, 1034, 1336 and 1451 cm$^{-1}$(C-C or C-H bending), and 1004 cm$^{-1}$ (phenylalanine). The main differences between the protein-dominated smooth muscle cell, elastic laminae, and collagen fiber spectra are in intensity variations in the phenylalanine (1004 cm$^{-1}$), desmosine/isodesmosine (1336 and 1104 cm$^{-1}$) and amide III (1268 cm$^{-1}$)bands.

Figure 31 shows examples of Raman spectra collected from fat cells (adipocytes) in the tunica adventitia in accordance with a preferred embodiment of the present invention. In total, eight adipocytes were examined from six coronary artery samples. The spectra collected from the fat cells are very similar, and are dominated by an ester band (1747 cm$^{-1}$), an unsaturated carbon/carbon band (C = C; 1654 cm$^{-1}$), and CH$_2$/CH$_3$ bands (1440 and 1301 cm$^{-1}$), which, in combination, indicate triglycerides.

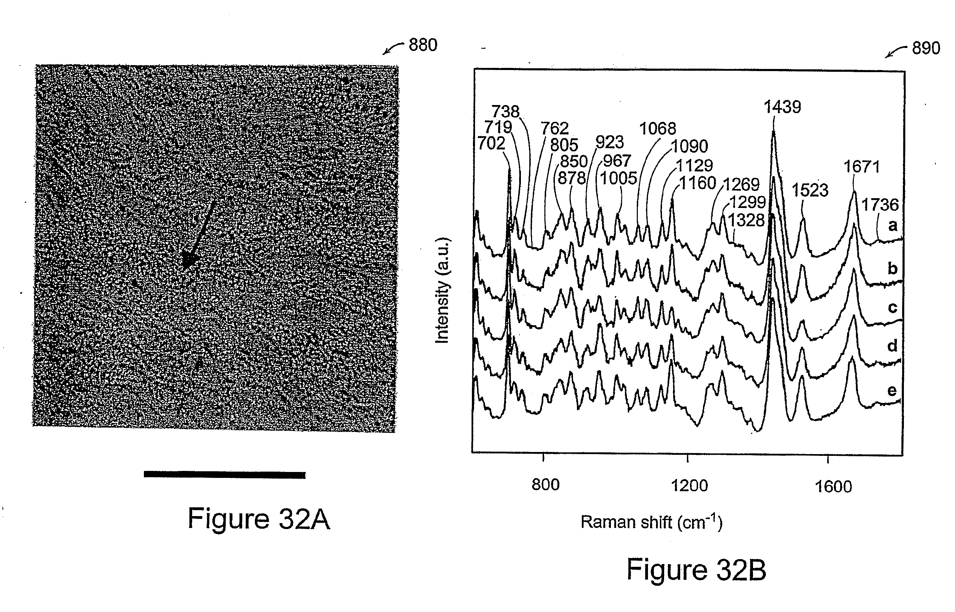

Figure 32A is a phase contrast photomicrograph of foam cells in the intima of an atheromatous plaque. The individual lipid droplets in these cells can easily be identified. In total, 30 foam cells in eight coronary artery samples were studied. In Figure 32B, the Raman spectra from three foam cells are shown (a-c). Although similar on visual inspection, these spectra show more variation among foam cells than the spectra of collagen fibers, the internal and external elastic laminae, and smooth muscle cells. More specifically, the foam cell spectra are distinctly different from the protein-dominated spectra of the elastic laminae, collagen fibers, and smooth muscle cells, particularly with regard to the numerous bands below 1100 cm$^{-1}$. The bands at 702, 878, 923, and 957 cm$^{-1}$ can be assigned to the steroid nucleus of both unesterified (free) cholesterol and cholesterol esters. The intense bands at 1671, 1439, 1299, and 1270 cm$^{-1}$ are due to C=C stretch and CH$_2$/CH$_3$ bending modes. The presence of bands at 1735 and 1026 cm$^{-1}$ (specific for cholesterol esters) and 1058 and 1328 cm$^{-1}$ (specific for free cholesterol) indicates that these foam cells contain both esterified and unesterified cholesterol. As discussed previously, the reduced CF$_{NCR}$ in non-calcified plaques is indicative of decreased plaque stability. The bands at 719 cm$^{-1}$ (symmetric choline stretch), 762 cm$^{-1}$ (symmetric O-P-O stretch), and 878 cm$^{-1}$ (asymmetric O-P-O stretch) indicate the presence of phospholipids, and those at 1523 and 1160 cm$^{-1}$ the presence of β-carotenoids. However, the foam cell spectra lack the triglyceride bands at 1747, 1654, 1440, and 1301 cm$^{-1}$ seen in adventitial fat cells.

In total, 31 necrotic core regions in 16 coronary artery samples were studied. Figure 32B also shows two examples of Raman spectra collected from necrotic core (d and e). Similar to the foam cell spectra, there is some variation from structure to structure within the necrotic core. However, the average spectra from foam cells and necrotic core are quite similar, indicating that the chemical contents of both morphologic structures are quite similar.

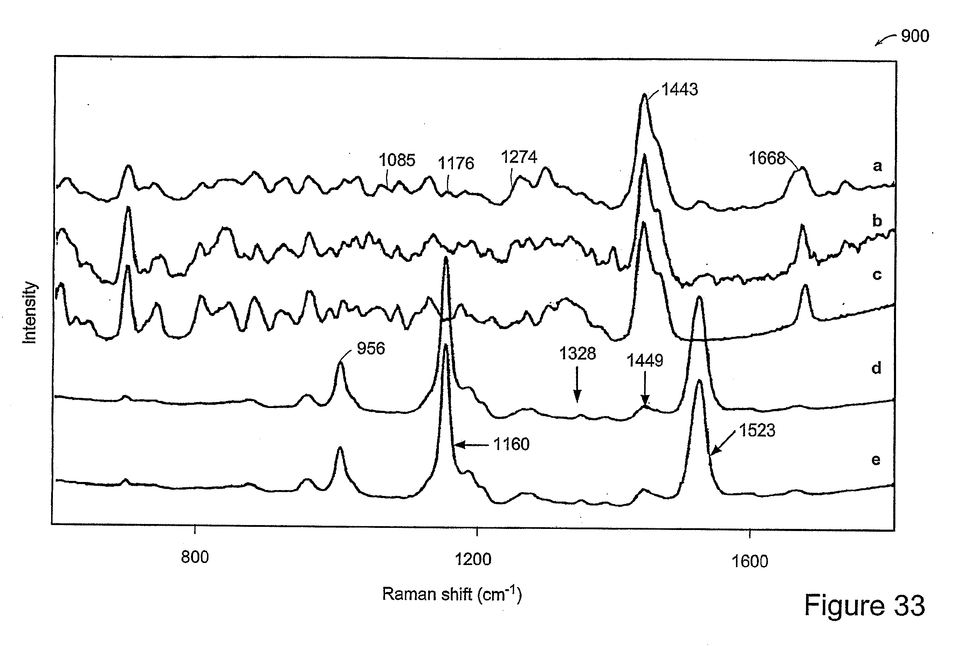

Figure 33 shows examples of Raman spectra taken from cholesterol crystals of different size in the necrotic core of atheromatous plaques. In total, cholesterol crystals in seven coronary artery samples were studied. The main spectral features of the cholesterol crystal spectra are at 1668 cm$^{-1}$ (C = C stretch), 1443, 1328, and 1274 cm$^{-1}$ (CH$_2$, CH$_3$ bending), and 1176 and 1085 cm$^{-1}$ (C - C stretch). The spectral features below 1000 cm$^{-1}$ are attributed to steroids, indicating the presence of unesterified cholesterol. Slightly more variation was seen between the spectra from individual crystals, mainly due to band intensity variations, indicative of differences in the ratio of free to esterified cholesterol in the crystals themselves or in the tissue components surrounding the crystals.

In necrotic core regions, yellow crystals could be identified under phase contrast occasionally. Figure 33 (d and e) shows the Raman spectra of two such crystals from two different coronary artery samples. In total, seven of these crystals from three samples were studied. The main spectral features are bands at 1523 and 1160cm$^{-1}$ , which are due to C-C and C=C stretches and indicative of β-carotene. Given the presence of bands at 1449 and 956 cm$^{-1}$, these yellow crystals also appear to contain some structural proteins and cholesterol esters.

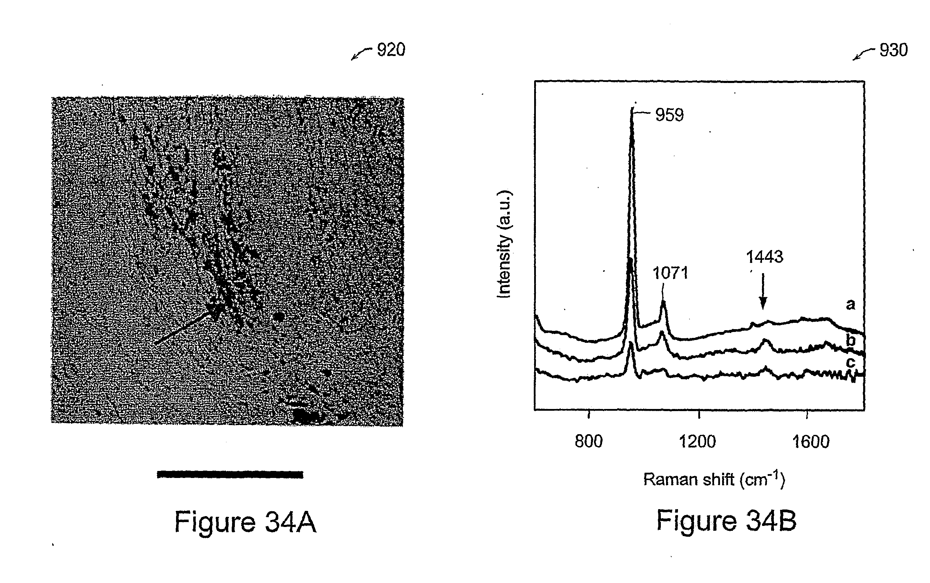

Figure 34A is a photomicrograph of an atherosclerotic plaque containing a calcification. In total, 15 calcium mineralizations in six coronary artery samples were studied. Raman spectra representing different stages of calcification in two atherosclerotic plaques are shown in Figure 34B. The main features of these spectra are 1071 and 959 cm$^{-1}$ bands attributed to $CO_3^{2-}$ (symmetric)/$PO_4^{3-}$ (asymmetric) and (symmetric) stretches, indicative of calcium carbonate and calcium hydroxyapatite, respectively. Large calcium mineralizations (Figure 34B, a and b) show spectral features different from those of minute punctate calcium mineralizations in the necrotic core (Figures 34B, c). The main difference is the presence of additional features in the spectra of the punctate calcium mineralizations attributable to lipids and/or phospholipids (1433 cm$^{-1}$), most likely due to the surrounding necrotic core.

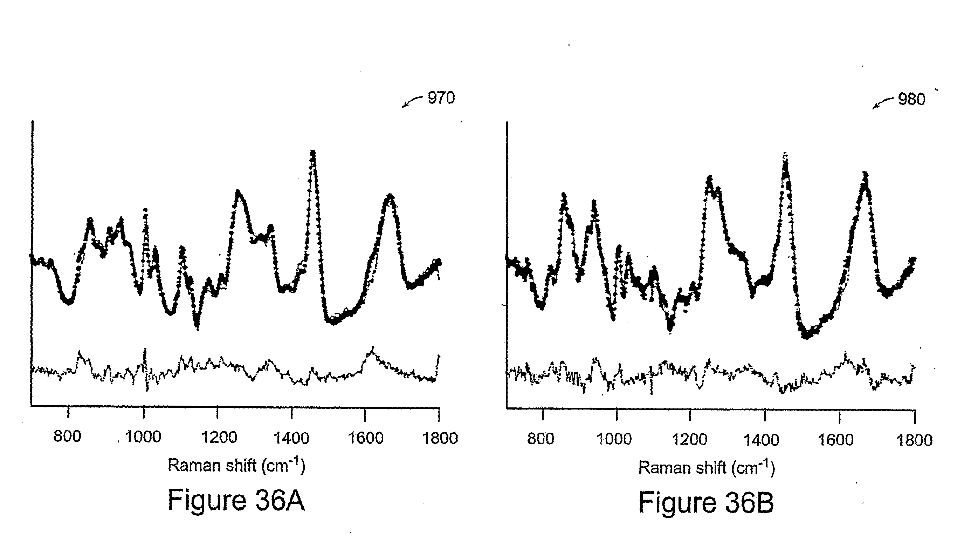

Using the basis spectra of pure chemicals as illustrated in Figure 35, the spectra of the individual morphologic structures were fitted in the biochemical model. Each panel in Figures 36A-H shows a Raman spectrum of one of the morphologic structures, and the result of the least-squares minimization fit of the biochemical model. Residuals (data minus the fit) are shown on the same scale. Because the Raman spectra from foam cells and necrotic core were very similar, only the fit results of the foam cells are shown. Judging from the residuals of the fits to the observed spectra, which are on the order of magnitude of the noise and show no consistent pattern from spectrum to spectrum, the Raman spectrum of each morphologic structure (panels A-H) is well described using the 12 biochemical basis spectra.

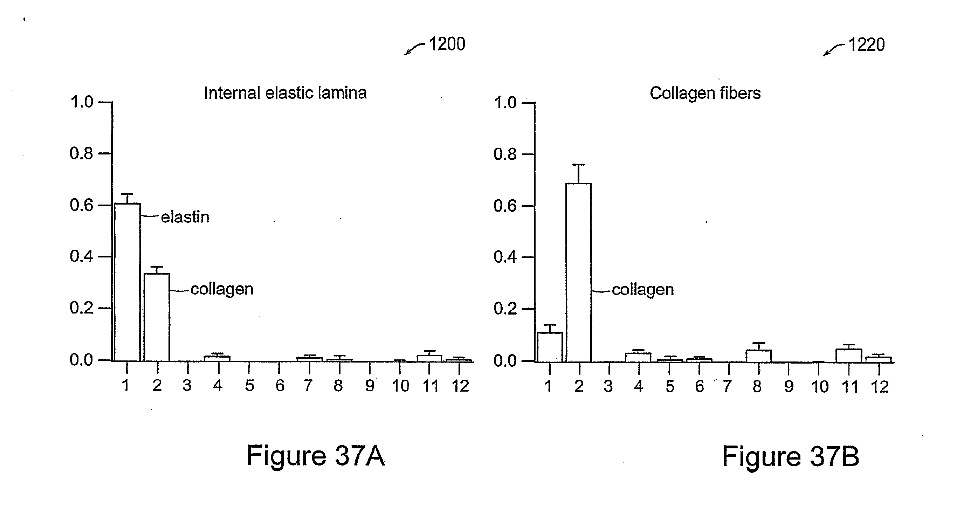

For each morphologic structure examined, the contribution of each biochemical component was determined. Figures 37A-H confirm that each morphologic structure has a characteristic biochemical composition. Generally, each morphologic structure is composed largely of one or two major biochemical components, combined with one or more less abundant biochemical components.

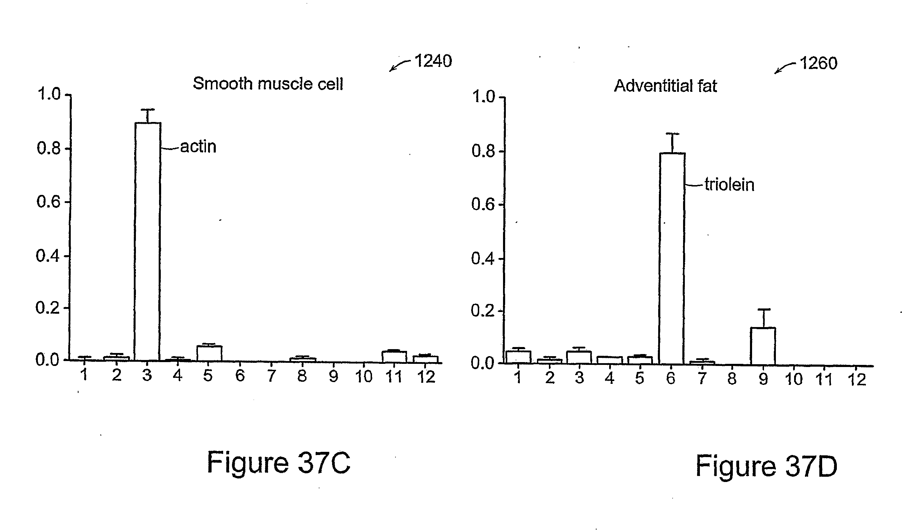

The internal and external elastic laminae (Figure 37A) are mainly composed of elastin with a smaller collagen component, whereas collagen fibers in both normal arteries and the fibrous cap of atherosclerotic lesions (Figure 37B) are mainly composed of collagen with a small elastin component. Smooth muscle cells (Figure 37C) were modeled almost entirely by actin and a small tropomyosin component. Myosin did not contribute at all.

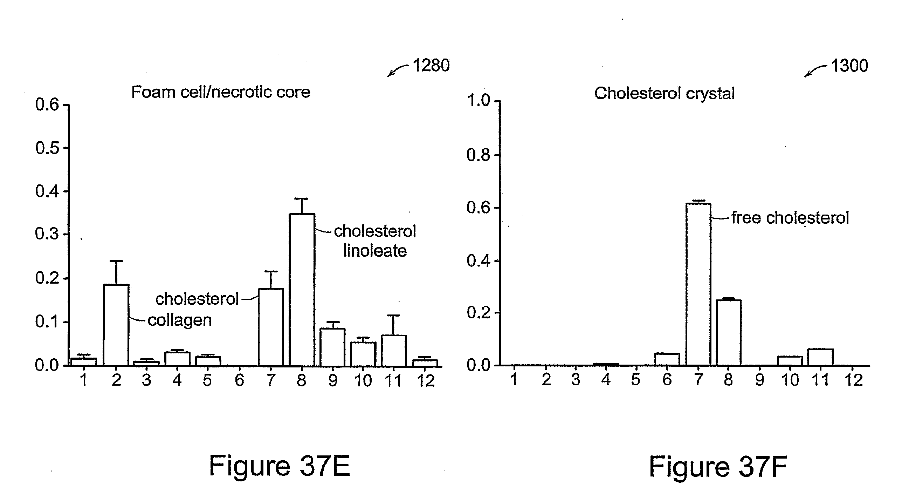

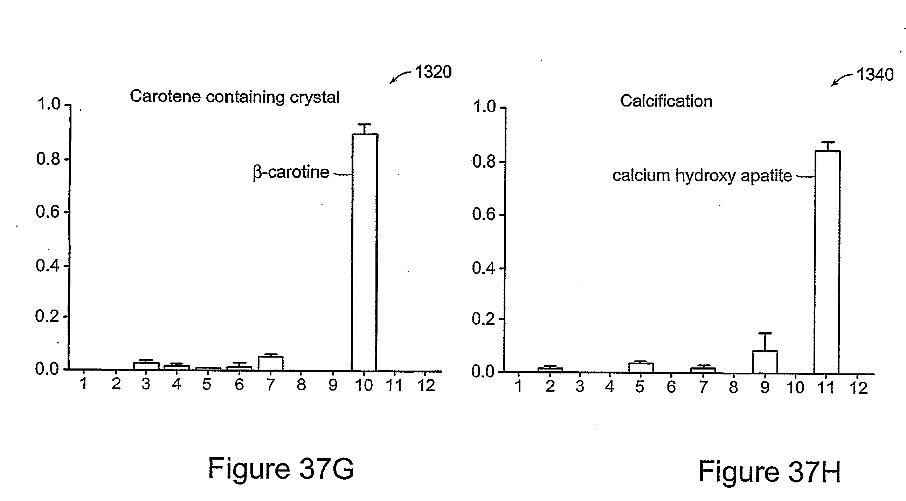

Adventitial fat cells (Figure 37D) contain almost exclusively triglycerides (triolein) with a small contribution of phospholipids (phosphatidyl choline). In contrast, foam cells and necrotic core (Figure 37E) contains mainly cholesterol esters (linoleate) and free cholesterol (monohydrate) at a ratio of about 2:1, with smaller contributions of collagen, phospholipids, and β-carotene. Foam cells appear similar in the current data and cannot be distinguished from necrotic core on the basis of their biochemical composition. However in a following assessment they can be distinguished. For example, the spectral feature at approximately 1750 cm$^{-1}$ can be used to distinguish the foam cells from the necrotic core. Cholesterol crystals (Figure 37F) contain free cholesterol and cholesterol ester at a ratio of about 3:1. The yellow crystals (Figure 37G) consist almost entirely of β-carotene, with a small contribution of cholesterol. This may indicate that these crystals are in fact cholesterol crystals that contain high concentrations of β-carotene. Calcium mineralizations (Figure 37H) are mainly composed of calcium hydroxypatite with small contributions of collagen, triglycerides, and calcium carbonate.

The presence of foam cells and other inflammatory cells may also play a role in plaque instability. Therefore, morphologic factors, such as the presence of crystalline-free cholesterol or foam cells, may be as important as biochemical composition in determining atherosclerotic plaque stability and progression.

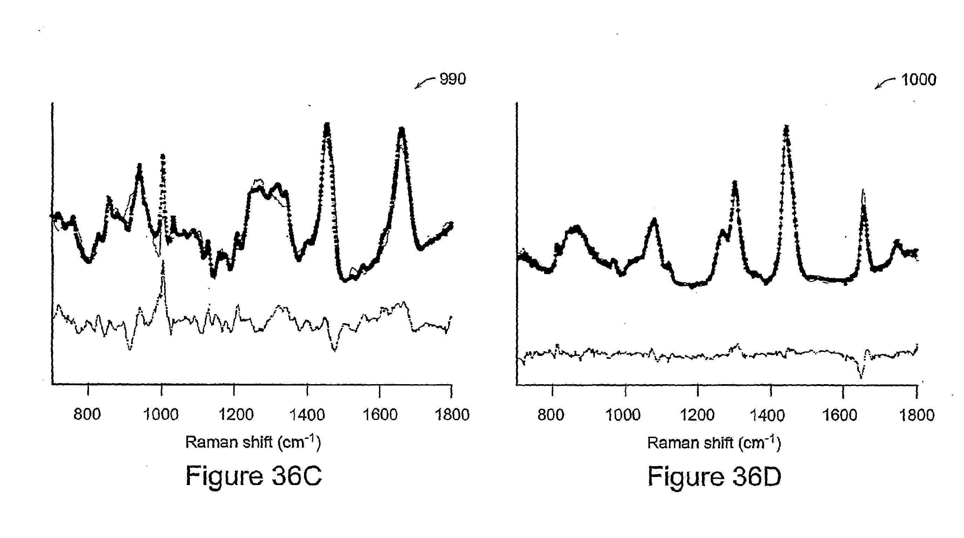

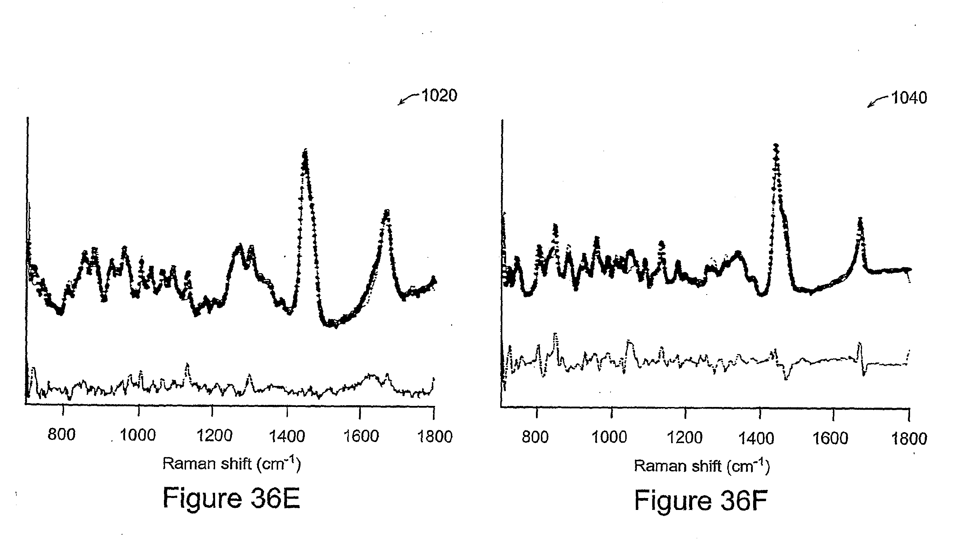

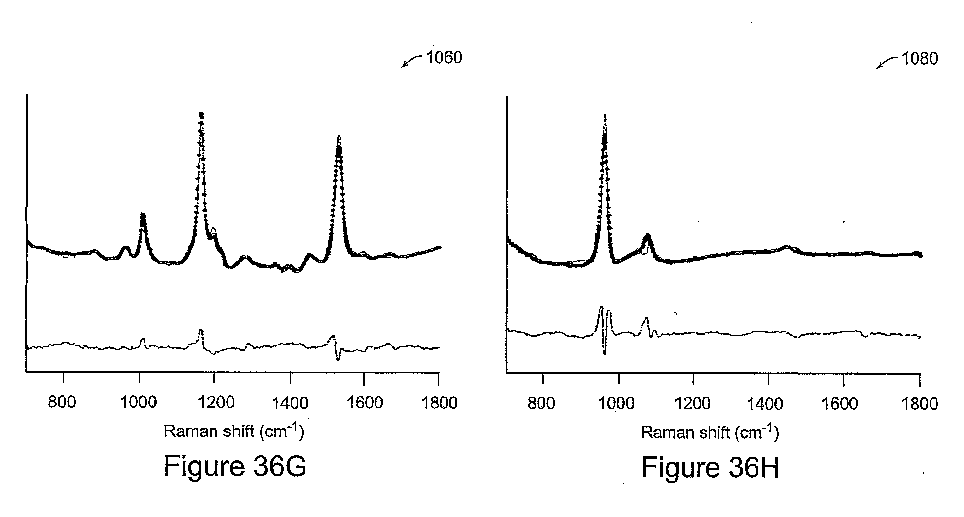

As was shown in Figure 36, the biochemical model of Figure 35 describes the spectrum of each morphologic structure well, which means that the most essential biochemical components are included in the reference. The biochemical composition of each structure, indicated by the fit contributions of the biochemical basis spectra to the morphologic structure spectrum, is very consistent (Figures 36A-H). The largest biochemical variations were found in foam cells, necrotic core, cholesterol crystals, and calcium mineralizations. The biochemical variations in both calcium mineralizations, cholesterol crystals, and β-carotene-containing crystals may be due to differences in their stage of progression (as reflected by size). The cause of the biochemical variations within foam cells and necrotic core (differences in collagen, β-carotene, and cholesterol esters) is less clear. Variations in the lipid composition of atherosclerotic plaques at various stages of progression have been described previously in in-vitro studies of homogenized or extracted tissues and cultured monocyte-derived foam cells. However, these biochemical data are the results of analysis of atherosclerotic plaque components in-situ, without the confounding effects of tissue preparation or in-vitro cell culture models. More detailed in-situ Raman microspectroscopy studies of foam cells and necrotic cores in atherosclerotic plaques at various stages of disease progression may help to further elucidate the origin of this variation.

Although the biochemical model did provide valuable information on the biochemical composition of the microscopic cellular and extracellular morphologic structures, it has its limitations. One of the major limitations of this model and/or reference was illustrated by the fits of the smooth muscle cell spectra. Previous in-vitro studies have shown that smooth muscle cells, which comprise the majority of the tunica media of muscular arteries such as the coronary artery, contain approximately three times more actin than myosin, but approximately equal amounts of myosin and tropomyosin. However, the fit contributions in the biochemical model indicated that smooth muscle cells contained almost exclusively actin, with a small amount of tropomyosin and virtually no myosin. These unexpected results may be due to conformational differences in spectroscopic characteristics of myosin between tissue-extracted myosin and intracellular myosin in-situ. In addition, as seen with the glycosaminoglycans, the contribution of weak Raman scatterers may be underestimated.

Observed variations may also be due to contributions of biochemical compounds that are not included in the reference. For example, only one class of collagen was included. This should not be a great concern, as there is little difference observed in the Raman spectra of the different classes of collagens in-vitro. However, there may be significant changes in the Raman spectra of collagen in-vivo due to increased crosslinking as atherosclerotic lesions progress.

Despite these limitations, the results of previous quantitative Raman spectroscopic biochemical analyses of normal and atherosclerotic arterial tissue, using the same biochemical model, compared well with standard analytical techniques. Previous studies have also shown that these quantitative Raman spectroscopy biochemical analyses could be used as the basis of a diagnostic algorithm that accurately classified arterial tissues as either nonatherosclerotic or calcified or noncalcified atherosclerotic plaque. The results of the preferred embodiments of the present invention indicate that a modification of the biochemical model can be used to perform a relative comparison of cellular and extra cellular morphologic components of normal and atherosclerotic arterial tissue. Furthermore, another preferred embodiment shows that these relative morphologic comparisons can be used as the basis for an algorithm that allows diagnosis of atherosclerosis in coronary arteries. This is the first step in developing a quantitative Raman spectroscopy morphologic analysis with the purpose to accurately classify normal arteries and atherosclerotic plaques ex vivo, and in the future to predict plaque stability and disease progression in-vivo.

Using the biochemical model reference of the preferred embodiment, confocal Raman microspectroscopy is illustrated to be used to perform an in-situ biochemical analysis of individual microscopic morphologic structures (such as foam cells and necrotic core) in intact arterial tissues that cannot be isolated or purified using conventional analytical techniques. Furthermore, the various morphologic structures have characteristic Raman spectra, which, as expected, vary little from structure to structure or from artery to artery, and can be used as basis spectra in a morphologic reference to perform a relative comparison of the morphology of normal and atherosclerotic coronary arteries ex-vivo. This nondestructive technique may ultimately be used to assess plaque stability and disease progression in humans in-vivo, as well as to study atherogenesis in animal models and lipid metabolism in cell cultures in-vitro.

The embodiments of the present invention interpret Raman spectra in terms of morphology. For example, the Raman spectra can be associated with a morphological structure, for example, a foam cell which can be associated with specific chemical compounds. Further, the number of spectra can be reduced, for example, from a large number of chemical spectra to only eight unique spectra associated with morphological structures thereby decreasing the error in the fit. The diagnostics that are available to identify and monitor vulnerable plaque using the optical fiber catheter system of the present invention include the use of chemical composition, information about the morphological structures, thickening of the intimal layer and the thinning of the overlying collagen layer. Preferred embodiments include the determination of the depth of collagen by measuring the percentage of collagen. Further, the presence of calcification is monitored and any edges are identified and located relative to the collagen as indicators of a potential rupture and blood clot. As discussed previously, the reduced fractional fit contributions of collagen fibers in non-calcified plaques is an indicator of unstable plaque.

Preferred embodiments implement an optical design to fully utilize system throughput by characterizing the Raman distribution from tissue. The embodiments optimize collection efficiency, minimize noise and have resulted in a small diameter, highly efficient Raman probe capable of collecting high-quality data in 1 second. Performance of the embodiments have been tested through simulations and experiments with tissue models and several in vitro tissue types, demonstrating that these embodiments can advance Raman spectroscopy as a clinically viable technique.

Raman spectroscopy is proving to be a valuable and accurate tool for diagnosing disease and studying biological tissue. Laser excitation is used to provide detailed information about vibrations and rotations of molecular bonds. Because each chemical moiety in a sample has a unique molecular structure, its composition can be evaluated through spectroscopic analysis of the inelastically scattered excitation light.

In vitro studies have established the medical potential of Raman spectroscopy. In fact, many diseases have been investigated because Raman spectroscopy has the ability to provide specific information about a wide variety of chemical and morphological constituents that cannot be obtained with other spectroscopic methods. For example, the rupture of unstable atherosclerotic plaques in coronary arteries accounts for the majority of fatal myocardial infarctions. It has been established that the likelihood of plaque rupture is related to chemical composition, and Raman spectroscopy may have a unique ability to differentiate the most culprit lesions on this basis. If this method can be successfully implemented clinically with real-time analysis, it can be used for a range of applications such as diagnosing cardiovascular disease and guiding effective therapy, or characterizing malignant tumors and ensuring their complete resection by monitoring surgical margins.

Many medical applications require remote sampling using optical fibers, where the size of the probe and fiber bundle is strictly limited by anatomic considerations. For example, the ability to clinically evaluate coronary atherosclerosis and breast cancer requires probes that are approximately 2 mm or less in diameter so they can be incorporated into standard cardiovascular catheters or configured for optical needle biopsy. In addition, data acquisition time must be limited to a few seconds at most.

With respect to coronary atherosclerosis, the detection of vulnerable atherosclerotic plaques is critical for the prediction and prevention of cardiac events. These vulnerable plaques occur in clinically silent vessels and can be characterized by biochemical changes, presence of foam cells, lipid pool, inflammatory cells, thin fibrous cap which is less than 65 $\mu$m in thickness and thrombosis. Preferred embodiments of the present invention use Raman probes to provide quantitative biochemical information and morphological analysis regarding the presence of the above mentioned factors to characterize vulnerable plaques. The use of the Raman spectra is spectroscopically advantageous at the use of narrow vibrational bands, are chemical specific and rich in information.

Diagnostically the use of Raman spectroscopy is advantageous as no biopsy is required; and it directly measures molecules in small concentrations, provides chemical composition and morphological features of the molecules. Raman spectroscopy can be used to evaluate plaque stability, monitor disease progression and evaluate therapeutic inventions by ascertaining plaque regression and restenosis.

There have been substantial advances in optical fiber probe designs over the past decade, indicating that Raman spectroscopy is a potentially useful clinical technique. Low-OH fused silica has been determined as the optimal fiber substrate for use in the near-infrared. The necessity of proper optical filters has been established and numerous probe configurations have been explored.

In vivo investigations, many using commercially available probes, have either been confined to skin and other easily accessible organs, or have used optical configurations which are not optimized for studying tissue. In addition to other difficulties, such as choice of excitation wavelength, sub-optimal probe designs result in collection times that are too long for practical clinical use. Thus, a key impediment to realizing the clinical potential of Raman spectroscopy is the development of small diameter optical fiber Raman probes capable of delivering excitation laser light to in vivo tissue, and efficiently collecting the Raman scattered light.

The preferred embodiments of the Raman probes include small (approximately 2 mm) probes that are flexible for accessing remote organs. The probes are able to collect high signal-to-noise ratio (SNR) spectra in approximately 1 second for accurate clinical application of the spectral models used for analysis. This is accomplished with safe levels of laser exposure and is accomplished by minimizing all sources of noise while maximizing throughput and efficiency.

There are several sources of noise which are minimized in preferred embodiments. Detector dark charge and read noise are reduced by using cryogenically cooled charge coupled device (CCD) detectors. The choice of excitation wavelength also influences the SNR. Excitation with 785 nm, as is done with many Raman probe designs, results in at least a four-fold increase in tissue fluorescence when compared to 830 nm excitation. This increased fluorescence adds significant shot-noise to the data. Although longer excitation wavelengths further reduce tissue fluorescence, the Raman cross-sections are simultaneously reduced because they depend on the excitation frequency to the fourth power. Furthermore, the absorption coefficient of water rapidly increases at longer wavelengths, thereby decreasing the penetration depth and attenuating the signal. Excitation wavelengths greater than 830 nm also prohibit the use of CCD detectors, thereby compromising the ability to collect an entire Raman spectrum with a single exposure.

A major source of noise specific to optical fiber Raman probes is the longrecognized problem of spectral background generated in the delivery and collection fibers themselves. This background is orders of magnitude larger than the signal from the tissue site being examined. It is composed of Raman light from the fused silica core, fluorescence from impurities and dopants used to produce fibers of a particular numerical aperture (NA), and signal from various jacket materials. Laser light in the delivery fibers generates an intense fiber background due to the long path length traversed in the fibers, typically three to four meters. This fiber spectrum is scattered from the tissue surface and gathered, along with the tissue Raman spectrum, by the collection fibers. The background often masks the tissue Raman signal which is generated from only approximately 1mm of sample due to the relatively short penetration of light into tissue. Laser light backscattered from the tissue also enters the collection fibers, producing additional fiber background and further compromising the quality of the tissue spectrum reaching the detector. In addition to obscuring and distorting the spectrum of interest, the intense fiber background adds shot-noise to the signal. This noise can often be larger than the tissue Raman bands, and it is therefore necessary to remove as much of the background as possible by using optical filters.

The other consideration in the design of the probes of the preferred embodiments, optimizing throughput and maximizing collection efficiency, has two components. The first concerns the inherently weak nature of the Raman effect. Approximately only one out of every billion excitation photons are converted into a Raman photon. It is therefore critical to design a high-throughput optical system in order to collect signals with sufficient SNR for accurate analysis in a clinically realistic timeframe. The second component is concerned with the optical characteristics of the tissue itself The signal of interest is directly attenuated via absorbance of the excitation laser and the generated Raman light. Furthermore, collection of the Raman light is confounded by light scattering, which causes the photons to be widely diffused over large areas and angles. Thus, the simple probe designs used for other types of spectroscopy, or for studying non-turbid samples, are not ideal for this application.

The preferred embodiments include an optical fiber Raman probe which removes a majority of the optical fiber background, employs 830 nm excitation, maximizes signal collection from the Raman source generated in the tissue, allowing data collection in a few seconds or less (1 or 2 seconds), and operates at safe fluence levels while limiting the rigid distal tip to less than a few mm in length and less than about 2 mm in diameter.

Analysis indicates that the detected fiber spectral background is produced equally in both the excitation and collection fibers. Two different filters are required at the distal end of the probe to suppress the unwanted signal: one for delivery and one for collection. Delivery fibers are terminated with a short wavelength-pass or band-pass filter that transmits the laser excitation light while blocking the longer wavelength spectral background from the fibers. The collection fibers are preceded by a long wavelength-pass filter or notch filter, which transmits the tissue Raman spectrum while blocking laser light backscattered from the tissue. The filters perform these functions over the range of angles corresponding to the NA of the fibers they serve.

In order to accommodate the.filters into the distal end of the probe, the preferred embodiments include a filter module. This module consists of a rod carrying a short-pass dielectric filter coating on one plane face, fitted into a tube carrying a long-pass dielectric coating, also on one plane face. Rods and tubes that are made of either sapphire or fused silica are used in the preferred embodiments which are separately coated with their respective filters prior to assembly and are fabricated, for example by, Research Electro-Optics, Inc., of Boulder, CO. The rod is wrapped or coated with a thin sheet of metal to prevent cross-talk by providing optical isolation between the components. The module is placed at the distal end of the probe between the fiber bundle and collection optics. Filter performance curves used in the Raman probe of the preferred embodiments are shown in Figure 5 (0 cm-1=830 nm). Peak transmissions are typically greater than 90%, while rejection of the unwanted light is greater than 96%.

In general, the throughput (or étendue) of an optical system is given by the product of its collection area (A) and projected solid angle (Ω'), where

$$\Omega' = \pi\, sin^2(\theta) \qquad\qquad (5)$$

and is evaluated for the half-angle (θ) of collection, measured with respect to the optical axis. Neglecting fiber coupling limitations along with reflection and transmission losses of all optical components, factors which are easily optimized, the system's collection ability is limited by the throughput of its most restrictive element, and this quantity is conserved throughout the system.

In ideal spectroscopy systems, throughput is determined by the spectrograph/CCD detection equipment. In preferred embodiments, the spectrograph has an NA=0.278 (Holospec f/1.8i. Kaiser Optical Systems, Inc., Ann Arbor, MI), such that $\Omega'_D$=0.225sr. The entrance slit height is 8 mm. This is coupled to a backilluminated, deep depletion CCD detector (Spec-10: 400BR, Roper Scientific, Trenton NJ) that also has a height of 8 mm, and does not therefore compromise throughput. To achieve sufficient spectral resolution (approximately 8 cm$^{-1}$) for biological Raman spectroscopy a 0.2 mm slit width is used at the entrance to the spectrograph. Thus, the maximal area of collection AD=1.6mm$^2$, resulting in a theoretical maximal throughput of $A_D\Omega'_D$=0.360mm$^2$-sr for the detection system. Detection of light from a Raman source is limited by this product and collection optics are designed to conserve system throughput.

Diffuse scattering in the tissue results in emission of the Raman light over a large area and 4π solid angle, each with a particular distribution. Optimizing signal collection from such a source requires two steps. First, the distribution of the Raman light emerging from the turbid medium is determined. This distribution defines the potential light collection efficiency for the given Raman source, within the throughput constraints, as the area (or angle) of collection is varied. These properties are used to determine the optimal trade-off between collection solid angle and area to maximize efficiency for design of the collection optics.

For an optical fiber probe, the optics in the distal end of the probe is also designed to transform the Raman scattered light for efficient coupling into the collection fibers, which is chosen to have the same NA as the spectrograph. Furthermore, in order to optimize signal collection it is necessary to maximize the area of the distal probe tip utilized for collection fibers. This is achieved by using a single central excitation fiber, surrounded by as many closely packed rings of collection fibers as possible, up to what can be accommodated by the spectrograph/CCD and incorporated into the probe diameter. The circular bundles of fibers in the distal end are then re-shaped at the proximal end into a linear array for coupling to the spectrograph.

Proper choice of excitation optics is also critical. The intensity of the background generated in the fused silica optical fibers is proportional to the square of the NA, but relatively independent of the core diameter. Therefore, although it is desirable to match the NA of the collection fibers to the spectrograph, it is preferable to use an excitation fiber with a lower NA. Reducing this NA also provides decreased beam divergence at the distal end, thereby improving the shortpass filter performance. Results have indicated that fibers with very low NA (0.12) exhibit substantially increased broadband fiber background, presumably generated from doping materials used in the cladding. Thus, using a moderate NA (0.22) is most effective. The appropriate excitation fiber diameter can then be chosen to ensure safe illumination fluence at the tissue while limiting the spot size to facilitate efficient collection.

Determination of optimal collection geometry requires characterization of the distribution of Raman light from the tissue. This source has a given brightness B(r,θ) emerging from the surface with a convoluted dependence on both source radius, r, and emission angle, θ. The total amount of Raman light available for collection from the tissue is given by

$$I_{Raman}(r,\theta) = \iint\limits_{A_S\Omega_S} B(r,\theta)\,dA\,d\Omega, \qquad\qquad (6)$$

with dA=(2πr)dr and dΩ=2πsin(θ)dθ. The integrals are carried over the entire area and solid angle of the source, the latter of which is limited to 2π for backscattering geometries.

If it is assumed that B(r,θ) can be independently separated into the discrete distributions $B_1(r)$, a function only of radial distance from the excitation light, and $B_2(\theta)$, dependent only on the angle from the surface normal, then each can be experimentally measured. These distributions are used to approximate the light emitted from the source such that

$$I_{Raman}(r,\theta) \approx \int_{A_S} B_1(r)dA \int_{\Omega_S} B_2(\theta)d\Omega = I_1(r)I_2(\theta) \tag{7}$$

where $I_1(r)$ and $I_2(\theta)$ are the integrated radial and angular distributions emanating from the tissue.

The optical system efficiency for this Raman source is calculated by integrating the radial and angular brightness over the properties of the collection optics, normalized by the total light emitted from the source and constrained by throughput conservation. This resulting efficiency curve

$$\eta_T(r,\theta) \approx \eta_1(r)\eta_2(\theta) = \frac{\int_{r=0}^{r_c} rB_1(r)dr}{\int_{r=0}^{\infty} rB_1(r)dr} \bullet \frac{\int_{\theta=0}^{\theta_c} sin(\theta)B_2(\theta)d\theta}{\int_{\theta=0}^{\pi/2} sin(\theta)B_2(\theta)d\theta} \tag{8}$$

is used to guide design of the probe optics by specifying the optimal trade-off between collection radius and solid angle. The angular efficiency $\eta_2(\theta)$ can be transformed to a function of radius, $\eta_2(r(\theta))$ by the employing throughput conservation, resulting in a single variable function $\eta_T(r)$ for the total collection efficiency.

Guided by the application diagnosing atherosclerosis, the radial and angular distributions of Raman light from arterial tissue were examined. Normal arterial tissue was used because it typically exhibits the weakest signal, as compared to other arterial disease states, due to it's optical properties (e.g. scattering and absorption coefficients) and relative Raman cross-sections.

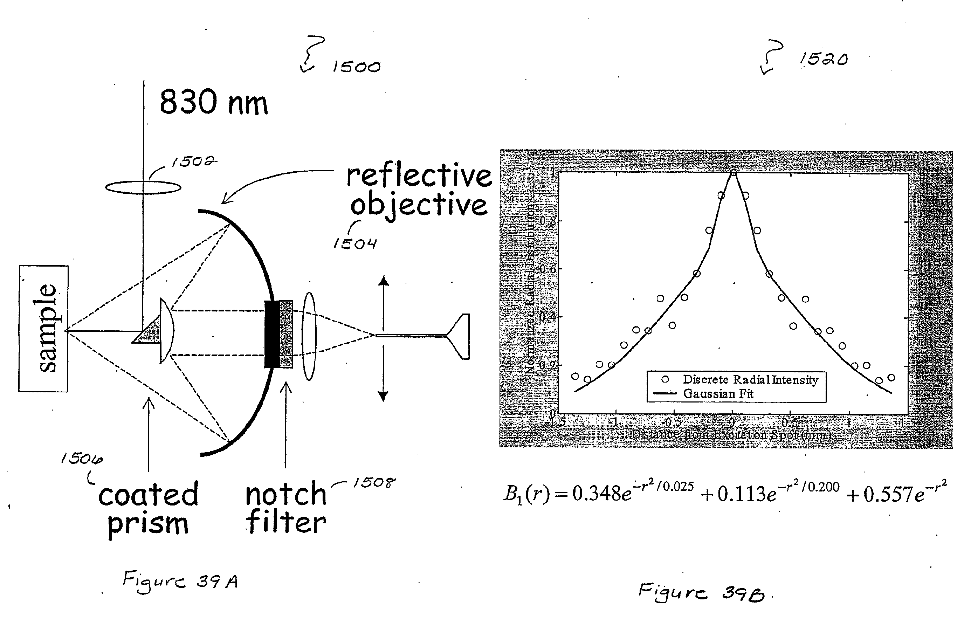

Characterization of the spatial distribution $B_1(r)$ of Raman light was determined in preferred embodiments of the present invention. Briefly, 830 nm excitation was focused and directed to the sample via a small prism. The excitation spot diameter was approximately 100 $\mu$m and the sample was held in a quartz cuvette containing phosphate buffered saline (PBS, pH=7.4). The backscattered light was collected by a Cassegrain objective with an angular range of 14° to 33°. This entire range of Raman light was collected, collimated and notch filtered to reject the Rayleigh scattered light. A single 100 $\mu$m core optical fiber was translated laterally across the beam with a step size of 250 $\mu$m to collect discrete spatial regions of the image. Accounting for the magnification from the objective, this corresponds to a step size of approximately 104 $\mu$m at the sample surface. The fiber was coupled into an f/1.8 spectrograph and the light was dispersed onto a CCD detector. The intensity of the 1450cm$^{-1}$ Raman band from -CH$_2$ bending modes was integrated, normalized to the maximum signal, and plotted as a function of radial distance from the excitation source. The results on either side of the excitation beam were nearly symmetrical and the average $B_1(r)$ for the two sides is presented in Figure 39B (circles).

This radial distribution was optimally fit with a multi-Gaussian (Figure 39B, line), resulting in

$$B_1(r) = 0.348e^{-r^2/0.025} + 0.113e^{-r^2/0.200} + 0.557e^{-r^2} \tag{9}$$

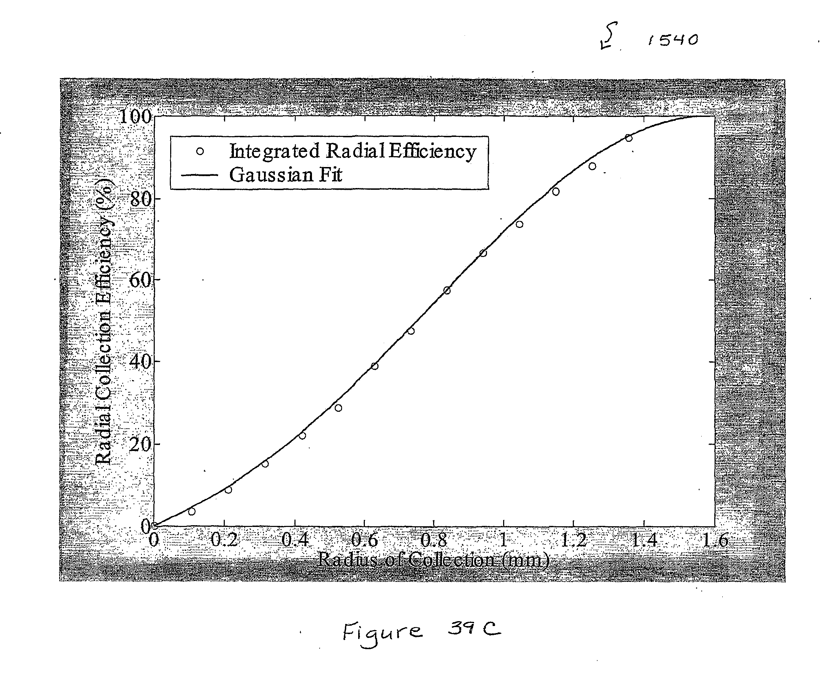

with r in mm. It is likely that the narrow distribution represented by the first term is related to Raman light generated by the ballistic laser light producing the most intense Raman energy distribution. The other terms account for diffused light which is also influenced by the layered structure of arterial tissue. This data is then integrated and normalized to determine the radial collection efficiency $\eta_1(r)$, shown in Figure 39C as a function of distance from the excitation beam (circles), along with a least-squares fit demonstrating the expected Gaussian dependence (line).

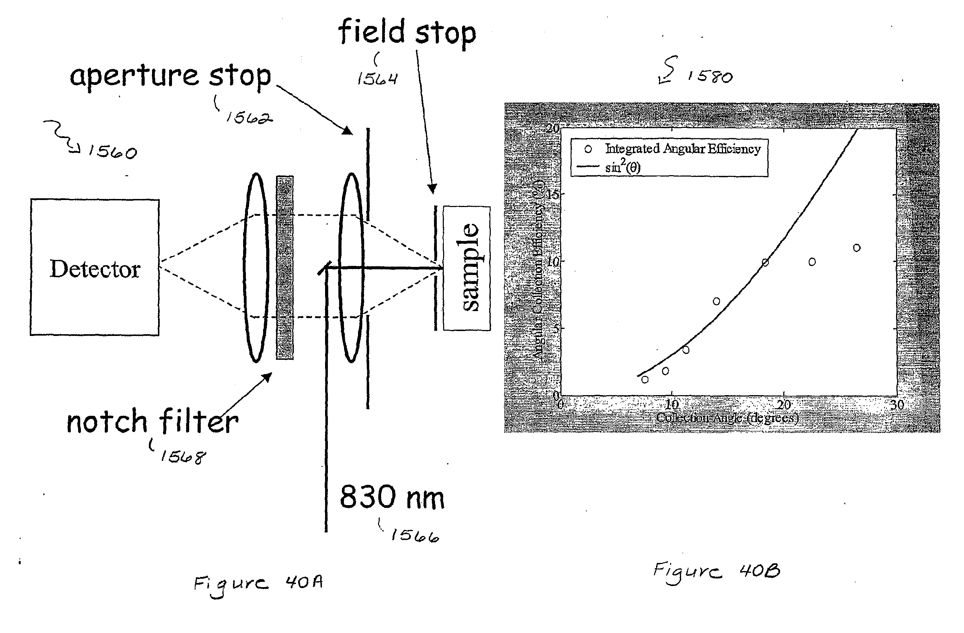

The angular distribution was determined with a slight modification of an open-air optics Raman system. $I_2(\theta)$ was measured directly, rather than measuring the discrete angular distribution $B_2(\theta)$. Briefly, 830 nm excitation light was incident normally upon the tissue by a small mirror between the collection lens and the sample. The backscattered Raman light was collected by an f/1.2 camera lens which collimates the beam before being notch filtered and then focused onto an f/4 spectrograph via a f/#-matched lens for detection by a CCD detector. The excitation light was focused down to approximately 100 $\mu$m diameter and the collection radius was approximately 1 mm. The collection lens was preceded by an aperture-stop iris allowing for variation of the collection angle and direct measurement of the integrated angular Raman distribution. Figure 40B plots the experimentally determined $\eta_2(\theta)$ of Raman light from tissue (circles). The distribution plateaus around 20° due to the limited angles collected by the lens. Light emerging from tissue generally follows a cos($\theta$), or Lambertian, dependence, where $\theta$ is the angle with respect to the surface normal. The integrated distribution should therefore have a sin$^2(\theta)$ dependence, which is also plotted in Figure 40B (line), demonstrating reasonably good agreement between experiment and theory over the range of angles collected.

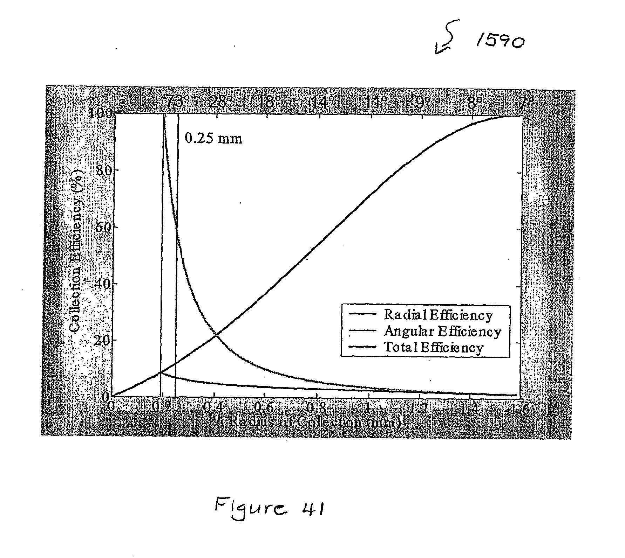

Taking the product of $\eta_1(r)$ and the transformed $\eta_2(\theta) \rightarrow \eta_2(r(\theta))$ from Figures 39C and 40B, results in the

efficiency curve $\eta_T(r)$ (Figure 41) for this particular combination of system throughput and tissue. Optimal efficiency of 8.62% occurs at a collection radius of 0.191mm and corresponding 90˚ angle. This indicates that it preferable to collect the full angular range of Raman light from the most intense area of illumination, rather than collecting a lower range of angles while extending to the weaker tails in the edge of the distribution.

The results of the Raman source characterization studies are then incorporated into an optical design code provided by Zemax v.10.0, Focus Software, Inc., of Tucson, AZ, to determine appropriate optics for maximal signal collection and transformation of the gathered light for efficient coupling into the optical fibers. Although it is possible to design sophisticated optics to perform close to these specific parameters, the spatial constraints imposed by the given application would make construction prohibitively difficult. In fact; investigations with Zemax indicated that the use of a simple ball lens results in reasonable performance if a high-index of refraction is used. Several substrates were investigated and it was determined that most high-index of refraction glasses are extremely fluorescent due to doping materials. However, sapphire, whose refractive index is 1.77, allows for wide-angle collection. Sapphire exhibits no fluorescence, has only a single, sharp Raman band, and is optically clear throughout a very broad wavelength region. In addition, sapphire is extremely hard, thus making it an excellent choice for a multiple use Raman probe.

The resulting Raman probe design is presented in Figures 4A and 4B. The left hand side shows a longitudinal view of the probe tip, while the right hand side shows a cross-sectional view at the level of the fiber-filter interface. There is a central excitation fiber with an aluminum jacket for optical isolation to prevent cross-talk with the collection fibers. This fiber is placed in registration with the short-pass excitation rod. The rod is placed inside the long-pass collection filter tube with the two being optically isolated by a metal sleeve. The excitation fiber is then buffered out to ensure proper alignment of the collection fibers, which are registered with the center of the long-pass filter tube. The central excitation fiber has a 200 $\mu$m core with a 0.22 NA. The collection fibers are also 200 $\mu$m core, but have a 0.27NA which is closely matched to that of the spectrograph. The filters are secured to the fibers with an index-matching optical cement and the entire fiber bundle/filter module is encased with black Teflon for binding and protection. The probe length is 4 meters.

The filter rod and tube are 1mm in length ensuring proper spatial placement of the sapphire ball lens. This geometry addresses two considerations. First, at this fiber-lens separation, the excitation light is roughly collimated and not focused to a tight spot on the tissue, thereby reducing the energy density incident upon the sample and preventing possible damage. Second, excellent coupling of the Raman scattered light into the collection fibers is ensured because the ball lens transforms the large angular distribution emerging from the tissue into a well collimated beam that falls within the fiber NA. The ball lens is secured into a crimped stainless steel tube with epoxy, which ensures that no fluid leaks into the tip. The stainless steel tube is then affixed to the fiber-bundle/filter assembly. In order to maximize the ball lens collection efficiency, there are no adherents used on the inner surface.

The total diameter of this probe is under 3 mm. The current size-limiting factor is the diameter of the ball lens, which is 2 mm to accommodate the entire width of the filter tube. This filter size was chosen because this geometry is used to construct Raman probes with two rings of collection fibers (a total of 27 fibers), which more fully utilizes the spectrograph throughput. In practice, a single-ring probe can be used because it provides excellent signal collection and is much more flexible and easier to construct. Recent studies have shown that the probe diameter can be reduced without significantly degrading the collection efficiency. The diameter of the central collection rod was chosen to be 0.55 mm for ease of construction. All components of the probe are constructed of medical grade materials that can withstand standard cold gas ethylene oxide sterilization for surgical procedures.

The Raman probe design was tested through simulations and experimentally. The simulated experiments were performed with a Zemax model of the probe to investigate two aspects of the probe design. First, excitation spot diameter was investigated to ensure safety. Second, collection efficiencies for various Raman sources were examined to determine probe performance over a range of conditions.

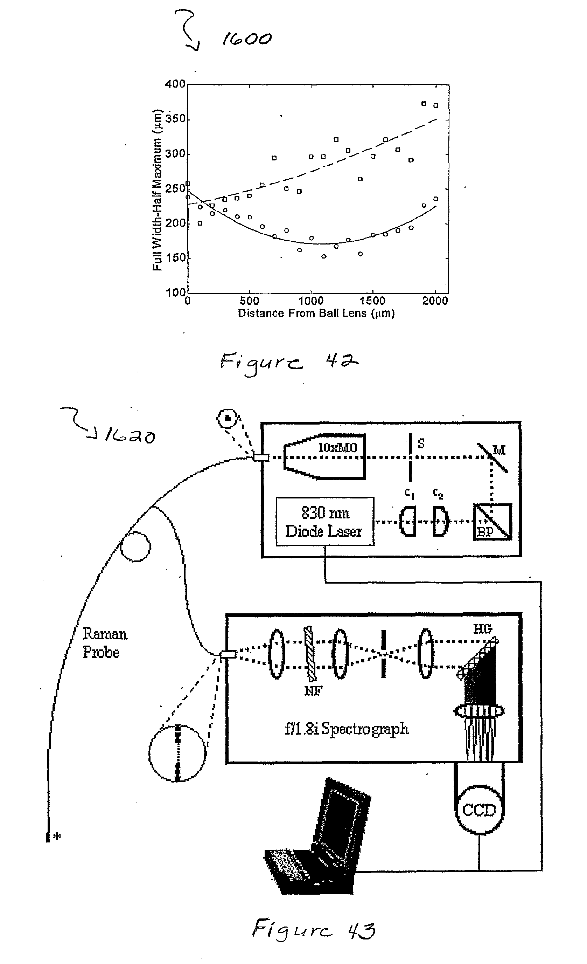

Results of the excitation spot size simulations are shown in Figure 42. Two configurations were investigated, one with the probe placed in air (circles) and one with the probe submerged in a simulated tissue model (squares), the more likely clinical geometry. The tissue model was constructed with the index of refraction of water and scattering properties for arterial tissue: g=0.9, mean-free path=0.27 mm. As can be seen from the figure, when the probe is in air there is a slight focusing to a full-width half maximum (FWHM) of approximately 175 $\mu$m approximately 1 mm away from the lens. However, in the scattering case the beam begins to diverge immediately from the surface of the ball lens, never falling below a FWHM less than 200 $\mu$m. This spot size produces fluences well below any reported damage thresholds with laser powers and exposure times that are used in a clinical setting. Data collection protocols are designed to use approximately 100 mW excitation for times less than 5 seconds, thus producing fluences much below what are typically reported.

Collection efficiencies were determined from the Zemax model in a similar way (Figure 14). Lambertian sources of various radii were placed in contact with the probe and the percentage of light emerging from the proximal end of the collection fibers was measured. For these simulations, a model of the dual-ring probe with 27 collection fibers was implemented because this more fully utilizes the throughput of the spectrograph. Again, two situations were investigated. The first had the source in contact with the probe, but with the lens maintained in air so that the external surface of the ball lens does not experience any index matching (circles with solid line). The second also had the source in contact with the probe, but now the lens and source were both submerged in the simulated tissue model described above (circles with dashed line). The probe exhibits high collection efficiencies (up to 35%) for small sources, but the efficiency falls off as the source becomes larger. The tissue model results were also excellent, producing efficiencies about 1.75 times less than for when the probe was not index matched.

An additional configuration in accordance with another preferred embodiment was also investigated, wherein the measured Raman distribution from the source was modeled and placed at the end of the probe. This resulted in a collection efficiency of 3.5% and 1.7% for the air and tissue interfaces, respectively. The maximal collection efficiency of 8.6% results from fully utilizing the throughput, however by using optical fibers only 53% of the spectrograph slit area is used. Therefore, the maximal collection efficiency expected is 4.6% showing that the ball lens is performing close to the peak. By multiplying the efficiency curve by the same 53% caused by the reduced throughput, one can see that the ball lens efficiency indicates a collection radius of 0.27 mm which corresponds to a collection angle of 45˚. Although there is room for improvement, this is an excellent collection efficiency and the ease of implementation is very practical.

The performance of the Raman probe was experimentally tested in three ways. First, various known Raman scatterers were examined to assess filter performance. Second, tissue phantoms were developed to evaluate the effects of scattering and absorption on signal and background collection. Finally, in vitro spectra of artery and breast tissue were collected and evaluated with spectroscopic models.

A schematic of the experimental system used in these investigations is shown in Figure 43. Light from an 830 nm diode laser (Process Instruments, Salt Lake City, UT) is collimated by two cylindrical lenses (c1, c2), directed through a bandpass filter (BP, Kaiser), redirected by a gold coated mirror (M) and focused onto the Raman probe excitation fiber by a 10x microscope objective (Newport, Irvine, CA). The proximal linear array of collection fibers from the Raman probe are input to the f/1.8 spectrograph which collimates the light before it is notch filtered (NF), focused onto a slit and re-collimated for dispersion by the holographic grating (HG). Finally, the dispersed light is focused onto a liquid nitrogen cooled, back-illuminated, deep depletion CCD detector, which is interfaced with a laptop computer. A slit (S) allows the laser excitation out of the probe during data acquisition. This reduces laser exposure to the patient and increases safety for the users. Further, the laser interfaces with the computer for more accurate control of the laser power, monitoring of laser power and incorporation of a feedback loop that automatically sets the power output from the probe to the set level independent of the system optics alignment.

Tissue phantom studies were designed to mimic the range of scattering and absorption properties of arterial tissue. Samples were prepared using a combination of monodispersed 1.03 $\mu$m latex microspheres (Duke Scientific Corp., Palo Alto, CA) for scattering, hemoglobin (Sigma, St. Louis, MO) and India ink (Triangle Biomedical Sciences, Durham, NC) for absorption, and de-ionized water. A stock solution of $NaClO_4$ was added to the samples with a constant concentration as the target Raman molecule. Various combinations of the constituents were mixed to produce 9 phantoms of constant volume and $CLO_4^-$ concentration with absorption and reduced scattering coefficients of 1.31, 1.79 and 2.25cm$^{-1}$ and 22, 29 and 36cm$^{-1}$, respectively. Concentrations to produce these optical properties were determined with a modified version of the Mie code of Bohren and Huffman.

The phantoms were placed in 2" deep, 3/4" wide glass vials and the probe tip was submerged just under the surface of the liquid for sampling. The sample was continually circulated by a magnetic stir-bar to prevent settling of the microspheres. Spectra were collected using 100 mW excitation with the dual-ring Raman probe for a total integration time of 10s. The 928cm$^{-1}$ band of $ClO_4^-$ was integrated to determine the Raman collection ability of the probe under these varying conditions. The probe background was assessed by examining the intensity of the maximum signal collected (approximately 420 cm$^{-1}$). Alternative methods for assessing background integrate over the 800 cm$^{-1}$ Raman band from the quartz background, or the 750 cm$^{-1}$ Raman band produced by the sapphire ball lens. All of these produced similar results.

Finally, in vitro tissue specimens were examined with the single-ring Raman probe using 100 mW excitation power and collection times ranging from 1 to 60 s. Samples of aortic tissue were collected post-mortem, while breast samples were collected during surgical resection. Samples were snap-frozen in liquid nitrogen immediately after being harvested, and stored at -85˚C until the time of examination. Samples were allowed to passively warm to room temperature in a PBS bath prior to examination. Spectra were corrected for filter and CCD spectral response using a tungsten white light source. The remaining fiber background was removed by subtracting the signal generated by directing the excitation light at a roughened aluminum surface. Tissue fluorescence was removed by subtracting a 5th-order polynomial. Finally, the spectra were fit with spectroscopic models developed in the laboratory. The breast data was fit with the model described hereinafter while the artery spectra were fit with a morphological model. Residuals are calculated as the data minus the fit and are shown on the same scale.

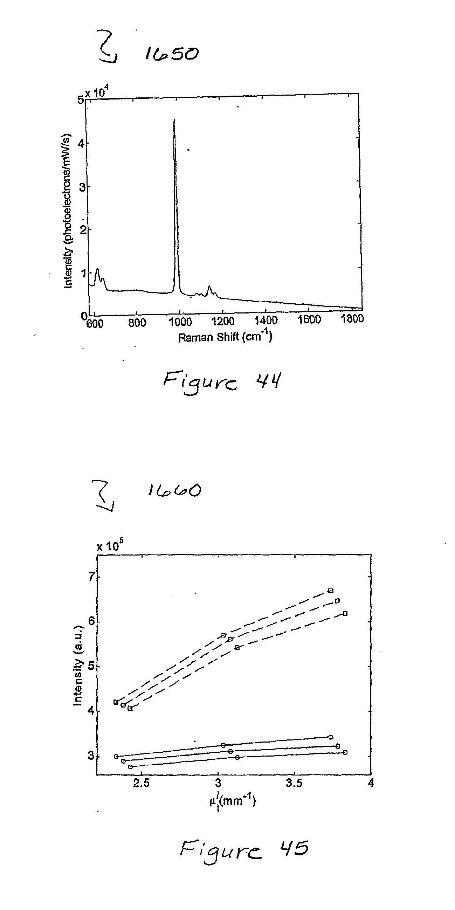

For biological tissue spectroscopy Raman features from 600-1800 cm$^{-1}$ are important. Figure 44 shows the Raman spectrum of packed BaySO$_4$, a well characterized Raman scatterer. Unlike liquid samples, which typically generate little detected fiber background because there is minimal backscattering, BaSO$_4$ is highly reflective when packed and generates intense fiber background in an unfiltered probe. This spectrum demonstrates the effectiveness of the filter module and optical isolation of the probe because there are almost no detectable features of the fiber background above 550 $cm^{-1}$, other than a slightly increased sloping background. Even the intense silica band at 800 $cm^{-1}$ is not discernable.

Results of the tissue phantom studies are presented in Figure 45. The signal from perchlorate (circles and solid lines) and from the probe background (squares and dashed lines) are plotted as a function of transport length. The plotted lines depict constant absorption and are drawn to demonstrate how signal collection increases as scattering increases. Conversely, the collected signal decreases with increasing absorption for a given scattering value. Similar trends are seen in both fiber background and the Raman signal, however the effects of scattering are more dramatic for the background.

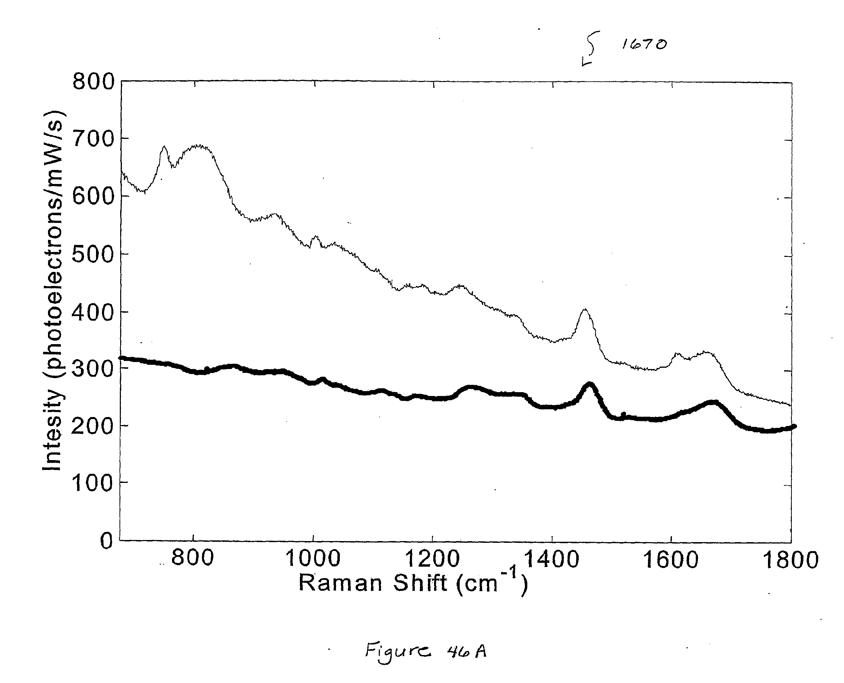

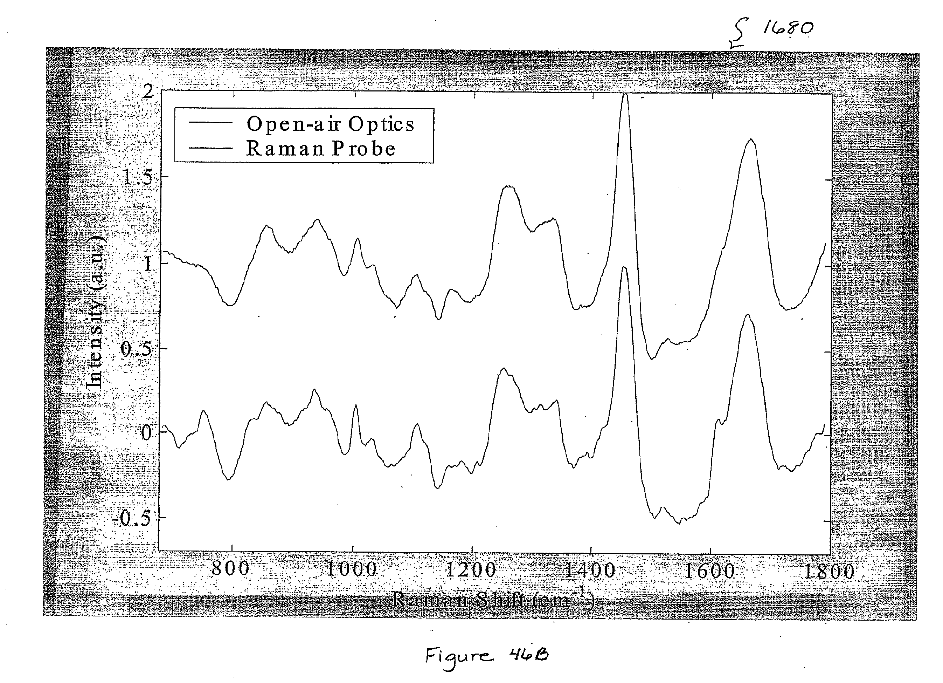

Figures 46A and 46B are a comparison between the single-ring Raman probe performance and the experimental system previously described, by looking at normal aorta, a tissue type which shows very little variation from site to site. The experimental system is an open-air optics configuration, unconstrained by the demands of micro-optics, and has been used to develop many successful Raman spectral models.

Figure 46A shows data taken with the open-air optics system (dots) and the single-ring Raman probe (line) with equivalent excitation powers and collection times. The data has been corrected for systematic spectral response and CCD detector gain. Although it is difficult to resolve the Raman bands over the tissue fluorescence background, observation of the 1450 $cm^{-1}$ band of -$CH_2$ bending, or the 1004 $cm^{-1}$ band due to phenylalanine indicates slightly increased signal collection from the Raman probe. There is still some evidence of probe background observed in this data despite the efficient filtering in the Raman probe because, unlike with $BaSO_4$, the Raman signal from tissue is extremely weak. However, Figure 46B shows the results after subtracting the fiber background and tissue fluorescence. All spectral features of fiber background have been accurately removed, and these spectra of normal aorta look nearly identical, other than the peak at 750 $cm^{-1}$ due to Raman scattering from the sapphire ball lens, and a small peak just below 1600 $cm^{-1}$ from the epoxy used to secure the lens. Despite the slightly increased background in the raw data from the Raman probe, on average the two processed spectra have the same SNR because of the increased collection efficiency of the probe. The dual-ring version of the Raman probe shows greatly enhanced performance over the experimental system.

Here weakly Raman scattering normal aorta has been evaluated because it is homogeneous. Experiments on more highly scattering tissue suggest that the probe shows even better performance over the experimental or laboratory system than for normal tissue. However, these differences are difficult to directly compare because of tissue heterogeneity.

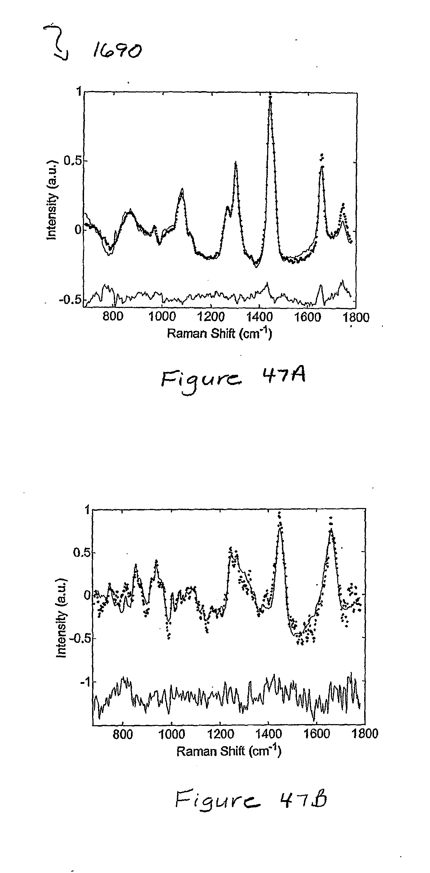

The Raman probe in accordance with preferred embodiments provides the ability to collect interpretable spectra of tissue in clinically relevant timescales. Figures 2A-2C and 47A-47B show processed spectra collected with the Raman probe in only 1 second with 100 mW excitation. Data are presented in dots, with the model fits shown in the overlapping lines and the residuals plotted beneath on the same scale. Figure 47A shows a spectrum of normal breast tissue, while 47B shows that of a malignant breast tumor. Even for the spectra with decreased signal collection, i.e. normal aorta and malignant breast tumors, model fits are excellent and all features remaining in the residual are noise.

For optical fiber probes, maximizing signals entails optimizing collection efficiency within the constraints of the spectrograph/CCD detector system in accordance with a preferred embodiment of the present invention. This is done by maximizing the area of the probe available for collection of Raman light, and characterizing the Raman source for proper design of collection optics. Minimization of noise also requires several considerations in accordance with a preferred embodiment of the present invention. First, the inherent noise sources from the CCD detector are reduced by using cryogenically cooled, back-illuminated detectors. Second, the excitation wavelength was carefully selected to minimize shot noise from tissue fluorescence. For biological samples, 830 nm excitation has proven to be ideal. Finally, in the case of fiber optic probes, the fiber background is eliminated as much as possible to reduce its associated shot noise.

The dual purpose filter module employed in Raman probes in accordance with a preferred embodiment of the present invention effectively reduces the fiber background to a level where there is minimal spectral distortion once the remaining background is removed. High quality spectra of several tissue types can be collected in only 1 second using excitation powers well below the tissue damage threshold. The magnitude of the residuals from the fits illustrated in Figures 2A-2C and 47A-47B are dependent upon the intensity of the Raman signal collected from the tissue, but in most cases they are purely noise and show no spectral structure. The only exception to this is for the non-calcified atherosclerotic plaque, where there is some minor structure in the residual due to discrepancy of spectral resolution between the data and the model.

Results from the phantom studies show that the probe has decreased collection with increased absorption due to attenuation of both the excitation and Raman light. Greater signal collection efficiency is observed with increased scattering. This increased signal from highly scattering samples is a result of the Raman source being confined more closely to the excitation beam, where the ball lens collects most efficiently. Similar trends for the fiber background are also observed, but the influence of scattering is more accentuated. Therefore only slight SNR changes are seen for tissues with different scattering and absorption properties if the concentration and cross-sections of the Raman scatterers are constant. In addition, further analysis of the background signal, especially from the sapphire ball lens, may result in an internal calibration for scattering and absorption properties of the samples, yielding additional information for disease diagnosis.

The single-ring probe in accordance with a preferred embodiment of the present invention can reduce the probe diameter. A smaller diameter means that the number of fibers in the probe must be reduced, thereby reducing the collection area. However, as the number of fibers are reduced, they are also brought closer to the excitation beam where the most intense Raman scattering occurs. Also, reduction of the ball lens diameter results in greater lens curvature, which leads to increased collection from the more central area of the Raman source. Thus, there is an additional efficiency curve, which relates to a tradeoff between the collection area of the Raman probe and the Raman source sampling volume. At worst, the collection efficiency may be reduced linearly with the number of fibers. Reducing to a total of 9 collection fibers results in a Raman probe of 1.5 mm total outside diameter and a collection efficiency of 1.2% which produces reasonable SNR for clinical work.

The Raman probes in accordance with a preferred embodiment of the present invention has general applicability and works well for both artery and breast. The modular nature of the design allows great flexibility with respect to the particular choice of optics for high-throughput collection so that a variety of optical elements can be used to collect the desired spatial and angular distribution from a target tissue.

Side-viewing probes can be used for alternate applications in accordance with a preferred embodiment of the present invention. For example, the use of an angled and mirrored half-ball lens, a prism, or a micro-optical paraboloidal mirror allows efficient radial collection. A tapered tip allows incorporation into needle, probes for optical breast biopsies and a slightly smaller diameter allows breast analysis through ductoscopy for detection of dysplasia. Due to the collection ability of the Raman probes in accordance with a preferred embodiment of the present invention, other potential uses include skin analysis, transcutaneous blood analyte monitoring, and gastrointestinal cancer evaluation.

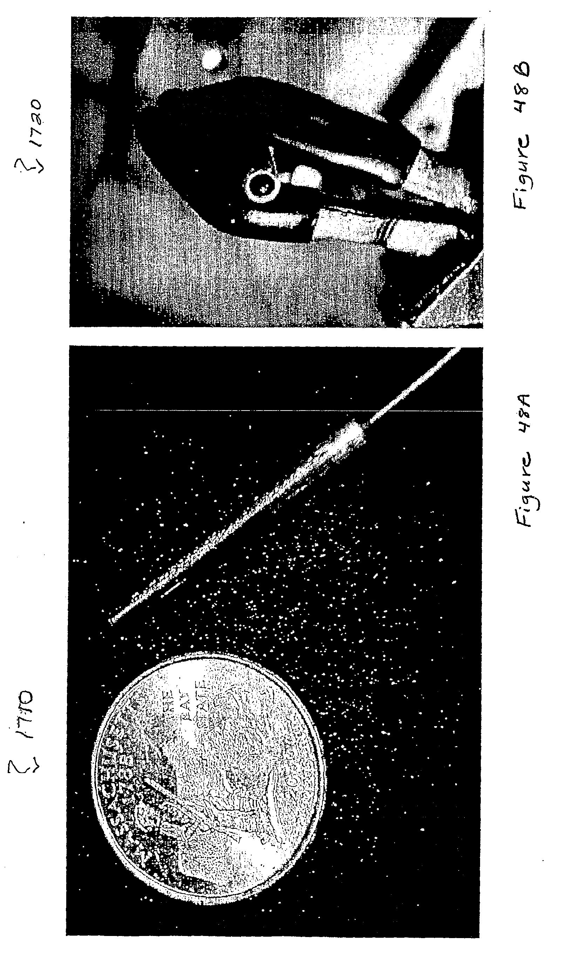

Figures 48A and 48B illustrate a clinical probe having a total diameter of less than 3 mm in accordance with a preferred embodiment of the present invention.

Preferred embodiments of the present invention have been used to collect clinical data. The laser power calibration is set with Teflon and is approximately 100 mW and ranges between 82-132 mW. The lights in the operating room were turned off similar to an angioscopy. Data was collected during peripheral vascular surgery. The spectra of the atheroma was collected during carotid endarterectomy. The spectra of an anastamosis site and of the posterior arterial wall was collected during femoral bypass surgery.

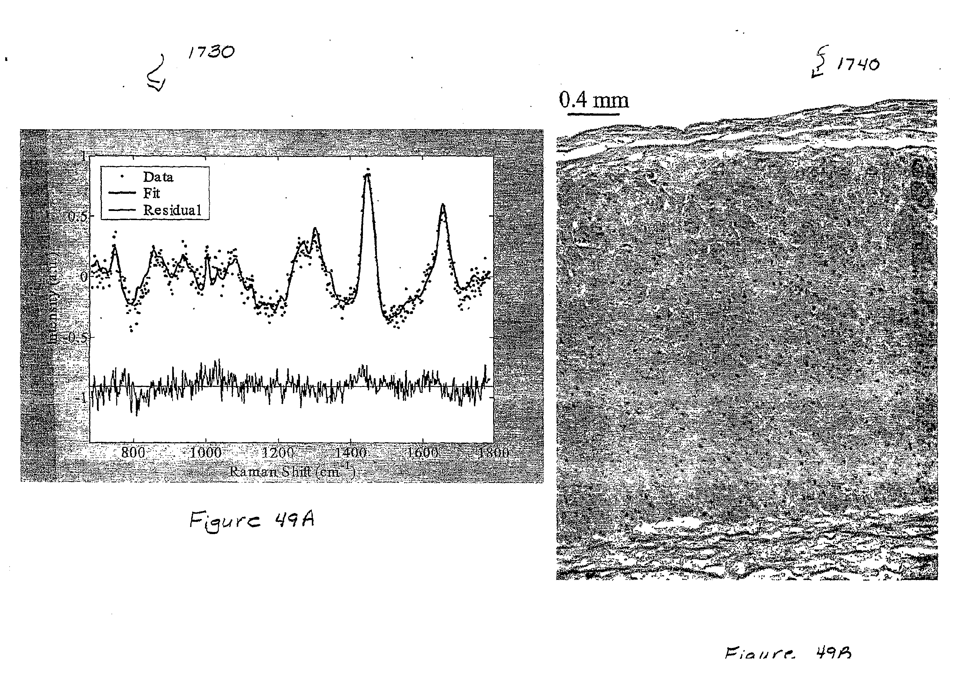

Figures 49A and 49B illustrate clinical data for the normal artery, intimal fibroplasias, wherein Figure 49A is the Raman spectra acquired and Figure 49B illustrates the corresponding histology in accordance with a preferred embodiment of the present invention. The spectra was collected for a total of 5 seconds with the probe being held normal to the arterial wall. There were 20 accumulations of 0.25 seconds each. Both 1 second and 5 second data were analyzed. All data has been integrated for 1 second.

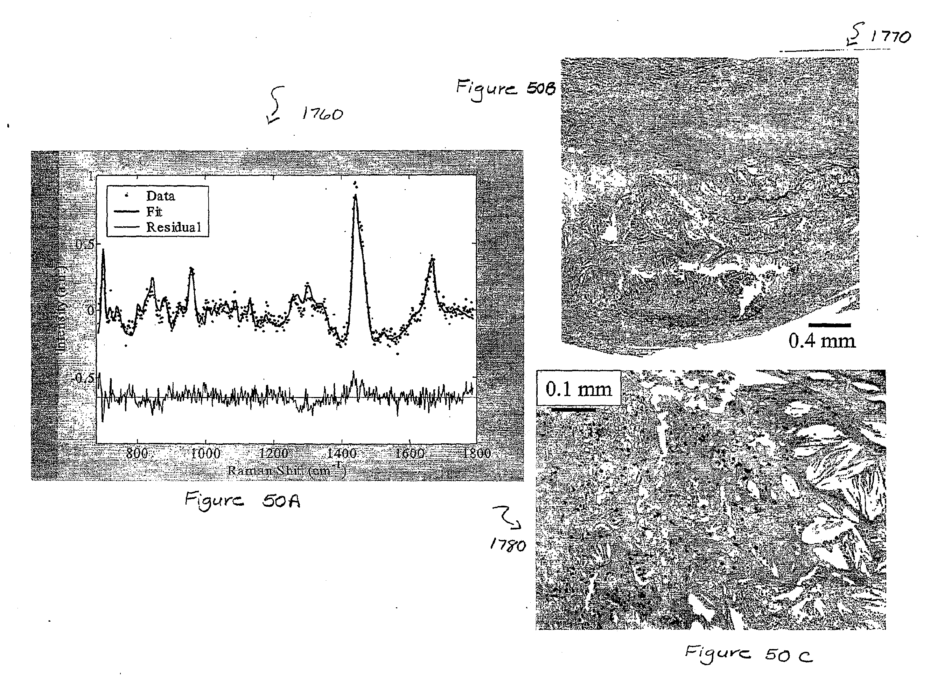

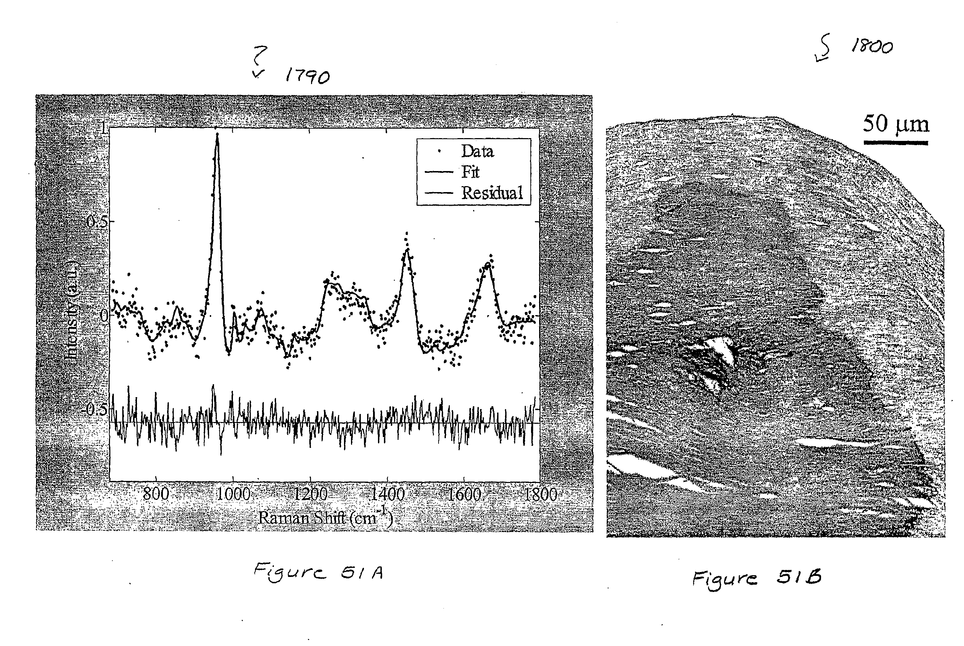

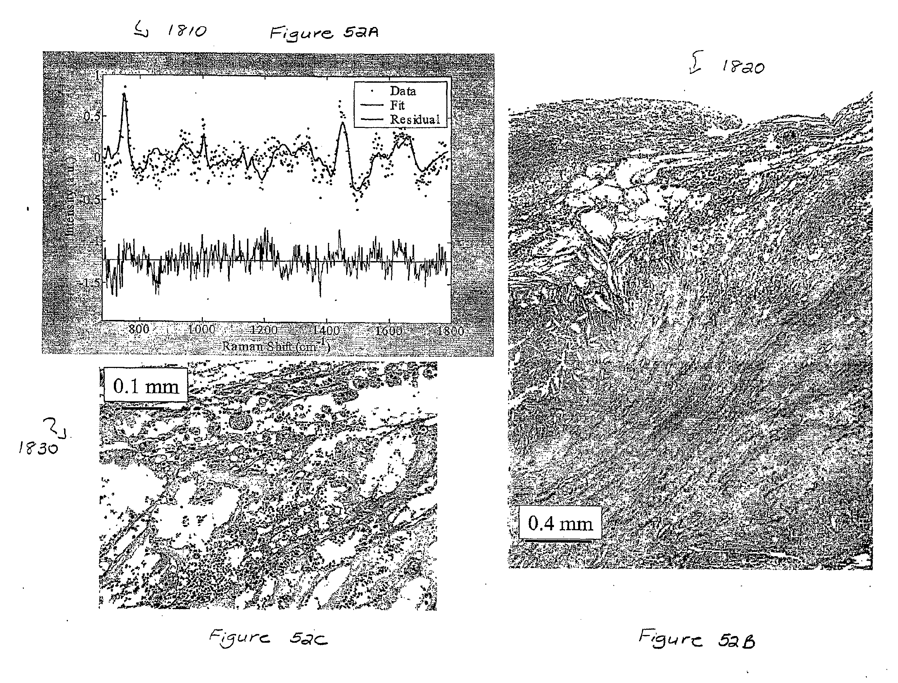

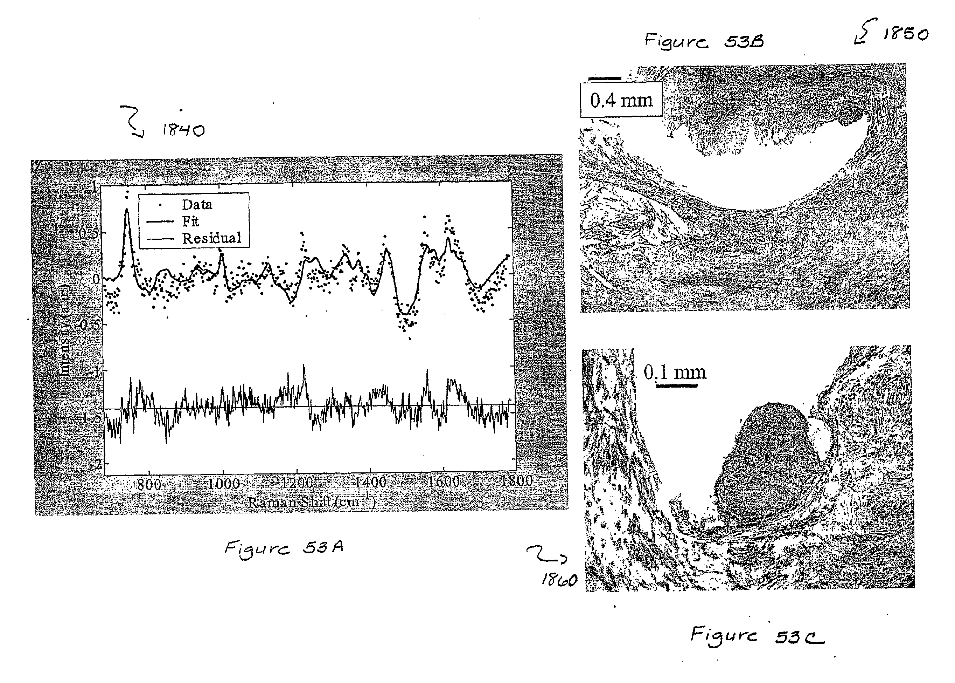

Figures 50A-50C illustrate clinical data for atheromatous plaque wherein Figure 50A illustrates the Raman spectra and Figures 50B and 50C the corresponding histology artery in accordance with a preferred embodiment of the present invention. The same methods of data analysis as for normal artery were applied to all clinical data collected. Figures 51A and 51 B illustrate clinical data acquired for calcified plaque wherein Figure 51A illustrates the Raman spectra and Figure 51B the corresponding histology in accordance with a preferred embodiment of the present invention. Figures 52A-52C illustrate clinical data acquired for ruptured plaque wherein Figure 52A illustrates the Raman spectra and Figures 52B and 52C the corresponding histology in accordance with a preferred embodiment of the present invention. Figures 53A-53C illustrate clinical data acquired for calcified plaque with thrombus wherein Figure 53A illustrates the Raman spectra and Figures 53B and 53C the corresponding histology in accordance with a preferred embodiment of the present invention.

Table 3

| Model Component | Intimal Fibroplasia | Atheromatous Plaque | Calcified Plaque | Ruptured Plaque | Thrombotic Plaque |
|---|---|---|---|---|---|
| Collagen (%) | 9 | 0 | 7 | 0 | 0 |
| Cholesterol (%) | 0 | 44 | 2 | 27 | 14 |
| Calcification (%) | 0 | 16 | 71 |  | 12 |
| Elastic Lamina (%) | 0 | 4 | 3 | 0 | 0 |
| Adventitial Fat (%) | 50 | 13 | 0 |  | 0 |

(continued)

| Model Component | Intimal Fibroplasia | Atheromatous Plaque | Calcified Plaque | Ruptured Plaque | Thrombotic Plaque |
|---|---|---|---|---|---|
| Lipid Core (%) | 13 | 16 | 0 | 0 | 0 |
| β-Carotene (%) | 0 | 7 | 4 | 23 | 13 |
| Smooth Muscle (%) | 28 | 0 | 12 | 47 | 61 |
| Hemoglobin (a.u.) | 3 | 0 | 0 | 13 | 27 |

The Table 3 illustrates the model components that were a result of the analysis of the clinical data representing the different arterial conditions.

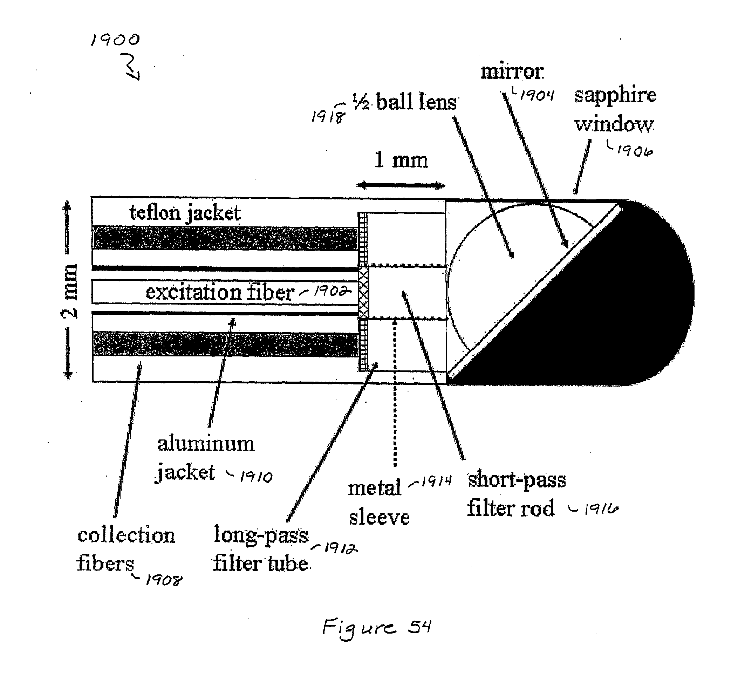

Figure 54 illustrates a side-viewing Raman probe 1900 including a single central excitation fiber 1902 in accordance with a preferred embodiment of the present invention. The buffer of the fiber is matched to the diameter of the excitation filter rod 1916 to facilitate proper fiber/filter registration and has an aluminum jacket 1910 to provide optical isolation from the collection fibers 1908. The construction of the side-viewing probe is similar to the front-viewing probe described with respect to Figures 4A-4D. The probe 1900 includes the dielectric filter module for minimizing and preferably eliminating fiber Raman background in the delivery and collection fibers and includes the rod 1916 fitted into the tube 1912 carrying the collection dielectric coating. The module is placed at the distal end of the probe between the fiber bundles and a lens system for collimating the light beams having a half ball lens 1918. The diameter of the probe is approximately 1.5 mm and has a sheath disposed around it.

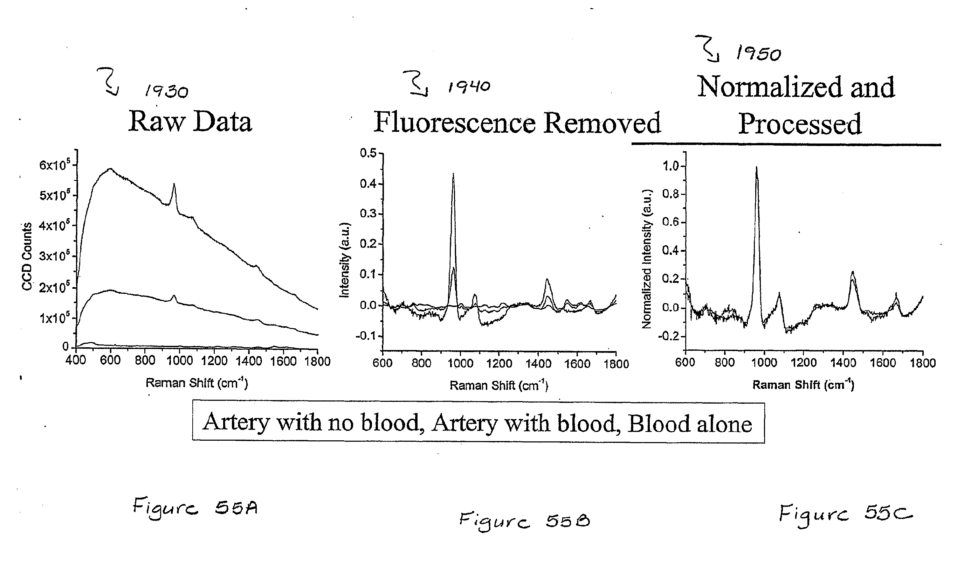

Figures 55A-55C illustrate the effects of blood on signal collection in accordance with a preferred embodiment of the present invention.

Figure 55A illustrates the raw data collected using a system having a Raman probe in accordance with a preferred embodiment of the present invention.

Figure 55B illustrates the spectra once the fluorescence is removed, while Figure 55C illustrates the spectra once the data has been normalized and processed. A calcified artery was perfused with blood to ascertain the effect of blood on signal collection. The blood spectra is less than 10% of the artery spectrum and the absorption and scattering due to the presence of blood reduces the artery signal by approximately three times.

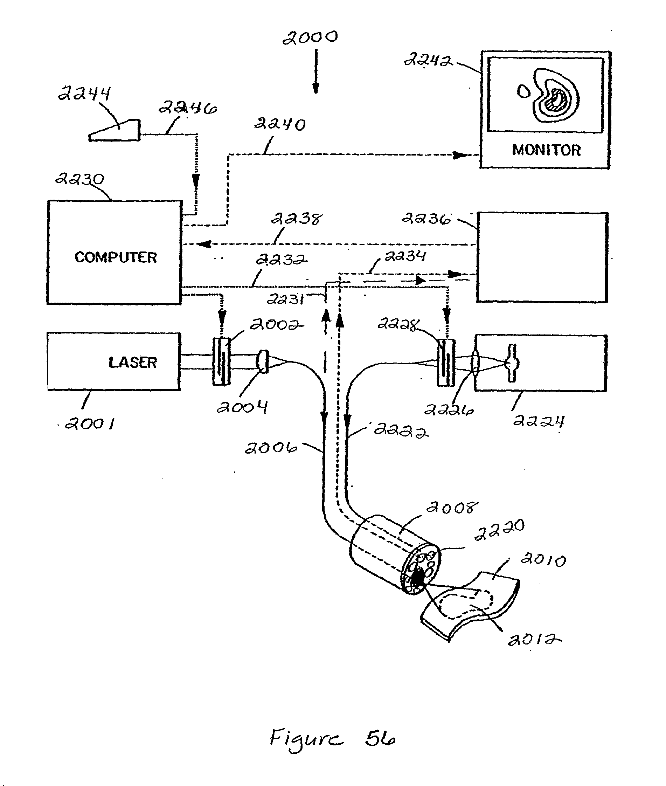

Figure 56 illustrates a schematic diagram of the system that can be used in clinical practice in accordance with a preferred embodiment of the present invention. The embodiment shown uses a laser force 2001 switched by a shutter 2002 and focused with a lens 2004 into a Raman probe 2006 inserted into a biopsy channel of an endoscope 2008 to deliver it to a tissue site 2010 so that it can illuminate the tissue over an area 2012. The collection optics 2231 provides a return of the Raman signal from the probe to the processor 2236 which is a spectrograph/CCD combination. The Raman signal and the endoscope camera are handled separately in the system 2236.

An endoscope camera 2220 obtains it white light illumination through its own fiberoptic illuminator 2222 from a broadband Xenon arc lamp 2224. A non-standard shutter 2228 under computer 2230 control 2232 can be attached. The image signal 2234 can be processed by the processor 2236 to produce a standard video signal 2238 which is digitized by a framegrabber in computer 2230. The processed image signal 2240 with its information on the state of the observed tissue is sent to monitor 2242. The entire diagnostic procedure can be initiated by a foot switch 2244 attached to the computer by a cable 2246.

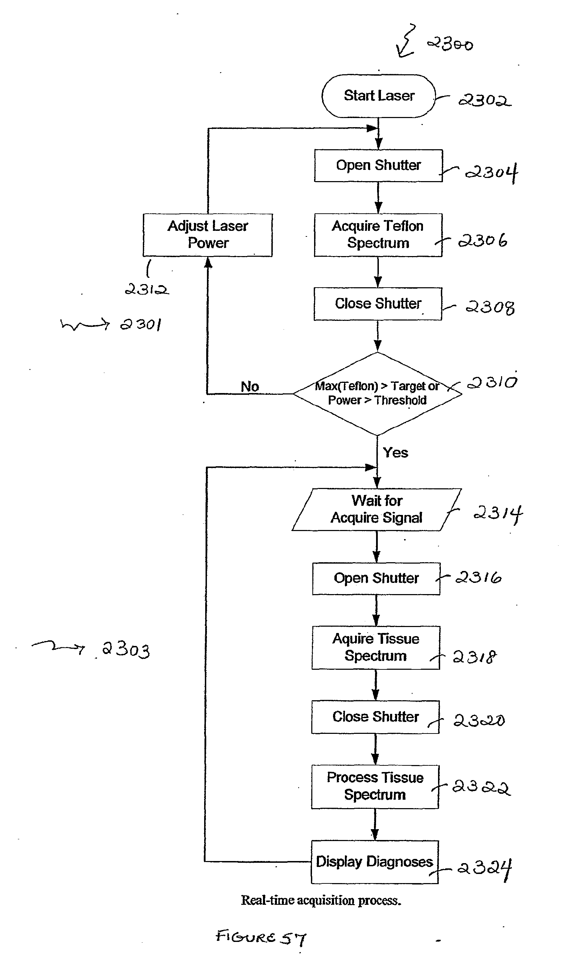

Figure 57 illustrates the flow of the methods 2300 used in acquiring data for in vivo Raman spectral diagnosis in accordance with a preferred embodiment of the present invention. The upper loop 2301 is used to ensure proper excitation laser power in a sterile operating room. Prior to the clinical procedure, calibration spectra are acquired to characterize the system performance. The spectrum of a Teflon standard is obtained to determine the expected signal with the desired excitation power. During the procedure, spectra of an identical sterilized Teflon block are taken with the sterilized Raman probe within this feedback algorithm. Automated adjustments to the laser power per step 2312 continue until the target Teflon intensity is obtained, or until a pre-determined threshold power is reached. Once the correct power is set, acquisition of tissue spectra is enabled. The laser is blocked by a shutter until data accumulation is initiated. The start of an acquisition opens the shutter per step 2316, collects the spectrum per step 2318, and closes the shutter per step 2320. Collected data is then processed and displayed in real-time along with the spectral diagnosis. The system is then ready to examine the next tissue site. Details of the data acquisition and processing are presented hereinafter.

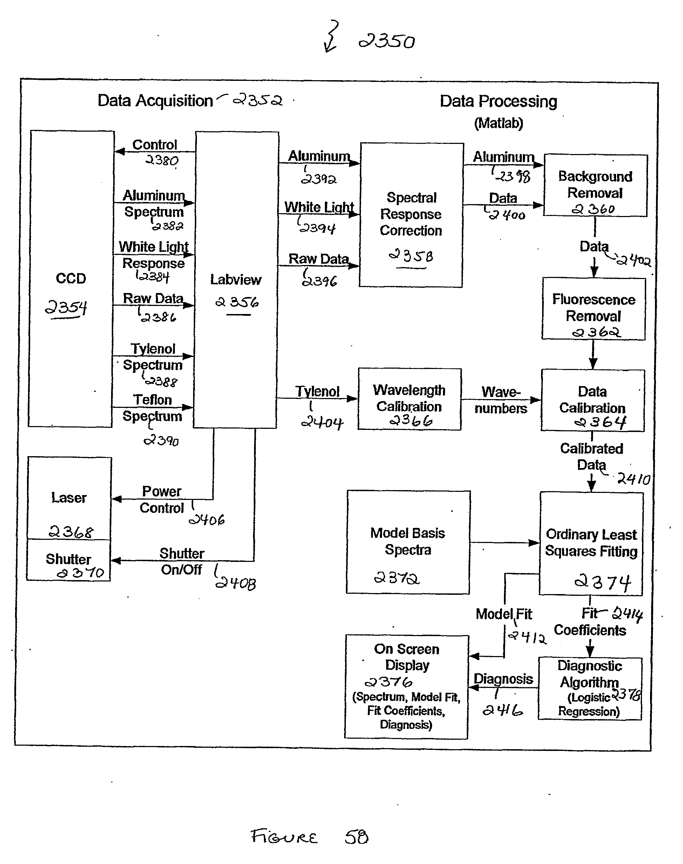

Figure 58 depicts the flow of data used in the real-time analysis Raman system in accordance with a preferred embodiment of the present invention. Control of the laser, shutter, and CCD detector are all accomplished with software such as, but not limited to, LabView. Drivers for the detector control have been written in software such as provided by

$R^3$-software, Inc. Any unfiltered probe background is characterized by collecting the excitation laser light reflected by an aluminum block. The spectral response of the system is characterized by collecting the spectrum of a calibrated white light source which is diffusely scattered by a reflectance standard ($BaSO_4$). A spectrum of 4-acetamidophenol (Tylenol) is acquired for Raman shift calibration.

The raw tissue spectra and aluminum spectrum are both corrected for system spectral response by division with the normalized white light spectrum. The remaining probe background is then removed from the tissue data by subtracting the aluminum spectrum. Tissue fluorescence is removed via a $5^{th}$ order polynomial fit, or some other means such as, for example, but not limited to, Fourier filtering, point difference derivatives, spline fitting, Savitsky-Golay derivatives, or weighted subtraction.

Characterization of the tissue is carried out by ordinary least-squares fitting of the data with an established Raman spectral model. The resultant fit coefficients are used to provide a diagnosis on the basis of the in vitro diagnostic algorithm developed with logistic regression. The processed data, model fit, and residual (data-fit) are then plotted in real-time along with the diagnosis and fit coefficients. A clinician can use the real-time data to make diagnoses and treatment decisions.

In 1999, approximately 176,000 new cases of breast cancer were diagnosed in the United States alone, 44,000 resulting in death. In the last 20 years, there has been increasing interest in using optical techniques to diagnose breast cancer in situ.

Current methodologies, such as x-ray mammography and ultrasound, look for density changes in the breast. These techniques cannot reliably distinguish between benign and malignant tumors, and thus can only be used for detecting suspicious lesions and not for diagnosis. A tissue biopsy must be performed to determine whether or not a lesion is malignant, and 70-90% of breast biopsies are found to be benign upon pathological analysis. However, instead of removing tissue for pathological analysis, it is possible to use optical techniques such as Raman spectroscopy to provide diagnostic information about a suspicious lesion in situ. Raman spectroscopy as described hereinbefore, studies the spectral sidebands generated by the light scattered from a sample illuminated with monochromatic excitation light. Each chemical present has its own unique Raman spectral signature. By inserting a fiber-optic needle device into the breast it is possible to collect Raman spectroscopic measurements from a lesion and extract chemical information almost instantaneously. Obtaining such information using a Raman needle device results in more objective and faster (real-time) diagnosis and diminished trauma to the patient compared with biopsy techniques currently in use.

In addition to Raman spectroscopy, several other optical techniques are currently being explored. These include optical tomography, fiber-optic ductoscopy and fluorescence spectroscopy. Optical tomography uses visible or near-infrared light to illuminate a point on the surface of the breast, while a detector records the diffusely reflected or transmitted light at other points. In addition to providing information about the attenuation of the light signal as it traverses the breast, scattering and absorption information can also be extracted to measure quantitatively water, lipid and oxy-/deoxyhemoglobin concentrations. The use of this information to distinguish between benign and malignant tumors is under study. Furthermore, an array of sources and detectors can be used to form a measurement cup, allowing three-dimensional imaging.

Fiber-optic ductoscopy adapts endoscopes developed to detect cancer in organs such as the colon, cervix and esophagus to the study of breast ducts. As most breast cancers and precancers start in the linings of the ducts and lobules, a very small fiberscope (less than 1 mm diameter) is introduced into the lactiferous duct through the nipple to look for intraductal abnormalities, primarily papillary lesions. The interior of the duct is illuminated and viewed via fiber-optics. The lactiferous duct, and its branches, can be observed using the device.

Fluorescence spectroscopy has been used successfully to study cancerous lesions in vivo in the esophagus, colon, bladder and oral cavity. Fluorescence spectroscopy of the breast has also been studied ex vivo, showing some promise for diagnosis, although as yet there is little understanding of the chemistry behind these results. Fluorescence-based diagnosis is limited by the number of endogenous fluorophores present in breast tissue linked with cancer (primarily collagen and NADH). In comparison, there are many more Raman-active molecules present in tissue, which have been associated with cancer development, for example, collagen, fibrinogen, DNA, calcium hydroxyapatite and various glycosaminoglycans.

Raman spectroscopy has been used for chemical analysis for many years, but only recently have researchers begun to apply it to biomedical problems. The ability to acquire Raman spectra in a clinical setting was made possible by the development of new technologies, such as compact diode lasers, CCD detectors and holographic notch filters. Each of these components contributes to the fabrication of compact, high-efficiency systems for medical diagnosis, previously unattainable.

Using 784 nm excitation to collect Raman spectra from normal, benign (fibrocystic disease) and malignant (infiltrating ductal carcinoma) breast tissue, t a shift of the 1439 $cm^{-1}$ band in normal tissue to 1450 $cm^{-1}$ has been observed in malignant tissue (due to changes in the chemical environment of the $CH_2$ bending mode). By using the area ratio of the 1654 (due to a combination of the C=C stretch and the amide I bands) and 1439 $cm^{-1}$ bands, it is possible to distinguish between malignant and normal tissue. This difference can be attributed to increased protein concentrations in the malignant sample. However, this test cannot be used to distinguish benign from malignant lesions.

Prior art studies used excisional biopsy specimens, fixed in formalin. The fixation process chemically alters the tissue, primarily cross-linking the collagen proteins, and thus affects the Raman spectral signature of the tissue. Raman spectroscopy can be used to diagnosis tissue in vivo, using tissue that has been frozen and not fixed. Raman spectra of normal, benign and malignant breast tissue samples (approximately 0.5 cm$^3$) using 830 nm excitation have been reported previously. Principal component analysis of this data permitted the differentiation of normal, benign and malignant tissue based on key spectroscopic features. However, principal component analysis does not allow the identification of the chemical or morphological origins of these spectroscopic signatures, and the data set at the time was too small for cross-validation (61 samples from 13 patients).

A clinical measurement of breast tissue using an optical fiber Raman needle probe samples a region of tissue typically 1 mm$^3$ in volume. Cancer-related changes in the breast involve subtle alterations in the biochemical and morphological composition of the tissue. These changes occur at the microscopic level. Consequently, in order to develop a diagnostic algorithm that provides insight into the microscopic state of the tissue, it is important to characterize the Raman spectral features of the individual morphological components. A model employing these microscopic spectral features as building blocks to describe the macroscopic spectrum can then be used to extract information about the composition of the tissue at the microscopic level. By identifying the specific contributors to the Raman spectrum, a robust diagnostic algorithm can be developed.

In previous embodiments, Raman spectroscopy was used for quantitative biochemical analysis of atherosclerotic lesions in aorta and coronary artery tissue in vitro. In these studies, the Raman spectrum of the tissue was modeled using a linear combination of Raman basis spectra collected from the major biochemicals present in arterial tissue. A related approach was instead to base the model on the Raman spectra of individual morphological features commonly found in artery, and to use these as the basis spectra for modeling. A similar morphological model for breast cancer diagnosis is used in preferred embodiments of the present invention.

Morphologically derived basis spectra is used primarily because the determination of which chemicals should be used to represent a morphological feature can be very difficult. For example, identifying every chemical in a complex mixture such as that found in a cell or tissue may not be possible. More importantly, those components that can be identified, such as collagen, may be present in human tissue in many different forms, each one having a slightly different Raman spectrum. The collagen found in breast tissue is, in fact, a combination of several different types of collagen, but if each type of collagen were individually included in the model, this can lead to over-fitting. By using a single, morphologically derived collagen spectrum, one then obtains a picture of that chemical component in its microenvironment within normal or diseased tissue. Finally, chemicals purified in the laboratory or bought from commercial sources are not in their natural state. For instance, proteins such as collagen may have been exposed to caustic acids or other organic solvents. All of these problems are avoided by using Raman spectra obtained from breast tissue itself. However, when necessary, synthesized or commercially available chemicals can be used.

A morphological model of human breast tissue is developed using a Raman confocal mircro-imaging system in accordance with a preferred embodiment of the present invention. This model can characterize all of the spectroscopic features observed in macroscopic samples of breast tissue, both normal and diseased. It identifies the morphological components present in breast tissue through their unique Raman spectra, and uses them as building blocks to describe the morphological features of macroscopic samples.

Samples of breast tissue were obtained from surgical biopsy specimens. The samples were snap frozen in liquid nitrogen and stored at -85˚C until spectroscopic examination. Samples were then mounted on a cryostat chuck using Histoprep (Fisher Diagnostics, Orangeburg, NY, USA) and sliced into 6-8 μm thick sections using a microtome (International Equipment, Needham Heights, MA, USA). These sections were subsequently mounted on MgF$_2$ flats (Moose Hill Enterprises, Sperryville, VA, USA), selected because of their small Raman background signal, and kept moist with phosphate buffered saline (pH 7.4).

Raman spectral images, produced using confocal Raman microspectroscopy, were collected from the unstained tissue sections and correlated with phase contrast images of the same section and serial hematoxylin and eosin-stained sections. The images were overlapped for comparison. When possible, examples of each morphological element were identified from a variety of patients and disease states. Spectra were then classified according to their morphological origin, i.e. as collagen fiber or epithelial cell, and the disease classification of the tissue sample. For example, initially extracellular matrix spectra from normal and malignant samples were kept separate. Once a library of spectra for each morphological element had been acquired, usually 60-80 spectra from 5-6 patients, they were analyzed for their degree of variation. If the spectra of a morphological element did not vary greatly or consistently, the spectra were averaged to create the morphologically derived basis spectrum used in the model. If consistent differences were observed, as was the case for the cellular components, the number of independently varying contributors was identified and used to extract independent basis spectra. In cases where single spectra had additional Raman bands when compared with other spectra in that morphological category, those spectra were removed from that category and analyzed independently to ensure that the additional spectral features could be explained by other elements in the model. If the spectral features could not be explained by the other elements of the model, a new basis spectrum was added to the model and the database of Raman micro-images was searched for similar spectral signatures. The phase contrast images and serial stained sections of all micro-images containing this new spectrum were reviewed. This methodology enabled new morphological features to be identified.

Raman spectra were also collected form macroscopic breast tissue samples and various chemicals either synthesized in the laboratory or obtained form commercial sources. Raman spectra were obtained from the following commercially available chemicals (Sigma, St. Louis, MO, USA) for use in model development and image analysis: actin (chicken gizzard), β-carotene, calcium hydroxyapatite, cholesterol, cholesterol linoleate, collagen (bovine Achilles tendon, type I), deoxyribonucleic acid (calf thymus), ribonucleic acid (calf liver), phosphatidylcholine and triolein, and also calcium oxalate, which was synthesized in the laboratory.

A schematic diagram of the experimental setup is shown in Figure 22. The same system was used for both macroscopic tissue samples and micro-imaging. The Raman excitation light (830 nm), provided by an argon ion laser-pumped Ti:sapphire laser (Coherent Innova 90/Spectra-Physics 3900S, Coherent, Santa Clara, CA, USA) traversed a band-pass filter (Kaiser Optical Systems, Ann Arbor, MI, USA) and was launched into either an aluminum holder for macroscopic tissue samples via a prism or into an epi-illuminated microscope (Zeiss Axioskop 50, Zeiss, Thomwood, NY, USA; axial resolution approximately 1 $\mu$m) for Raman micro-imaging using two mirrors. The microscope objective both focused the excitation and collected the Raman scattered light in a backscattering geometry. A dichroic beamsplitter and mirror combination redirected the Raman-scattered light from the microscope through a confocal pinhole of variable diameter to increase axial resolution. If the macroscopic assembly was used, a camera lens collected the Raman scattered light. The diameter of the light spot on a macroscopic tissue sample was approximately 1 mm, and the tissue volume sampled was typically 1 mm3. For both configurations, the light passed through a holographic notch filter (Kaiser Optical Systems) and was then focused into a 0.25m $f$/4 imaging spectrograph (Model 250IS/SM spectrograph monochromator, Chromex, Albuquerque, NM, USA) attached to a liquid nitrogen cooled CCD detector (Princeton Instruments, Princeton, NJ, USA). At the smallest confocal aperture diameter (approximately 100 $\mu$m) the spatial resolution of the microscope system was approximately 2 $\mu$m3.

The spectrograph itself had an adjustable slit and a turret, which held three gratings (Chromex) for a range of measurements. For the Raman studies, a 600 groove mm[-1] grating blazed at 1 $\mu$lm was used along with the 140 $\mu$m spectrograph entrance slit setting, providing approximately 8 cm[-1] resolution. As most biological samples do not exhibit Raman bandwidths narrower than 10 cm[-1], a spectrograph entrance slit 140 $\mu$m wide was generally used, providing maximized optical throughput (sometimes a 70 $\mu$m entrance slit was used for macroscopic measurements.)

A CCD camera (Sony, Cambridge, MA, USA) atop the microscope allowed for registration of the focused laser spot with a white light transilluminated image and recording of the image on a videotape. The microscope itself was equipped with a range of objectives, both normal and phase contrast. Typically, for Raman studies a 63x infinity-corrected water immersion objective (Zeiss Achroplan, numerical aperture 0.9) was used. Both the detector and the microscope translation stage were controlled by a computer. A complete Raman spectrum was collected at each tissue location, and spectral micro-images of the tissue were then created by moving the translation state (Prior Scientific Instruments, Cambridge, MA, USA) in a raster-scan pattern under the microscope objective. This method produced an image of typically 50 x 50 $\mu$m2, each pixel of which contained an entire Raman spectrum from 400 to 1850 cm[-1]. The step size in both the x and y directions was typically 2 $\mu$m, consistent with the spatial resolution of the confocal microscope. Spectra were usually collected for 20 s at each pixel location at a power between 50 and 100 mW, hence an entire image required approximately 3.5 h. A droplet of phosphate-buffered saline kept the tissue section moist during data collection. Experiments were also performed to test for photochemical damage to the tissue. At 220 mW, the intensity of the fluorescence signal was observed to decrease with increased exposure time (over a period of minutes), whereas the Raman signal remained unaffected. At this power photobleaching occurred, but its effect on the Raman signal was negligible. This photobleaching effect was not observed with lower excitation powers. As a result, the power was kept below 100 mW for 20-30 s exposures to reduce the effect of photobleaching during the collection of spectroscopic data.

The Raman spectra acquired underwent processing to ensure reproducibility of the data from day to day. First, they were corrected for the spectral response of the system using a tungsten light source. Then data were frequency calibrated using the known Raman line of toluene. The MgF$_2$ background spectrum was then subtracted and the broad, slowly varying fluorescence background was removed by fitting the spectrum to a fifth-order polynomial (in wavenumber), and then subtracting this polynomial from the spectrum. Also, contributions from cosmic rays were removed, if necessary, using a derivative filter.

Micro-imaging or macroscopic tissue spectroscopic data were fitted simultaneously with the model basis spectra using MATLAB's non-negative least-squares fitting algorithm or sequence of instructions (MathWorks, Natick, MA, USA). For an estimate of the number of independently varying components, principal component analysis was used (MATLAB). In order to use either least-squares fitting techniques or principal component analysis, each Raman spectrum was represented as a vector of intensity values corresponding to each wavelength.

Another key issue when using the linear model was the orthogonality of the basis spectra. The degree of orthogonality of the elements was tested using the equation

$$\frac{x^T y}{\left(x^T x\right)\left(y^T y\right)} \tag{10}$$

where x and y represent basis spectra of two morphological components, arranged as Raman intensities at each wavelength ($x^T$ is the transpose of x). A value of zero indicates that the vectors are orthogonal and a value of one means that they are identical.

The level of error in the morphological model is determined by the signal-to-noise ratio of the spectra being used. Provided that the model basis spectra are not identical within the limits of the noise (i.e. they are more orthogonal than identical spectra 'altered' by noise), the ordinary least squares method can be used to separate them. Since the basis spectra are the average of many data points, collected for as long as necessary, they are virtually noiseless. Therefore, the limiting source of error in the model is due to the noise in the data being fitted. The error in the fit contribution of a particular basis spectrum is proportional to the noise in the spectrum being fitted:

$$E = NB \tag{11}$$

where $B = P^T(PP^T)^{-1}$ is the calibration vector for the morphological basis spectrum P and N is the noise in the sample.

Raman spectroscopy was used to extract information about the morphological and chemical components present in relatively large abundance in breast tissue, reviewed here. The breast contains two types of tissue: glandular and stromal. The glandular elements consist of lobules and ducts. The lobules are dense clusters of epithelial cells, which produce and secrete milk into a system of ducts that transport the milk to the nipple. The ducts consist of an inner layer of epithelial cells surrounded by a layer of myoepithelial cells. Both layers are enclosed by a basement membrane, made primarily of collagen. The stromal elements provide the supportive network for these glandular units and include the extracellular matrix, fibroblasts, fat and blood vessels. Whereas the glandular elements of the breast are mostly cellular, there are only a small number of cells in the stroma. Most of these cells are fibroblasts, responsible for producing the extracellular matrix, a supportive network of structural proteins and carbohydrates, mainly collagen and glycosaminoglycans. Fat is the only other major morphological structure present and makes up the bulk of normal breast tissue. Sometimes crystalline deposit of $\beta$-carotene, a lipophilic precursor to vitamin A, are also present.

Many of the morphological structures in benign and malignant breast lesions are similar to those in normal breast tissue. For example, fibrosis occurs in both benign and malignant breast lesions and involves a proliferation of the stroma. Fibrotic tissue is mainly collagen in composition, like most of the extracellular matrix, with an increase in the presence of proteins such as fibrinogen and fibronectin.

However, some of the morphological features of diseased breast are different from those in normal breast tissue. For example, breast cancer most commonly originates in the lobules and ducts as a rapid proliferation of epithelial cells, associated with nuclear enlargement, pleomorphism (variation in size and shape) and hyperchromatism (darker staining), atypical mitoses and DNA aneuploidy (gain or loss of a chromosome). These morphological changes are not associated with a largescale production of new chemicals, but rather a change in the relative concentrations of chemicals that are already present in the breast. For example, the above morphological changes are associated with a change in the nucleus-to-cytoplasm ratio, a qualitative indicator of malignancy used by pathologists.

Two additional morphological features that can be observed in breast cancer are calcifications and necrosis. Calcifications are important since they are radiodense, can be detected mammographically and are often seen in association with cancer. There are two major types that have similar morphological characteristics on mammograms. Type I calcifications are calcium oxalate dihydrate crystals, whereas type II calicifications are mainly calcium hydroxyapatite but contain other calcium phosphates and possibly also calcium carbonate. Calcium oxalate crystals are more often found in benign than in malignant lesions and are thought to be the product of cellular secretions, whereas calcium hydroxyapatite deposits are found in both benign and malignant lesions and are thought to be the result of cellular degradation or necrosis (death).

With this basic knowledge of breast chemistry and architecture, and the changes induced by disease progression, it is possible to explain all of the major Raman spectral features of normal and diseased breast tissue.

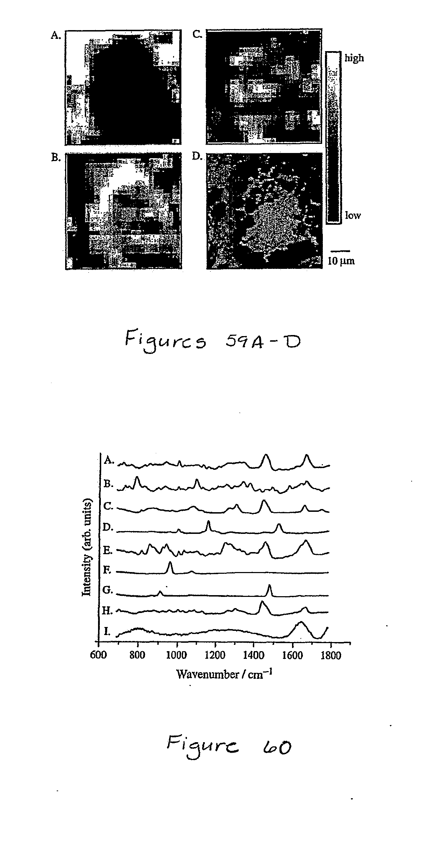

More than 60 Raman images from samples of normal, benign and malignant breast tissue were collected. Raman images of a normal breast duct are shown in Figures 59A-59B. Micro-images of collagen, cell cytoplasm and cell nucleus are produced by ordinary least-squares fitting of each data point in the image with these basis spectra. The serial stained section is shown for comparison. It is evident that the structures observed in the Raman images correlate well with the tissue architecture.

From the micro-imaging data, nine key basis spectra were identified: cell cytoplasm, cell nucleus, collagen, fat, cholesterol-like, β -carotene, calcium hydroxyapatite, calcium oxalate dihydrate and water. Some features were identified using Raman imaging, such as the cell membrane, but were not included in the model because they are not present in large quantities and have small Raman cross-sections, and therefore do not contribute significantly to macroscopic tissue spectra. Others were found to have virtually the same chemical composition as elements already in the model, and therefore could not be included as separate morphological features, as was the case for the basement membrane, which is composed mostly of collagen like the extracellular matrix. The number of spectra used to determine a model component spectrum depended on that morphological element's abundance, and also signal-to-noise ratio issues. For example, fat has an extremely strong Raman cross-section. As a result, very few fat spectra were needed from each patient to produce a clean spectrum. Both the extracellular matrix and the cellular components discussed required more spectra to increase the signal-to-noise ratio. The basis spectra used for the complete model of breast tissue are shown in Figure 60.

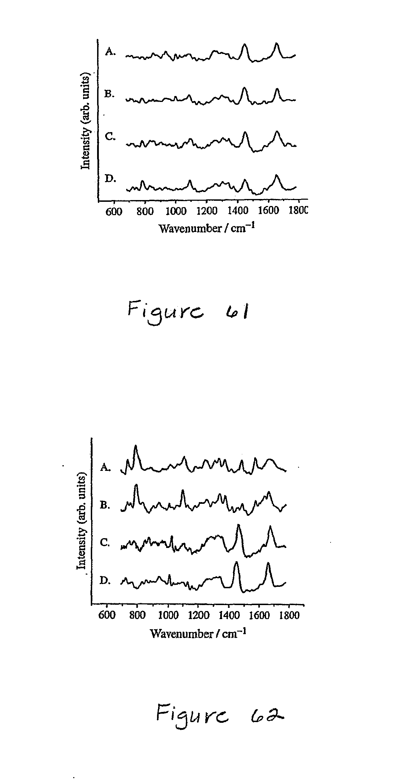

In Figure 61, spectra of a fibroblast and epithelial cells taken from normal, fibrocystic and malignant ducts are compared. Statistical analysis indicated that there are two major independently varying components, originating from the cell cytoplasm and cell nucleus. The spectrum of DNA [Figure 62A] was very similar to that of the cell nucleus [Figure 62B], although the cell nucleus spectrum also contained minor features related to RNA and histones. Similarly, the spectrum of actin [Figure 62C] was the major contributor to the cell cytoplasm spectrum [Figure 62D]. The cell cytoplasm spectrum also included minor features related to other elements found in the cytoplasm.

Because the ability to collect pure spectra from the cell cytoplasm and the cell nucleus was limited by the collection volume of the Raman confocal microscope, the two basis spectra were separated mathematically. To separate the two components, spectra of hundreds of cells (all types) from eight patients were collected using the Raman imaging system. Initially the spectra were fitted with two basis spectra, one taken from a cellular region with low nuclear content (determined by looking at the Raman signal) and one from purified DNA. Spectra with especially high DNA fit coefficients (DNA-rich), corresponding to spectra taken from the nuclear regions, were then separated from those spectra with little to no DNA (DNA-poor), collected from regions in the cell cytoplasm. The mean DNA-rich spectrum was then scaled and subtracted from the mean DNA-poor spectrum to produce a new cytoplasm-only spectrum with no DNA content. This new cytoplasm-only spectrum was then subtracted from the mean DNA-rich spectrum to remove all cytoplasm features, leaving a spectrum representative of only the nuclear material. The original data (both DNA-rich and DNA-poor) were then fitted with these two modified basis spectra. The procedure was repeated, using the two modified basis spectra rather than the purified DNA and the low nuclear content spectra, to produce the final cell cytoplasm and cell nucleus spectra. By using this iterative process, artifacts due to the inability of the purified DNA spectrum to model the nucleus (which contains DNA, RNA, histones and more) were minimized. These two basis spectra can be used to extract key diagnostic information about the cells, such as the nuclear-to-cytoplasm ratio.

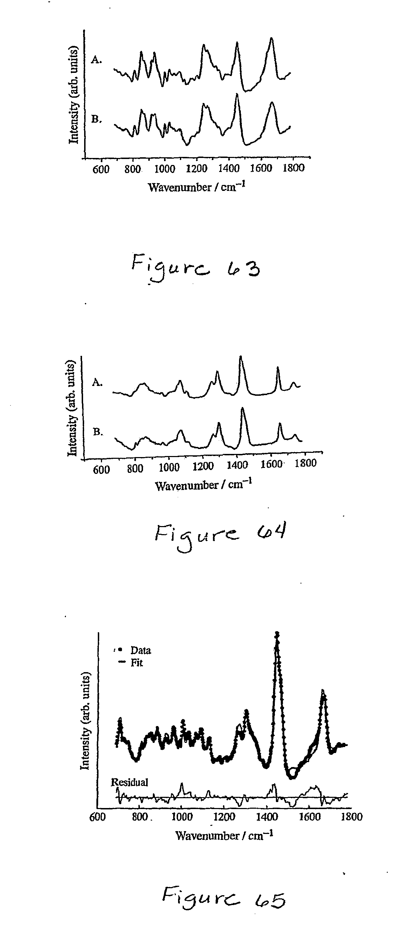

Both the extracellular matrix and the basement membrane are composed primarily of collagen. Other structural proteins, such as fibrinogen and fibronectin, and proteoglycans are also present, but in such minute quantities and with such small Raman cross-sections that they did not contribute significantly to the overall Raman spectrum. Figure 63 compares the spectra of morphologically derived collagen (mostly type I, but some types III, IV and V were also present) and that of purified collagen (type I). They are very similar, although a few minor differences can be observed in the region between 800 and 1200 cm$^{-1}$. The morphologically derived collagen spectrum was the mean of 215 spectra taken from seven patients, mostly from regions of extracellular matrix.

Fat is one of the strongest contributors to the Raman spectrum of normal breast tissue. It is present in large quantities and has a strong Raman cross-section. Its storage in humans primarily takes the form of triglycerides, especially triolein. Figure 64 compares the Raman spectrum of fat acquired from breast tissue with that of triolein, shown that, as expected, triolein was the major contributor to the spectrum. The fat spectrum included in the model and shown in Fig. 64 was the average of 28 spectra collected using data from five patients.

Necrosis within the lumen of a malignant duct or the center of a malignant tumor is essentially the product of cellular degradation. Consequently, its composition varied significantly from location to location within even a single duct. Analysis of Raman spectra from three patients indicated that the necrotic material contained fat, collagen, calcification (calcium hydroxyapatite), free cholesterol and cholesterol ester (linoleate), in addition to cellular material (both cell cytoplasm and cell nucleus). As the ratios of these elements could vary significantly, the spectrum included in the model ('cholesterol-like') represents the common elements of these spectra not represented elsewhere in the model, mainly the cholesterol components, collected from a single patient. Chemical modeling tells us that the 'cholesterol-like' spectrum has major contributions from cholesterol and cholesterol linoleate, with minor contributions from cellular material (cell cytoplasm and cell nucleus) and fat. Figure 65 shows an ordinary least-squares fit to the data using these five elements. Pure chemical spectra of cholesterol and cholesterol linoleate were not used, because there is more than one type of cholesterol ester present that cannot be individually determined. As was the case for collagen, it is best to model the tissue using a biologically derived mixture than with one or two pure components. The necrotic material was not the only element in breast tissue containing cholesterol and cholesterol esters. Cell membranes also contain both of these chemicals, although they also include other chemicals, such as phospholipids. Thus, the 'cholesterol-like' basis spectrum was found to be present in small quantities in all tissue spectra (not just malignant specimens).

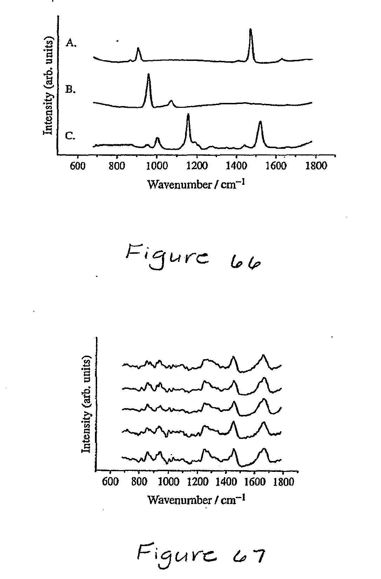

Calcium hydroxyapatite and calcium oxalate dihydrate both have very strong Raman spectra [Figures 66A and 66B]. However, they were not commonly found in the frozen breast tissue specimens, because calcifications are important for medical diagnosis and therefore tissue containing calcifications is generally not released for scientific study. Although calcifications were found in occasional frozen specimens, they were often punctate calcifications and difficult to study. For these reasons, spectra obtained from 6 μm thick deparaffinized sections of breast tissue fixed in formalin were included, in which calcifications were larger and more numerous. The fixation process altered the tissue proteins, but did not affect the relatively inert mineral deposits in the calcifications. Therefore, deparaffinized sections could be used to analyze a larger number of calcifications, identified for us by an experienced pathologist, from a range of patients and disease states.

Calcium hydroxyapatite was identified in frozen sections from three patients and from deparaffinized tissue sections in an additional 11 patients. The spectra from the frozen and deparaffinized samples were the same. The calcium hydroxyapatite basis spectrum used was acquired from a combination of these spectra. Calcium oxalate was only observed in one deparaffinized tissue section, owing to its rarity. Although its presence in breast tissue is well documented, calcium oxalate dihydrate is significantly less common than calcium hydroxyapatite in breast tissue. Therefore, calcium oxalate dihydrate was synthesized in the laboratory for incorporation into the model in accordance with a preferred embodiment of the present invention. Both calcification spectra were consistent with previously published spectra.

β -Carotene is resonance enhanced when excited with 830 nm radiation. As a result, it has an extremely strong Raman signal. Although its peaks stand out, it is often found in conjunction with fat throughout the breast. To eliminate the need for extracting the fat content from the morphologically derived β -carotene spectra, the spectrum acquired from commercially available β -carotene [Figure 66C] was used. Using the morphologically derived Raman spectra of β -carotene, it was confirmed that the commercially available sample was an accurate representation of the β - carotene found in tissue.

Although water is a weak Raman scatterer, it contributed to the spectrum through sheer volume. Water constitutes approximately 80% by weight of human tissue and is present in the phosphate-buffered saline used to keep the tissue moist. Previously, in studies of artery, it was determined that water did not contribute significantly to the Raman spectrum of human tissue. However, in these studies of breast tissue its inclusion was found to be essential for fitting the data properly. Water has a single, relatively broad Raman band centered at 1650 cm$^{-1}$. If water was not included in the model, fitting of this band by the other morphological components was incomplete.

One of the key requirements for successful morphological modeling was that there be very little inter-patient variation in the Raman spectra of a given morphological structure. By developing a model through averaging several spectra from many patients, one can ensure that the model includes the common elements of all morphological features. The extracellular matrix spectra were similar. The extracellular matrix spectrum is primarily collagen, regardless of the patient. In Figure 67, the extracellular matrix spectra from five patients are shown. The interpatient variability is similar for all morphological features. The minor differences observed were due to the close proximity of other morphological features, i.e. a small fat droplet close to a collagen fiber being studied might result in small amounts of fat being observed in addition to collagen. It was found that in the development of a Raman model of breast tissue, the lineshape variability unexplained by other basis spectra in the model was not significant.

When analyzing the orthogonality of the model components, four components were found to have values greater than 0.5 when compared with each other: cell cytoplasm, fat, collagen and cholesterol-like (Table 4).

Table 4

| | Cell cytoplasm | Cell nucleus | Collagen | Fat | β-Carotene | Cholesterol -like | Calcium hydroxy apatite | Calcium oxalate | Water |
|---|---|---|---|---|---|---|---|---|---|
| Cell cytoplasm | 1 | | | | | | | | |
| Cell Nucleus | 0.22 | 1 | | | | | | | |
| Collagen | 0.83 | 0.29 | 1 | | | | | | |
| Fat | 0.73 | 0.08 | 0.58 | 1 | | | | | |
| β-carotene | 0.27 | 0.36 | 0.35 | 0.29 | 1 | | | | |
| Cholesterol-like | 0.88 | 0.07 | 0.68 | 0.89 | 0.28 | 1 | | | |
| Calcium hydroxy apatite | 0.11 | 0.10 | 0.06 | 0.06 | 0.07 | 0.13 | 1 | | |
| Calcium oxalate | 0.11 | 0.12 | 0.06 | 0.10 | 0.10 | 0.13 | 0.00 | 1 | |
| Water | 0.26 | 0.61 | 0.46 | 0.01 | 0.16 | 0.14 | 0.20 | 0.17 | 1 |

EP 2 327 978 A2

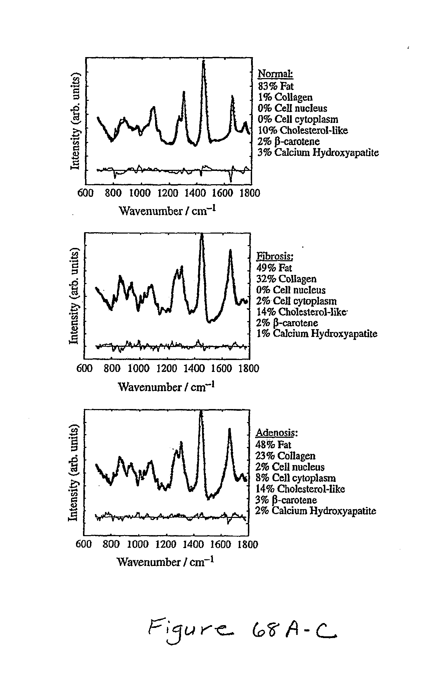

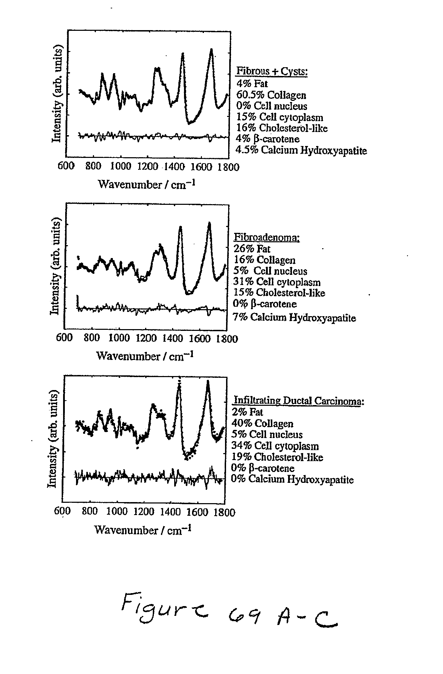

These four elements have many of the same functional groups ($CH_2$ bends, C-C stretches, etc.) Still, they were sufficiently orthogonal to be differentiated amongst when using ordinary least-squares fitting. Water and cell nucleus also overlapped considerably. Nonetheless, the key to successful fitting was to use as few elements as possible, while retaining relevant spectral information. in order to avoid over-determining the spectrum. Despite having the highest degree of overlap (0.89), the differences between the fat and cholesterol-like spectra are greater than the noise component of the data fit with the model in Figures 68A-68C and 69A-69C. Hence their incorporation in the model is reasonable. As discussed earlier, in this situation, the predictive value of the model is dependent on the signal-to-noise ratio of the data being fitted.

Using the morphological model developed here, the spectral features of a range of macroscopic tissue samples can be explained in terms of each sample's morphological composition. In Figures 68A-68C and 69A-69C, Raman spectra from normal, fibrosis, adenosis, fibrosis/cysts, fibroadenoma and infiltrating ductal carcinoma tissue samples were fitted to a linear combination of the basis spectra of the morphological model. The fit coefficients given by the model (also shown in Figures 68A-68C and 69A-69C), normalized to sum to one, represent percentage contributions of the normalized chemical and morphological basis spectra to the bulk tissue spectrum (excluding water, which varies independently). For example, the fibroadenoma and malignant samples shown in Figures 69A-69C both have a large cell cytoplasm content (31 and 34%, respectively) whereas the normal sample shown here has none. This observation reflected the greater cellularity of infiltrating carcinoma and fibroadenoma as compared with normal tissue or even the other benign lesions, which was confirmed by subsequent microscopic analysis of the samples by an experienced pathologist. The strong correlation between the model fit coefficients and the morphological changes known to accompany disease attests to the accuracy of the model. The small residuals observed in Figures 68A-68C and 69A-69C indicate that all of the major spectroscopic features are explained by the model. Similarly, small residuals were observed when 101 macroscopic tissue spectra, collected form 37 patients representing a range of disease states, were fitted with the morphological model.

By comparing Raman images with phase contrast images, and also serial stained sections of the same tissue, it is possible to monitor spectral and thus chemical changes across a tissue surface. For example, not only can one compare spectra of ductal epithelial cells found in malignant tissue with those found in normal or benign tissue, but also progressive changes in these spectra can be monitored as the transition is made between a region of infiltrating carcinoma and one unaffected by the disease process within the same tissue section. Imaging also allows the identification of chemical/morphological differences that are not made visible by phase contrast or staining. With such information, a better understanding of the disease process and how it affects both the morphology and the chemistry of the tissue can be acquired, and a morphological model developed.

Construction of a morphological model of breast tissue relied on three assumptions: first, that the Raman spectrum of a mixture was equal to the weighted linear sum of the individual components of the mixture; second, that biological morphological features, such as cells, had the same Raman spectrum from one patient to another; and third that the basis spectra included in the model were sufficiently distinct to enable their differentiation based on their Raman spectrum.

Although only some of the Raman micro-images collected were used to create the model presented, all of them were used to test the model's comprehensiveness. By using spectral data from a wide variety of patients with different pathologies, it was ensured that the model explains all the major spectral features found in breast tissue including breast cancer. Excellent model fits also confirmed that the Raman spectrum of breast tissue is equal to the weighted linear sum of the spectra of the nine morphological/chemical elements included in the model. Each of the elements included has a strong spectroscopic signature, varied little from patient to patient and, except for calcium oxalate dihydrate, was present in large quantities. Some elements were not independently considered because their Raman spectrum overlapped too much with those of other elements. This overlap was an issue for the many cell types (epithelial, fibroblast, etc.), the basement membrane and the cell membrane (which contributes weakly to the tissue spectrum but was very similar to the necrotic material spectrum). Other chemicals present in breast tissue contributed so little to the aggregate Raman spectrum that they were insignificant. For example, glycosaminoglycans are present in the extracellular matrix in large quantities but have very weak Raman cross-sections, whereas matrix metalloproteinases are present in small quantities. Neither was observed in the breast tissue Raman spectrum.

The chemical composition of the morphological features identified by Raman micro-imaging was as expected. For example, the extracellular matrix was found to be mostly collagen, whereas fat droplets were primarily triolein. The cell types examined (fibroblasts, epithelial cells from a range of normal and diseased states and inflammatory cells) were all composed of the same basic components, cholesterol and cholesterol linoleate, actin and DNA. Each cell is enclosed by a cell membrane, mainly a lipid bilayer composed of phospholipids, cholesterols, triglycerides and some proteins. Making up the bulk of the cell is the cell cytoplasm, mostly the cytosol, an aqueous solution that fills the cell. Within the cytoplasm is the cytoskeleton, composed primarily of actin filaments, which allows controlled movement and organization within the cell; RNA and proteins involved in the machinery of the cell (mostly making and regulating the production of more proteins); and various organelles. The largest of these organelles is the cell nucleus. The nucleus is rich in DNA, RNA and histones (involved in helping DNA to form a compact structure).

Depending on the function of the cell, it has varying amounts of each of these components and possibly a few additional ones. For example, fibroblasts are responsible for making and maintaining the extracellular matrix. In order to do so, they must produce collagen, fibrinogen and glycosaminoglycans within their cytoplasm and export them to the extracellular space. However, in terms of developing a Raman model of breast tissue, these components are already included in the spectrum of collagen, and therefore need not be considered independently.

Most differences among cells, either within a type or between types, can be observed in the ratio of the cell cytoplasm to the cell nucleus. It is natural that there be some variation in this ratio, but it should be exaggerated greatly in malignant cells due to the occurrence of aneuploidy and is used by pathologists to diagnose malignancy. Parameters such as the nuclear-to-cytoplasm ratio may be measurable in macroscopic tissue specimens in the future.

A number of non-cellular components were also found to be significant for modeling the Raman spectrum of breast tissue: collagen (extracellular matrix and basement membrane), fat, cholesterol-like (necrosis), calcium hydroxyapatite, calcium oxalate and β-carotene. Some of these, such as β-carotene, were significant only because they are strong Raman scatterers and therefore needed for good model fits. Others, such as 'cholesterol-like' are also key features used by pathologists to diagnose malignancy.

The proteins that contribute the most to the Raman spectrum of breast tissue are collagen and actin. Collagen is representative of the extracellular matrix while actin is found in cells. As both are proteins, their Raman spectra are very similar, especially in the 1440-1660 cm$^{-1}$ region, where researchers have previously looked for differences among normal, benign and malignant lesions. However, if one uses the information contained in these basis spectra to fit macroscopic tissue spectra in the model, it is possible to extract information about the relative quantities of cellular material (actin) and extracellular matrix (collagen) in a particular sample. This information is used to develop an algorithm based on Raman spectroscopy to diagnose breast cancer, which is explained hereinafter.

Screening mammography is an important tool in the early detection of breast carcinoma. One feature of particular diagnostic significance is the presence of microcalcifications on the mammogram. Two major types of microcalcifications are found in breast tissue. Type I deposits consist of calcium oxalate dihydrate, a birefringent colorless crystal, whereas type II deposits are composed of calcium phosphates, mainly calcium hydroxyapatite. Type II microcalcifications are typically basophilic on light microscopic examination of H&E stains and nonbirefringent.

There is no reliable way to distinguish between type I and type II microcalcifications in a clinical mammogram, but the type is thought to correlate with disease. Calcium oxalate dihydrate crystals are seen most frequently in benign ductal cysts and are rarely found in foci of carcinoma, whereas calcium phosphate deposits are most often seen in proliferative lesions, including carcinoma. This distribution is consistent with the hypothesis that type I microcalcifications are a product of secretions, whereas type II calcium deposits result from cellular degradation or necrosis.

Type II microcalcifications are estimated to occur two to three times more frequently than type I. Nonpalpable type II microcalcifications discovered by mammography frequently geographically target the location of the most important abnormality within the breast. As such, calcifications are a key component that radiologists look for in a mammogram. Several algorithms have been proposed that attempt to correlate parameters such as the shape, size, number, and roughness of mammographically detected microcalcifications with disease. However, these studies often exclude cases because of dark mammographic backgrounds, low-density calcific flecks, or densely clustered calcifications, and, thus, are limited to only certain patients and mammograms. The highest reported sensitivity and specificity for a cross-validated algorithm bashed on mammography is 71 % and 74%, respectively. Although these studies show promising results, the diagnosis of breast carcinoma using mammographically detected microcalcifications remains elusive. Whereas the mammographic appearance of microcalcifications bears some relationship to the histological type of lesion, currently diagnosis cannot be reliably made on this basis.

Because calcium deposits in breast tissue have only been categorized morphologically, significant insight may be gained by examining them using a more rigorous method. Raman spectroscopy is a technique based on the exchange of energy between light and matter. It is an inelastic scattering process in which photons incident on a sample transfer energy to or from the vibrational or rotational modes of a sample. It is a two-photon process and can be thought of as the simultaneous absorption of an incident photon and emission of a Raman photon. The difference between the energies of these two photons corresponds to the transition of a molecule from one energy level to another. Because the energy levels are unique for every molecule, Raman spectra are chemical specific. Individual bands in the Raman spectrum are characteristic of specific molecular motions. Raman spectroscopy is particularly amenable to in vivo measurements as the powers and excitation wavelengths used are nondestructive to the tissue. Raman spectroscopy is well suited to further the study of microcalcifications in breast tissue, as it can provide a definitive chemical analysis of these morphological structures in vitro. In fact, Raman spectroscopy has been used successfully to study calcium deposits in several other organs, such as the kidney and urinary tract.

Preferred embodiments of the present invention use Raman spectroscopy to highlight differences in the chemical composition or structure of microcalcifications that exist in different lesions in the breast. Results from the embodiments further the understanding of the chemical changes accompanying the onset and progression of breast disease and provide an important parameter for the diagnosis of breast disease using Raman spectroscopy.

Raman spectra were acquired from 6-$\mu$m thick deparaffinized sections of formalin-fixed, paraffin-embedded breast tissue. Because of their diagnostic importance, microcalcifications in fresh breast tissue cannon typically be spared for scientific research, and, thus, the preferred embodiment systems were confined to examining microcalcifications in fixed tissue sections. Because microcalcifications are relatively inert, the protein cross-linking effects of the fixative should be minimal. Furthermore, Raman spectral line shapes from the calcifications examined are consistent with previously published data acquired from unfixed tissue in other organ systems. Samples were mounted on $MgF_2$ flats (Moose Hill Enterprises Inc., Sperryville, VA). Each microcalcification studied was photographed using a phase contrast microscope. The phase contrast images and H&E-stained serial sections were reviewed by an experienced pathologist, who was blinded to the spectroscopy results and rendered a histological diagnosis of the disease state of regions where data were acquired. A total of 30 type I and 60 type II microcalcifications in breast biopsies from 11 patients were examined using Raman spectroscopy, 74 from histologically benign ducts and 16 from histologically malignant ducts. Histological diagnoses for benign ducts ranged from ductal epithelial hyperplasia, sclerosing adenosis, fibrocystic disease, and fibroadenoma, to Mönckeberg's arteriosclerosis, whereas all 16 of the malignant ducts were diagnosed as ductal carcinoma in situ (DCIS). No invasive carcinomas were encountered in the regions where data were acquired. All 11 of the patients were Caucasian females with a mean age of 53.4 years (range, 41-85 years) and had undergone excisional breast biopsy for suspicious microcalcifications found on mammography. These patients had no palpable breast lesions and, with the exception of the fibroadenomas, had no mass lesion of other significant findings on mammography.

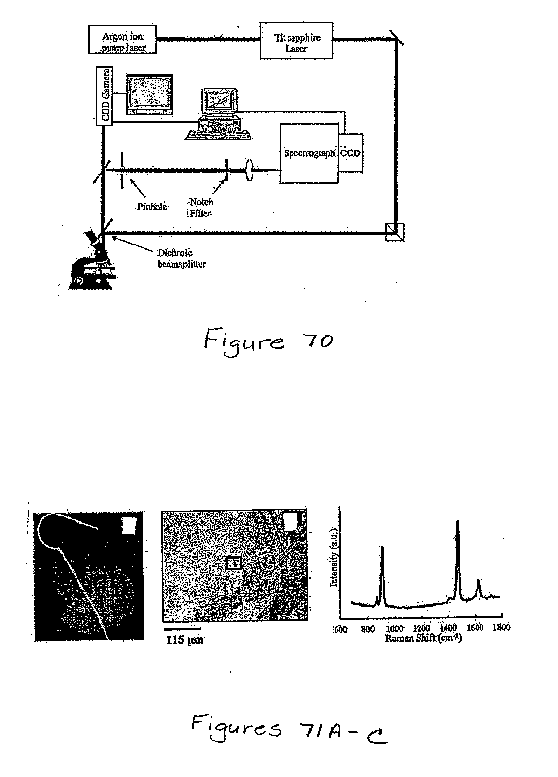

Data were acquired using the Raman microscopy system shown in Figure 70, which has been described previously. In short, Raman excitation light, 830 nm, is launched into a confocal microscope and focused to a spot size of approximately 2 $\mu$m. The objective, 63x(NA 0.9); Zeiss Achroplan), both focuses the excitation and collects the Raman scattered light in a backscattering geometry. A charge coupled device camera atop the microscope allows for registration of the focused laser spot with a while light trans-illuminated image. A dichroic beamsplitter and mirror combination redirect the Raman-scattered light to the spectrograph system where it is recorded by a deep-depletion CCD detector (Princeton Instruments, Princeton, NJ) cooled to -100˚C. The dispersion of Raman scattered light onto the CCD results in 1.6 cm$^{-1}$ per pixel. All of the Raman spectra were acquired with a 60 s integration time and a spectral resolution of 8 cm$^{-1}$. The average laser excitation power used varied between 100 and 150 mW.

All of the data processing was preformed using software algorithms such as, for example, in MATLAB 5.30. Spectra were Raman shift frequency-calibrated using known spectral lines of toluene. A fifth order polynomial was fit to the spectra by least-square minimization and subsequently subtracted to remove the slowly varying fluorescence background. Cosmic rays were removed through the use of a derivative filter.

Figure 71A is a specimen radiograph, which exhibits features indicative of the presence of microcalcifications, whereas Figure 71B displays a phase contrast image collected from a thin section of this specimen. The Raman spectrum of a type I microcalcification acquired from the deposit highlighted by a small box in Figure 71B is shown in Figure 71C. On the basis of the overall histology of this sample as well as the specific features apparent in the phase contrast image, this lesion was diagnosed as fibrocystic disease. Vibrational features characteristic of calcium oxalate dihydrate can be seen at 912 cm$^{-1}$, 1477 cm$^{-1}$, and 1632 cm$^{-1}$. These Raman features are attributed to C-C stretching, and C-O symmetric and asymmetric stretching, respectively, and are consistent with previously published Raman spectra of calcium oxalate dihydrate.

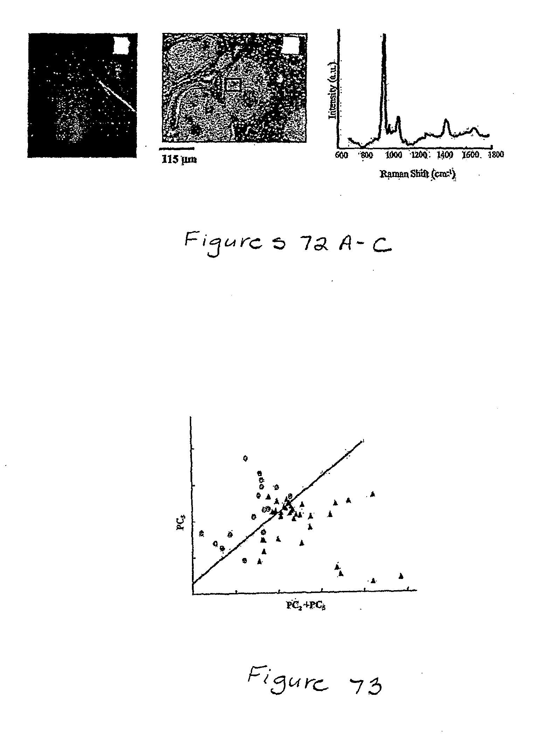

Figures 72A and 72B display a phase contrast image of a type II microcalcification in a malignant duct and the corresponding specimen radiograph. Figure 72C shows the Raman spectrum acquired from the deposit highlighted in Figure 72A by a small box. Through examination of this spectrum, it is evident that the microcalcification is not composed of pure calcium hydroxyapatite. The Raman spectrum of pure stoichiometric calcium hydroxyapatite contains four phosphate internal vibrational modes as well as bands because of the hydroxyl ion stretching and librational modes. Two of the phosphate vibrational modes are out of the spectral range chosen to examine as well as both of the hydroxyl ion modes. The large band at 960 cm$^{-1}$ is the $v_1(PO_4)$ totally symmetric stretching mode of the "free" tetrahedral phosphate ion. Another phosphate $v_1$ mode occurs at 948 cm$^{-1}$ but is obscured by the broad phosphate stretching mode at 960 cm$^{-1}$. Overlapping Raman structure resulting from five $v_3(PO_4)$ bands can be seen between 1028 cm$^{-1}$ and 1061 cm$^{-1}$. The sixth $v_3(PO_4)$ mode appears at 1075 cm$^{-1}$. The phosphate features present are consistent with Raman spectra of calcium hydroxyapatite published previously. In addition to the phosphate peaks resulting from calcium hydroxyapatite there are several other vibrational modes present in this spectrum. Protein contributions can be seen at 1445 cm$^{-1}$ and 1650 cm$^{-1}$. Also the sharp peak present at 1004 cm$^{-1}$ can be attributed to phenylalanine. Small contributions from lipid are manifest as a C-C stretch and C-H ($CH_2$) bend at 1130 cm$^{-1}$ and 1300 cm$^{-1}$, respectively.

Initially, data acquired from type I and type II microcalcifications were separated based on their Raman spectra. The presence or absence of vibrational intensity at specific wavenumbers was used to distinguish between type I and type II microcalcifications. Spectra containing large peaks at 912 cm$^{-1}$, and 1477 cm$^{-1}$, characteristic of calcium oxalate dihydrate, were grouped into the type I category, whereas spectra displaying intensity at 960 cm$^{-1}$, characteristic of

calcium hydroxyapatite, were grouped into the type II category. In analysis performed in a preferred embodiment, the separation into type I and type II microcalcifications was performed by visual inspection. However, an automated computer algorithm, which normalizes the spectra and distinguishes them based on an intensity value of one occurring at either 960 $cm^{-1}$, type II, of 1477 $cm^{-1}$, type I can be implemented in an alternate emobdiment. All 30 of the type I microcalcifications examined were formed in loci of fibrocystic disease and, thus, all 30 of the type I microcalcifications were diagnosed as benign. This is consistent with the fact that type I microcalcifications are formed as a product of secretions and are typically located in cystic lesions. Although type I microcalcifications have been found in malignant lesions, specifically, lobular carcinoma in situ, it is exceedingly rare.

To differentiate type II microcalcifications occurring in benign and malignant breast lesions, a multivariate statistical method of analysis called principal component analysis (PCA). Similar methods have been used to classify diseased tissue samples in several other organ systems. PCA uses the entire Raman spectrum and does not assume any knowledge about the chemical composition of the tissue. It is a chemometric technique that resolves the spectra of an entire data set into a few orthogonal principal component (PC) spectra. These PC spectra can have negative and positive components, and form a complete casis set that accurately describes all of the data (within limitations imposed by noise) if the PCs are multiplied by the proper weighting coefficients. These weighting coefficients, called scores, are analogous to chemical fractions. As a method based on factor analysis/chemometrics, PCA can recognize small spectral variations and, thus, differentiate samples based on similarities. This method of analysis is well suited for the examination of type II breast microcalcifications, as they are primarily composed of calcium hydroxyapatite with minute chemical variance because of biological impurities. PCA provides little physical information in and of itself; however, it is adept at isolating spectral trends that correlate with physical information and thereby provides a basis for development of a diagnostic algorithm. Furthermore, by comparing the line shapes of the diagnostic PC spectra with the spectra of pure chemicals, it is possible to ascribe meaning to them. More importantly, this method of analysis in accordance with a preferred embodiment provides a proof of principle that there is indeed important diagnostic information contained within the Raman spectra of type II microcalcifications.

A singular value decomposition algorithm to determine the PCs of the data set is used in a preferred embodiment. The data set was normalized to the 960 $cm^{-1}$ peak height to remove any possible intensity biases and subsequently mean centered before performing PCA to remove features common to all of the spectra thereby highlighting spectral variance. All 60 of the spectra could be accurately modeled above the noise level using nine PCs. The first 6 PCs account for greater than 97% of the total variance in the data. Next, a logistic regression, a discriminate analysis method, is used to generate a diagnostic algorithm that was used to classify the breast lesions into benign and malignant categories. Logistic regression correlates the weighting coefficients (scores) of the PCs calculated for each Raman spectrum with the diagnostic categories. Diagnoses were provided by a blinded pathologist. A leave one out cross-validation analysis was used to obtain a robust algorithm.

Fibroadenoma is a benign tumor of a completely different lineage than all of the other lesions examined. It is most closely related to phylloides tumors, the malignant counterpart of which the stroma rather than the epithelium is malignant. Furthermore, the clinician typically knows that a breast lesion is in the fibroadenoma/phyllodes tumor family based on physical examination and features other than microcalcification on mammography. As these lesions must be surgically excised for treatment, physicians would be unlikely to use a technique like Raman spectroscopy as an adjunct tool for diagnosis of fibroadenoma. For these reasons, the performance of the algorithm is assessed after excluding samples diagnosed as fibroadenoma from the analysis.

Using a combination of PCA and logistic regression, the Raman spectral signatures of type II microcalcifications were examined to determine whether or not breast disease diagnosis could be made on this basis. A high level of diagnostic accuracy was obtained with three PC scores. The significant scores are associated with $PC_2$, $PC_3$ and $PC_5$. $PC_5$ accounts for 1.0% of the total variance in the data, whereas $PC_2$ and $PC_3$ account for 8.8% and 5.2%, respectively. Using these three PCs and a leave one out cross-validation method one could predict 14 of 16 DCIS and 34 of 39 benign samples correctly. Thus, type II microcalcifications occurring in benign and malignant breast ducts could be distinguished with a sensitivity of 88% and a specificity of 87%. If all of the samples were retained, the sensitivity and specificity are only slightly degraded, maintaining a sensitivity of 88% with a drop in sensitivity to 80%. A graphic representation of the diagnostic algorithm for type II microcalcifications is shown in Figure 73. To condense the algorithm into a two-dimensional representation, $PC_5$ and $PC_2$, which both have a higher scores for benign microcalcifications, were added together to form a single axis. On the basis of this algorithm, all of the samples diagnosed as ductal epithelial hyperplasia and sclerosing adenosis, the benign conditions most commonly confused morphologically with carcinoma, were predicted correctly. Four of five type II stromal calcifications occurring in fibroadenoma were misdiagnosed, as well as two of four arterial calcifications in Mönckeberg's arteriosclerosis and three of thirteen ductal calcifications in fibrocystic disease.

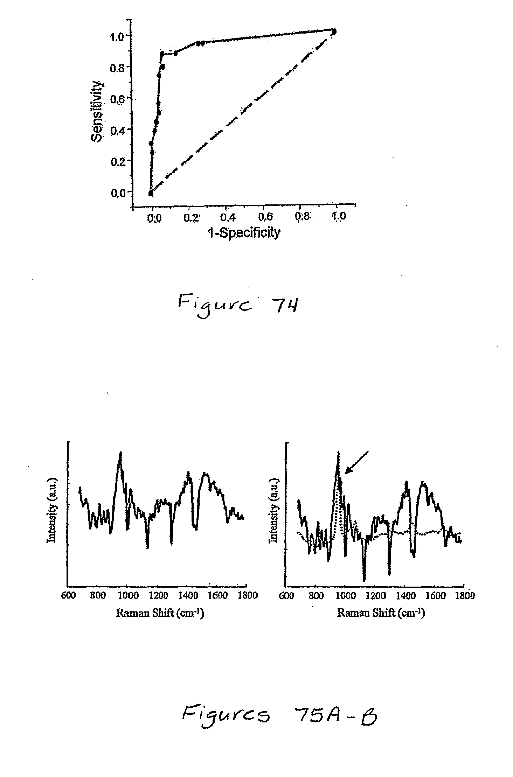

In general, only one microcalcification was studied from each lesion. However, in 2 samples, multiple microcalcifications were studied from the same lesion, and no significant differences were seen in the spectra for each given lesion. When data is combined data from both type I and type II microcalcifications an overall sensitivity of 88% and a specificity of 74% and a negative predictive value of 97% was obtained. A receiver operating characteristic (ROC) curve generated from these results is shown in Figure 74. On the basis of these in vitro results in fixed tissue, it is demonstrated that Raman spectroscopy has the potential to discriminate microcalcifications associated with benign malignant breast lesions more accurately than mammography. Additional studies performed in vitro on fresh tissue and ultimately in vivo can better evaluate the clinical utility of Raman spectroscopy as compared with X-ray mammography for the diagnosis of breast cancer.

Through examination of three diagnostic PC spectra, one can gain insight into the physical basis responsible for this discrimination. The most diagnostically significant PC spectrum was $PC_5$, shown in Figure 75A. Examination of this PC spectrum reveals a broadening of the 960 $cm^{-1}$ phosphate stretching peak. This broadening is clearly demonstrated in Figure 75B, in which $PC_5$ is overlaid with the mean spectrum from all of the type II microcalcifications. Broadening of this peak has been reported in the literature to result from the presence of calcium carbonate. In these embodiments, the application of Raman spectroscopy to carbonated apatite model systems demonstrated a broadening of the phosphate peak with increased carbonate content. The introduction of carbonate ions into the apatite structure reduces the symmetry of its unit cell. The peak broadening results from a loss of long-range translational order in the apatite structure as the carbonate content of the sample increases. The analysis found a linear relationship between the FWHM of the 960 $cm^{-1}$ phosphate stretching mode and the calcium carbonate content of the sample. Evidence that the broadening at 960 $cm^{-1}$ in $PC_5$ may result from variations in the calcium carbonate content of the microcalcifications is manifest in a peak at 1070 $cm^{-1}$ attributable to the calcium carbonate $\nu_1$ ($CO_3$) mode. However, the difficulty in interpreting PC spectra conferred by the inclusion of both positive and negative features necessitates additional investigation.

If indeed $PC_5$ accounts for variations in the amount of calcium carbonate present, then spectra that have a higher score for $PC_5$ contains a larger amount of calcium carbonate than spectra with a lower weighting coefficient. As benign spectra typically have a larger score for $PC_5$ than malignant spectra, it can be postulated that type II microcalcifications occurring in benign lesions of the breast contain a larger amount of calcium carbonate than those deposits found in DCIS. To check this hypothesis, the full width at half maximum (FWHM) was calculated for the 960 $cm^{-1}$ phosphate-stretching peak in each Raman spectrum. In accordance with the theory that type II microcalcifications formed in benign lesions have a larger calcium carbonate content, it was found that type II microcalcifications occurring in benign breast lesions had an average FWHM of 18.0 $\pm$ 0.5 $cm^{-1}$. The significance of this difference is reflected in a P of 0.03. This value was calculated based on the Wilcoxon rank-sum test, which does not assume a normal distribution of data. Furthermore, if the FWHM of those samples incorrectly diagnosed is examined an opposite trend is found. The FWHM of benign samples incorrectly diagnosed as malignant was 15.8 $\pm$ 0.5 $cm^{-1}$ whereas that of malignant samples incorrectly diagnosed as benign was 17.5 $\pm$ 0.5 $cm^{-1}$, indicating that the width of the phosphate stretching mode is a key diagnostic feature. However, although the peak height of the 1070 $cm^{-1}$ carbonate stretching mode is on average four times larger in benign samples, it does not correlate linearly with the FWHM of the 960 $cm^{-1}$ phosphate-stretching mode. This indicates that additional impurities in the apatite structure contribute to disruption of the symmetry and thereby the broadening of the 960 $cm^{-1}$ peak. These impurities are manifest in the complex vibrational structure of $PC_5$ but presently have not been identified. $PC_5$ also contains several features representative of proteins such as the $CH_2$, $CH_3$ bending mode at 1445 $cm^{-1}$, and the Amide I vibration at 1650 $cm^{-1}$. Unlike the calcium carbonate features, which have a positive intensity, the protein features are negatively directed. This indicates that the protein and carbonate contents are negatively correlated and, thus, that benign samples tend to have a lower protein content than malignant samples.

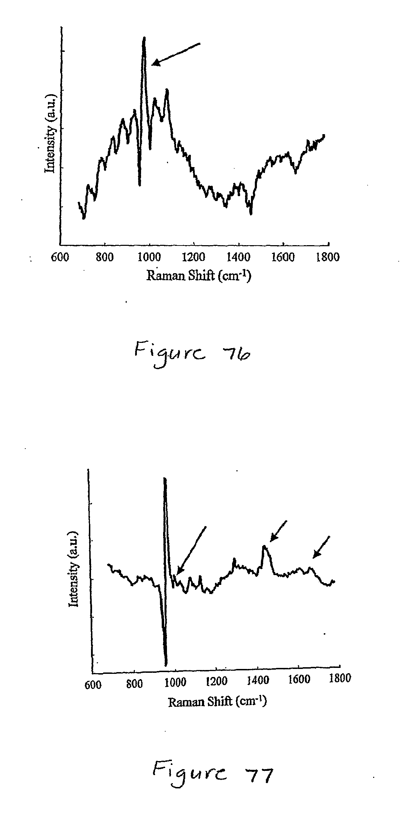

The amount of protein and calcium carbonate present in type II calcifications in benign and malignant lesions is additionally confirmed by examination of $PC_2$, shown in Figure 76. This spectrum also appears to contain positively directed calcium carbonate features, particularly at 1070 $cm^{-1}$, as well as negatively directed protein features and contributes more, on average, to the Raman spectra of microcalcifications formed in benign ducts. Additionally, $PC_2$ exhibits a large, second derivative-like feature around 960 $cm^{-1}$. This type of structure accounts for peak broadening in the data and additionally supports the hypothesis that type II microcalcifications formed in benign ducts tend to have a larger amount of calcium carbonate and, thus, more broadening of the 960 $cm^{-1}$ peak than those formed in malignant ducts.

$PC_3$ was also found to be diagnostically significant and is shown in Figure 77. However, $PC_3$ contributes more to Raman spectra acquired from type II calcifications in malignant ducts. It has positively directed protein features, thus lending additional support to the theory that microcalcifications formed in malignant ducts have a larger amount of protein than deposits in benign ducts. The amount of protein in microcalcifications in benign and malignant ducts is confirmed by monitoring the peak height of the Amide I vibration at 1650 $cm^{-1}$. The intensity of this mode is approximately one and a half times greater in type II microcalcifications formed in malignant lesions. Additionally, contributions from phenylalanine, an amino acid often found in conjunction with collagen and other proteins, can be seen in $PC_3$, at 1004 $cm^{-1}$. $PC_3$ exhibits a large first derivative-like feature at approximately 960 $cm^{-1}$. This feature accounts for a peak shift in the phosphate-stretching mode, which is positively correlated with the protein features. The presence of these protein features may explain the misdiagnosis of stromal calcifications in fibroadenomas and arterial calcifications in Mönckeberg's arteriosclerosis, which are the result of stromal or arterial degradation similar to the cellular degradation that occurs in DCIS.

Preferred embodiments including Raman probes have demonstrated the diagnostic potential of Raman spectroscopy to differentiate microcalcifications found in benign and malignant lesions. Additionally, using PCA subtle differences in the chemical composition of type II microcalcifications occurring in benign and malignant breast lesions have been discovered. One the basis of the results, one can postulate that type II microcalcifications occurring in benign lesions of the breast have both a lower protein and a higher calcium carbonate chemical content than those formed in malignant lesions. Preferred embodiments use the Raman technique in vitro in breast biopsies in which little tissue is obtained, and the lesion may not be well represented but microcalcifications are present. Further, the embodiments may be used as an in vivo adjunct to mammography to help select those patients with microcalcifications who need to go on to biopsy.

Raman spectroscopy can provide detailed qualitative and quantitative information about a sample being studied. Several approaches have been employed to acquire Raman imaging data sets. The three standard approaches are point scanning, line scanning, and direct imaging. Direct imaging involves the collection of a full image with a single spectral component. Wavelength selectivity is achieved by using either an acousto-optic or a liquid crystal tunable filter that sweeps through specified wavelength intervals capturing a frame at each. Line scanning and point scanning collect a full spectrum (usually covering Raman shifts between 400 and 1800 cm$^{-1}$ for biological media), either while imaging a line or a single point. The resultant data set from each of these approaches can be thought of as a hypercube of Raman intensity as a function of Raman shift and two spatial axes.

In addition to mapping tissue architecture, Raman imaging can be used for in situ chemical investigation of disease processes. One such example is atherosclerosis where the end product of the disease, ceroid, is defined as an autofluorescent lipid product whose chemical composition is unknown. Surface-enhanced Raman spectroscopy in conjunction with imaging can be used to study the chemical composition of live cells. In particular, the DNA and phenylalanine contents of the cells can be monitored.

A time-honored technique for creating spectral images is by examination of a specific peak height. In this approach, the intensity of a particular Raman band at each spatial location is plotted to produce an image. This method has been widely used and provides information about the spatial location of every molecule in the sample that contributes intensity to the vibrational frequency chosen. However, this approach only takes advantage of a small portion of the data available. In complex biological samples, where several distinct moieties may contribute intensity to a particular Raman band, it is necessary to incorporate all of the spectral information in order to differentiate them. This is achieved by the application of a model that utilizes the full spectrum, as is done with point and line scanning, when creating an image. The key is to compress the information into a manageable, yet still informative form. Some common data compression techniques, are principal component analysis (PCA), multivariate curve resolution (MCR), and Euclidean distance. Morphological modeling is an approach also used in preferred embodiments of the present invention.

Each one of these method rely on the basic assumption that the Raman spectrum of a mixture of chemicals can be represented as a linear combination of the mixture's component spectra. Raman images are generated by fitting basis spectra contained within the model to the Raman spectrum obtained at each position in the image. Generally, the more a basis spectrum contributes to a data spectrum, the larger the fit coefficient and the brighter that spot appears in the image of the component being examined. In the cases of PCA and MCR, basis spectra are mathematically derived, whereas for Euclidean distance and morphological modeling, basis spectra are experimentally determined.

In PCA, singular-value decomposition is used to calculate basis spectra. The first basis spectrum, or principal component, accounts for the maximum variance in the data if the data is mean-centered prior to analysis. The second basis spectrum accounts for the next most variance, and so on, until the basis spectra account only for the noise in the data. These basis spectra are created such that they are orthogonal to each other, and therefore contain no overlapping spectral information. The fit coefficients obtained when these principal components are fit to the imaging data set can be used to create a two-dimensional image. This image provides a map of how the spectral features represented by the principal components are distributed in the sample. In turn, this map can be correlated with morphological features observed through another optical technique, such as phase contrast microscopy or light microscopy with histological staining. The lineshapes of the principal components might also be correlated with the Raman spectra of known chemicals, however this is difficult as the principal components contain both negative and positive spectral features.

MCR is designed to extract basis spectra that are similar to the real Raman spectra of the chemicals present in the sample. An initial estimate of the concentrations or basis spectra present in the sample is used in a constrained, alternating least-squares optimization. New estimates for the concentrations and basis spectra are generated by iterating between least-squares solutions for basis spectra and concentrations. These equations can be solved subject to non-negativity constraints to ensure that both the basis spectra and concentrations are all positive and thus physically relevant. Optimization continues until the changes in the concentrations and basis spectra from one iteration to the next are minimal. The more complex the system, the better the initial estimates need to be to obtain meaningful solutions to these equations. Due to the high-degree of overlap in the spectral features of different components and the noise inherent in the data, MCR cannot always converge on the correct solution. However, when a solution is found, the basis spectra produced resemble the Raman spectra of the individual chemicals present in the sample. Once again, the fit coefficients of the basis spectra can be used to produce an image.

Both PCA and MCR are useful techniques when little is known about the sample a priori. They enable one to extract spectral information without knowing its chemical origin. Both Euclidean distance measurements and morphological modeling both use information about the known chemistry of a sample to create an image. Euclidean distance only requires the knowledge of a few chemicals present whereas morphological modeling requires knowledge of all of the major contributors to the sample's Raman spectrum. Despite requiring the most prior knowledge of the sample, morphological modeling produces the most easily interpretable results.

Euclidean distance classifies spectral variance in the image data from a basis spectrum, usually a pure chemical spectrum, according to the data's geometric distance. The distance is calculated using the equation:

$$\sqrt{\sum_\lambda \left(S(\lambda) - P(\lambda)\right)^2}$$ , here d is the Euclidean distance, S is the sample data, P is the pure chemical spectrum, and $\lambda$

represents the wavelengths over which the spectra are acquired. The more a spectrum in the image differs from the basis spectrum, the larger the distance.

Morphological modeling is a new technique for analyzing Raman images, which uses ordinary least-squares to fit a set of basis spectra to the data. The origin of the basis spectra is what makes this approach so useful. The basis spectra are acquired from the major morphological features found in a set representative samples using a Raman confocal microscope. By using a spectrum of a morphological feature acquired in situ, one obtains a spectrum that represents that morphological component in its chemical microenvironment. The basis spectra should account for all of the major chemicals present in the sample, but both the signal to noise of the data as well as the degree of overlap of the basis spectra must be considered to determine which basis spectra can accurately be resolved. Although basis spectra can be acquired from pure chemical compounds, morphologically-derived components are preferable as they are derived from actual samples, and are thus closer than pure chemical spectra to what is observed in situ. Sometimes, a combination of pure chemical components and morphologically-derived components produce the best result if the chemicals of interest do not occur independently within a sample. If a model is well chosen, the images produced can reveal detailed morphological and chemical structure in the sample.

Preferred embodiments apply morphological modeling to Raman images of human colonic carcinoma cells as well as human breast and artery samples. This method of morphological modeling is compared with other commonly used techniques, primarily: peak height analysis, PCA, MCR, and Euclidean distance.

Breast tissue samples were obtained from excisional biopsy specimens while artery samples were obtained from explanted hearts at the time of transplant. Once removed, the tissue was snap frozen in liquid nitrogen and stored at -80˚C. The tissue samples were then mounted on a cryostat chuck using Histoprep (Fisher Diagnostics, Orangeburg, NY) and cut into 6 $\mu$m thick sections using a cryomicrotome (International Equipment Company, Needham Heights, MA). Several consecutive sections were cut, one mounted on a $MgF_2$ slide (Moose Hill Enterprises Inc., Sperryville, VA) for Raman data acquisition and at least two others on glass slides for histological staining. The stained slides were used for pathological confirmation of features observed in the Raman maps. During measurements, the tissue was kept moist with PBS, pH=7.4. In addition to the Raman micro-images, phase contrast images of the unstained tissue were recorded via a CCD camera.

Cell studies were performed using the human colonic carcinoma cell line HT29. They were grown using high-glucose Dulbecco's modified Eagle medium (DMEM) supplemented with 10% fetal calf serum, 100 units/ml penicillin and 100 $\mu$g/ml streptomycin (all Gibco BRL products, Life Technologies, Grand Island, NY). Cells were grown to confluency at 37˚C in a humidified atmosphere of 5% $CO_2$ in air and dispersed into suspension using trypsin. Cell suspensions were placed on $MgF_2$ flats, rinsed with phosphate buffered saline (PBS, buffered at pH=7.4), and allowed to air dry. Drying of the sample was necessary in order to immobilize the cells for the entire mapping experiment. The dried samples were then rewet with PBS and Raman maps were subsequently acquired. Raman imaging microscope data collected from the dried cells were compared to data collected from viable cells still in suspension using a bulk Raman system. The spectra acquired from the dried cells were used to model the spectra obtained from the viable cells. No residual from the model fit was observed.

The Raman micro-imaging set-up used to collect the data for the images presented here was a point scan system. Raman excitation was provided by an argon ion laser-pumped Ti:sapphire laser (Coherent Innova 90/Spectra Physics 3900S, Coherent Inc., Santa Clara, CA). Typically 50-150 mW of 830 nm excitation light was focused through a microscope objective (63x Zeiss Achroplan, infinity corrected, water immersion, numerical aperture 0.9) to a spot on the sample with a diameter of less than 2 $\mu$m. The spectral resolution was approximately 8 cm$^{-1}$. Spectral maps of the tissue were created by raster scanning the translation stage (Prior Scientific Instruments Ltd., Cambridge, MA) under the microscope objective. Maps were normally acquired with a step size of 2 $\mu$m, consistent with the spatial resolution of the confocal microscope. Although data collection time depended on several user defined parameters, such as the image step size, number of steps, and spectral acquisition time, an entire Raman image was typically generated in 2-5 hours. A CCD camera atop the microscope allowed for registration of the focused laser spot with a white light trans-illuminated or phase contrast image.

All spectral data processing was performed using software, for example, MATLAB (MathWorks, Inc., Natick, MA). The data were corrected for the spectral response of the system using a tungsten light source and then frequency calibrated using the known Raman lines of toluene. Cosmic rays were removed with a derivative filter and the small background from the $MgF_2$ flat was subtracted. Data were then fit with a fourth or fifth order polynomial, which was subtracted from the spectrum in order to remove any fluorescence background. All data was peak-height normalized to one. Finally, MATLAB was used to implement the various data compression techniques: PCA, MCR, Euclidean distance, and morphological modeling. In preferred embodiments algorithms for PCA and ordinary least-squares used as the fitting algorithm for morphological modeling were sourced from software such as, but not limited to, MATLAB, while the algorithm for MCR was a part of PLS_Toolbox (Eigenvector Research, Inc, Manson, WA). The pure chemicals used for spectroscopic modeling of the HT29 cells: triolein, phosphatidyl choline, cholesterol, and DNA (calf thymus), were purchased from Sigma (St. Louis, MO).

In order to obtain improved image contrast a smoothing algorithm based on spatial filtering was applied to data of preferred embodiments. Spatial filtering relies on the assumption that adjacent pixels in a digital image contain related information. A group of pixels surrounding and including the central pixel is called a kernel. The smoothing algorithm is based on a kernel size of 3 x 3. The preferred algorithm uses a mask that weights the contributing pixels according to the reciprocal of their geometric distance from the center of the kernel. The resultant mask is:

Table 5

| 2/28 | 3/28 | 2/28 |
|------|------|------|
| 3/28 | 8/28 | 3/28 |
| 2/28 | 3/28 | 2/28 |

where each fraction represents the weight of a pixel in the kernel.

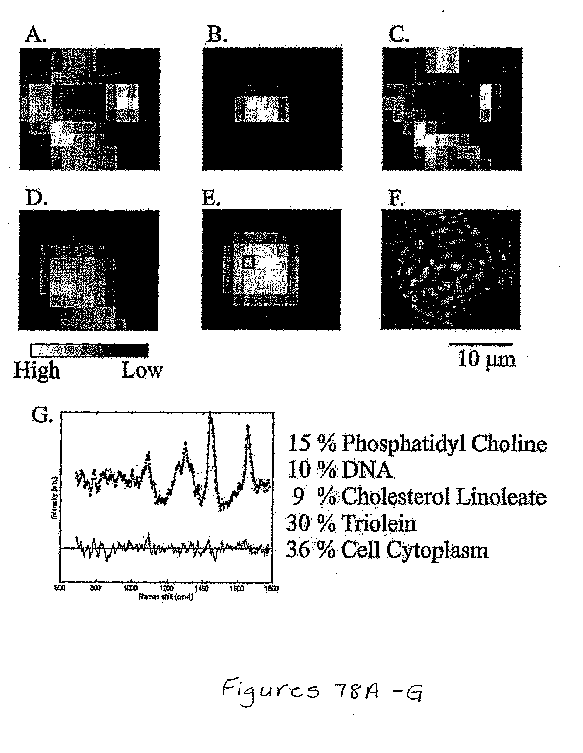

Morphological modeling is a powerful tool for collecting architectural and chemical information on a small scale. In Figures 78A-78G, features such as the cell membrane, nucleus, and cytoplasm are easily identified when spectra of human colonic carcinoma cells (HT29) are fit with the pure chemical spectra of phosphatidyl choline (A), DNA (B), cholesterol (C), triolein (D), and "cell cytoplasm" (E), a morphologically-derived spectrum developed for the breast tissue model, mostly actin). The spectrum corresponding to the voxel indicated in Figure 78E can be seen in G, along with the corresponding fit and residual. The fit contributions of the individual model elements are also shown. The spectral images agree with the phase contrast image, demonstrating that using a simple model of five basis spectra, it is possible to obtain structural and chemical information about a sample at the sub-cellular level. As the cell shown in the image is evenly bisected by the plane of focus of the confocal microscope, the cell membrane (mostly phosphatidyl choline and cholesterol) is observed as a ring structure with the cell cytoplasm and DNA contributions observed clearly as distinct features within. The average nuclear size for HT29 cells is 10 $\mu$m, consistent with the dimensions provided by the Raman image of the cell DNA content.

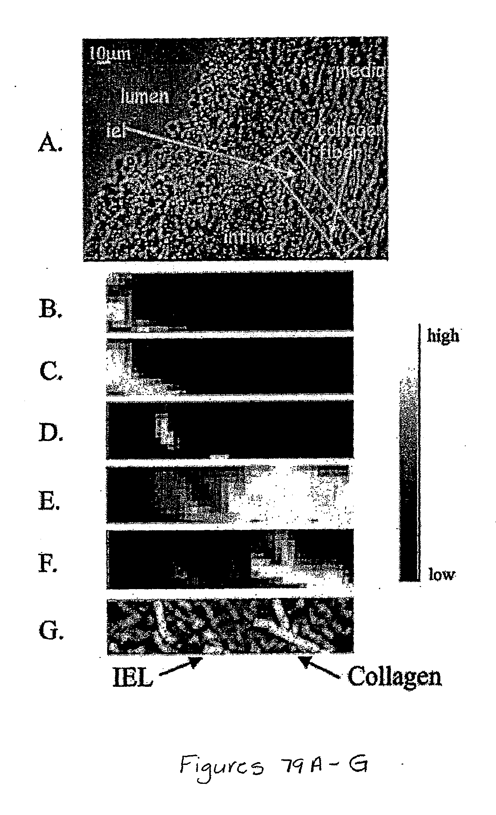

Morphological modeling can be applied to human tissue samples as well. Figures 79A-79G show phase contrast images (79A and G) of a mildly atherosclerotic artery along with Raman images depicting the distribution of some of the morphological structures (79B-F). The images clearly show that the cholesterol (79B), foam cells and necrotic core (79C) are solely confined to the intima while the smooth muscle cells (79E) are more prominently found in the media. This finding is consistent with the known architecture of atherosclerotic vessels. There is only a slight demarcation between one smooth muscle cell and the next because they are so closely spaced and even overlapping in the media. The images demonstrate the high spatial resolution of this technique and show evidence of fenestration of the elastic lamina, a process known to occur with the development of atherosclerosis. The fenestration can be observed in the Raman image of the internal elastic lamina (IEL), Figure 79D. The smooth muscle cells, shown in Figure 79E, can be seen migrating through the break in the IEL into the intima. Smooth muscle cell migration is a characteristic of atherosclerotic disease progression. In addition, one can identify a prominent collagen fiber (2F) in the media atop a diffuse connective tissue background, a feature that is difficult to fully appreciate from the phase contrast image.

Figures 80A-80G show Raman images of a normal human breast duct obtained using a morphological model created specifically to analyze breast tissue (Figures 80A-D). These images can be compared with those created by plotting the intensities of two Raman bands (Figures 80E and F) characteristic of the DNA phosphate stretch (1094 cm$^{-1}$) and the amide I band (1664 cm$^{-1}$). The morphologically based Raman images represent the regions where a particular component (cell cytoplasm (80A), cell nucleus (80B), fat (80C), or collagen (80D)) contribute strongly to the spectrum (bright regions). Histological analysis of the tissue sample showed a normal breast duct with a diameter of approximately 25 $\mu$m. A typical breast duct of this size consists of a ring of epithelial cells surrounded by a basement membrane (primarily collagen). Within and surrounding the duct is some fat. The morphological model images clearly show the architecture of the duct, whereas the peak height images produced using the Raman bands found at 1094 and 1664 $cm^{-1}$ are much less informative. Although the DNA phosphate stretch (1094 $cm^{-1}$, Figure 80E) should be found primarily in cellular regions, while the amide I band (1664 $cm^{-1}$, Figure 80F), indicative of protein, should be found mainly in collagenous regions, the images produced show neither the cellular component nor the collagen as clearly as the morphological model images do. This is because the amide I band can be found in many proteins, including those that form the cell cytoskeleton, whereas the phosphate stretch overlaps with bands present in the collagen spectrum. The inability of peak height analysis to accurately distinguish morphological features due to spectral overlap results in a much less informative image.

The Raman spectrum in Figure 80G represents a single point in the Raman image. The spectrum is a mixture of many chemical components, all of which contribute to the Raman spectrum. By fitting the spectrum with a morphological model it is possible to account for the major spectral features in the data. The residual of the fit, also shown in Figure 80G, is predominately noise, indicating that all of the information in the Raman imaging data hypercube can be represented by model-based images.

Although morphological modeling is an effective means of representing Raman images, it requires much advanced knowledge of the sample being studied. As discussed earlier, PCA, MCR, and Euclidean distance can also be used to compress the data into a manageable form and are much more effective when little is known about the system. Figures 81A-81E show a side-by-side comparison of PCA, MCR, Euclidean distance, and morphological modeling. The images, generated from the same data set, are of a sample of normal breast tissue containing three ductal units (mostly cells) surrounded by a collagen matrix. As can be seen, the images created by all four techniques are similar. The Euclidean distance images are shown as inverses (as they represent differences from input spectra rather than similarities as the other methods do) for easy comparison with the other techniques. On the left, the contributions attributable to collagen are shown, while on the right, the more subtle contributions of the cell nucleus (mostly DNA) are displayed. Both PCA (Figure 81A) and MCR (Figure 81B) were able to find seven independently varying basis spectra. The complete morphological model for breast tissue has nine basis spectra, however this includes several elements, such as microcalcifications, that are pathologically very important but that are observed only rarely in human breast tissue and not at all in this specimen.

The first two principal components, two of the spectra derived using MCR, and the collagen and cell nucleus basis spectra are shown in Figure 81E. The first principal component and the MCR spectra are similar to the collagen spectrum, the largest contributor to the image. The second principal component and the spectrum produced by MCR both contain some features of the cell nucleus spectrum (as negative peaks), but as can be seen from the image produced (Figures 81A and B, right), they are much less effective at extracting the nuclear content within the ductal units than the morphological model (Figure 81D, right). The filled-in rounded shape of the ductal units observed in Figure 81D (right) is consistent with the pathology of this tissue slice.

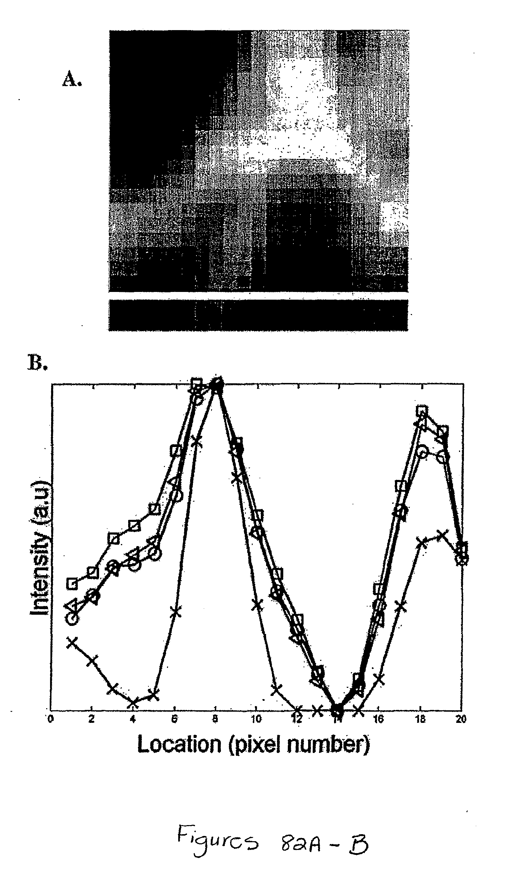

Figures 82A and 82B show the normalized fit coefficients of a particular row of the Raman images used in Figures 81A-D, left (row indicated in Figure 82A). Although PCA ($\Delta$), MCR ($\square$), Euclidean distance ($\bigcirc$), and morphological model (X) all display some form of transition from the collagenous to cellular regions of the tissue, indicated by a change in the intensity of the fit coefficient, the transition is sharpest when using the morphological model. Therefore, not only does the morphological model provide information about more of the constituents of the sample (e.g. cell nucleus), but it also produces images with a higher resolution.

The simplest method for displaying a Raman image is to plot the intensity of a particular Raman band, or alternatively the ratio of two Raman bands. This method of analysis only takes advantage of a small portion of the data and because most biological samples contain many compounds with similar spectral features, is not applicable to biological systems. Spectral overlap makes it difficult to obtain structural or chemical information about a sample from a Raman image based solely on peak height.

These imaging techniques PCA, MCR, Euclidean distance, and morphological modeling are applicable not only to Raman, but also to many other spectroscopic imaging methods, such as fluorescence. Each method has it advantages and disadvantages. Some require no (PCA) or little (MCR) prior knowledge of the sample being studied, while others require some (Euclidean distance) or complete (morphological modeling) knowledge. The quality of the images produced is usually related to how much information is known.

PCA requires the least input from the user and consequently is the best tool for studying new types of samples. PCA is used to map out regions based on their spectral variance. Due to the mathematical process by which they are created, the principal components explains all of the spectral features present in the data. However, as the principal components themselves are mathematical constructs, they can be difficult or impossible to correlate with known chemicals. Despite this drawback, information gained from PCA can be used to build more sophisticated models, such as the morphological models developed for breast and artery tissues.

While MCR is also mathematically driven, non-negativity constraints can be applied to ensure that the basis spectra developed have more identifiable features than those produced by PCA. In fact, spectra determined using MCR can be very similar to the true chemical spectra. The disadvantage of MCR is that the more complex the system being studied is, especially if there is much overlap in spectral features, the more difficult it is to perform the analysis. A skilled user can recognize when MCR has failed and adjust the parameters accordingly if the system is simple enough, but this too becomes more challenging as more component spectra are added to the sample mixture. In addition, as more curves are resolved in a complex system, noise plays a larger and larger role. Nonetheless, MCR is extremely useful for obtaining spectral lineshapes that can be used to direct further analysis of a sample.

When some, but not all, of the components of a sample are known, Euclidean distance is very effective. For example, it is not uncommon to have a sample in which the spectrum of the specific chemical being studied is known, but where the background chemicals are unknown. In this case, Euclidean distance can map the distribution of that particular chemical within the sample, unencumbered by the lack of knowledge of the background.

For detailed analysis of a system, especially for producing images with similar information content to pathology slides, morphological modeling is the best technique. However, development of a good morphological model can take time and requires much data acquisition in its own right. If the model is incomplete, the images give less accurate information. Therefore, morphological modeling is best used when extensive studies are being performed and model development is a part of the experiment. Raman spectral imaging is a powerful tool for determining chemical information in a biological specimen. The challenge is to capitalize on all of the spectral information, condensing it into an image with maximal information content. Preferred embodiments include the methods of morphological modeling and imaging approaches: PCA, MCR, and Euclidean distance.

The ability to combine Raman confocal microscopy with imaging modalities to produce images of tissue or cells is included in preferred embodiments. Embodiments of the present invention are used for monitoring sub-cellular processes in real time using Raman imaging.

Figures 78A-78G illustrate Raman images (A-E) of HT29 cells with corresponding phase contrast image (F). Raman spectra are fit with phosphatidyl choline (A), DNA (B), cholesterol linoleate (C), triolein (D), and morphologically derived cell cytoplasm (E) spectra to produce chemical maps of the cells. G: shows the spectrum ($\cdot$) acquired from within the box indicated in image E along with the corresponding fit (-) and residual (below, with zero line drawn). The fit contributions of each model element are listed to the side in accordance with a preferred embodiment of the present invention.

Figures 79A-79G illustrate phase contrast images (A and G) of a mildly atherosclerotic artery, with the internal elastic lamina (IEL) and collagen fibers highlighted in G. Also shown are the Raman images of cholesterol (B), foam cells and necrotic core (C), IEL (D), smooth muscle cells (E), and collagen (F). Key morphological features, such as the fenestration of the IEL can be observed in accordance with a preferred embodiment of the present invention.

Figures 80A-80G illustrate Raman images of normal breast duct based on ordinary least-squares fitting of morphologically derived components: cell cytoplasm (A), cell nucleus (B), fat (C), and collagen (D). Images E and F plot the intensity of single bands: the DNA phosphate (1094 cm$^{-1}$) and the protein-based amide I (1664 cm$^{-1}$) peaks, respectively. Demonstration of the fitting of a morphologically based model ($\cdot$) to the spectrum of an individual pixel (located in a region with cellular content) in a Raman image (-) is shown in G. The residual of the fit is plotted below the spectrum (with the zero line drawn) in accordance with a preferred embodiment of the present invention.

Figures 81A-81E illustrate the comparison of four different methods for analyzing Raman images of a region with multiple ductal units, separated by collagen. The images produced by the fit coefficients of the first two principal components are shown in A. B: This shows the two corresponding images produced by multivariate curve resolution (MCR). C: This shows images based on Euclidean distance, using the collagen (left) and cell nucleus (right) spectra from the morphological model. The images in D are produced using the fit coefficients produced by ordinary least-squares fitting with the morphological model, only collagen (left) and cell nucleus (right) are shown, but the complete model was used. E: shows the basis vectors used to create the images, from top to bottom: the first two principal components, the corresponding spectra produced by MCR, the morphologically derived spectrum of collagen and the morphologically derived spectrum of the cell nucleus. The last two spectra were used in both the Euclidean distance measurements and morphological modeling in accordance with a preferred embodiment of the present invention.

Figures 82A and 82B illustrate A: Raman image with third row indicated by white line and (B) heights for corresponding fit coefficients for the indicated row obtained using the four different models: PCA ($\Delta$) MCR (D), Euclideaan distance (O), and morphological model (X) in accordance with a preferred embodiment of the present invention.

In view of the wide variety of embodiments to which the principles of the present invention can be applied, it should be understood that the illustrated embodiments are exemplary only, and should not be taken as limiting the scope of the present invention. For example, the steps of the flow diagrams may be taken in sequences other than those described, and more or fewer elements may be used in the block diagrams. While various elements of the preferred embodiments have been described as being implemented in software, other embodiments in hardware or firmware implementations may alternatively be used, and vice-versa.

It will be apparent to those of ordinary skill in the art that methods involved in the system and method for determining and controlling contamination may be embodied in a computer program product that includes a computer usable medium. For example, such a computer usable medium can include a readable memory device, such as, a hard drive device, a CD-ROM, a DVD-ROM, or a computer diskette, having computer readable program code segments stored thereon. The computer readable medium can also include a communications or transmission medium, such as, a bus or a communications link, either optical, wired, or wireless having program code segments carried thereon as digital or analog data signals.

An operating environment for the systems and methods for spectroscopy of biological tissue includes a processing system with at least one high speed processing unit and a memory system. In accordance with the practices of persons skilled in the art of computer programming, the present invention is described with reference to acts and symbolic representations of operations or instructions that are performed by the processing system, unless indicated otherwise. Such acts and operations or instructions are sometimes referred to as being "computer-executed", or "processing unit executed."

It will be appreciated that the acts and symbolically represented operations or instructions include the manipulation of electrical signals by the processing unit. An electrical system with data bits causes a resulting transformation or reduction of the electrical signal representation, and the maintenance of data bits at memory locations in the memory system to thereby reconfigure or otherwise alter the processing unit's operation, as well as other processing of signals. The memory locations where data bits are maintained are physical locations that have particular electrical, magnetic, optical, or organic properties corresponding to the data bits.

Preferred embodiment of the present invention include side-viewing probes and alternatively front-viewing probes to collect Raman spectra. Preferred embodiments implement an optical design to fully utilize system throughput by characterizing the Raman distribution from tissue. The embodiments optimize collection efficiency, minimize noise and have resulted in a small diameter, highly efficient Raman probe capable of collecting high-quality data in 1 second. Performance of the embodiments have been tested through simulations and experiments with tissue models and several in vitro tissue types, demonstrating that these embodiments can advance Raman spectroscopy as a clinically viable technique. Preferred embodiments of the present invention use Raman spectroscopy to highlight differences in the chemical composition or structure of microcalcifications that exist in different lesions in the breast. Results from the embodiments further the understanding of the chemical changes accompanying the onset and progression of breast disease and provide an important parameter for the diagnosis of breast disease using Raman spectroscopy.

Preferred embodiments including Raman probes have demonstrated the diagnostic potential of Raman spectroscopy to differentiate microcalcifications found in benign and malignant lesions. Additionally, using principal component analysis (PCA) subtle differences in the chemical composition of type II microcalcifications occurring in benign and malignant breast lesions have been discovered. One the basis of the results, we postulate the type II microcalcifications occurring in benign lesions of the breast have both a lower protein and a higher calcium carbonate chemical content than those formed in malignant lesions.

The data bits may also be maintained on a computer readable medium including magnetic disks, optical disks, organic disks, and any other volatile or nonvolatile mass storage system readable by the processing unit. The computer readable medium includes cooperating or interconnected computer readable media, which exit exclusively on the processing system or is distributed among multiple interconnected processing systems that may be local or remote to the processing system.

Embodiments of the present invention may include the features of the following enumerated paragraphs.

1. A probe for measuring tissue, comprising:

a fiber optic probe having a proximal end and a distal end;
a delivery optical fiber in the probe coupled at the proximal end to a light source and having a first filter at the distal end;
a collection optical fiber in the probe that collects Raman scattered light from tissue, the collection optical fiber being coupled at the proximal end to a detector and having, a second filter at the distal end; and
an optical system at the distal end of the probe including a delivery waveguide coupled to the delivery fiber, and a collection waveguide coupled to the collection fiber.

2. The probe of Paragraph 1 wherein the delivery waveguide comprises a rod and the collection waveguide comprises a cylindrical tube, the tube being concentric about the rod.

3. The probe of Claim 1 wherein the lens comprises a ball lens optically coupled to the delivery fiber and the collection fiber.

4. The probe of Paragraph 1 further comprising a sleeve that optically isolates the delivery waveguide from the collection waveguide.

5. The probe of Paragraph 1 further comprising a first plurality of collection fibers arranged concentrically about the delivery fiber at a first radius, and a second plurality of collection fibers arranged concentrically about the delivery fiber at a second radius that is larger than the first radius.

6. The probe of Claim 1 further comprising a controller that gates a collection- time, the collection time being less than 2 seconds.

7. The probe of Paragraph 1 wherein the optical system has a length less than 10mm.

8. The probe of Paragraph 1 wherein the optical system has a length of less than 4mm.

9. The probe of Paragraph 1 wherein the light source has a wavelength longer than 750nm.

10. The probe of Paragraph 1 wherein the optical system delivers and collects light in a radial direction.

11. The probe of Paragraph 1 wherein the probe measures spectral features of cardiac tissue.

12. The probe of Paragraph 1 wherein the distal end has a diameter of 2 mm or less.

13. The probe of Paragraph 1 further comprising a light source that is optically coupled to the proximal end of the delivery optical fiber.

14. The probe of Paragraph 1 wherein the optical system comprises a refractive optical element.

15. The probe of Paragraph 1 wherein the optical system comprises a reflective optical element.

16. The probe of Paragraph 1 wherein the optical system comprises a portion of a ball lens.

17. The probe of Paragraph 1 further comprising an endoscope having a channel through which the probe is inserted.

18. A spectroscopic diagnostic system for measuring tissue comprising:

   a fiber optic probe having a proximal end, a distal end;
   a delivery optical fiber in the probe coupled at the proximal end to a light source to deliver radiation to the distal end, the delivery optical fiber having a first filter at the distal end;
   a collection optical fiber in the probe that collects Raman scattered radiation from tissue, the collection optical fiber being coupled at the proximal end to a detector system, the collection optical fiber having a second filter at the distal end; and
   an optical lens system at the distal end of the probe including a delivery waveguide coupled to the delivery optical fiber and a collection waveguide coupled to the collection optical fiber and lens system.

19. The spectroscopic diagnostic system of Paragraph 18 wherein the delivery waveguide comprises a rod and the collection waveguide comprises a cylindrical tube, the tube being concentric about the rod.

20. The .spectroscopic diagnostic system of Paragraph 18 wherein the delivery waveguide comprises a first cylindrical tube and the collection waveguide comprises a second cylindrical tube, the second cylindrical tube being concentric about the first cylindrical tube.

21. The spectroscopic diagnostic system of Paragraph 18 wherein the lens system comprises an elliptical axicon optically coupled to the delivery optical fiber and the collection optical fiber.

22. The spectroscopic diagnostic system of Paragraph 18 further comprising a sleeve that optically isolates the delivery waveguide from the collection waveguide.

23. The spectroscopic diagnostic system of Paragraph 18 further comprising a first plurality of collection fibers arranged concentrically about the delivery fiber at a first radius, and a second plurality of collection fibers arranged concentrically about the delivery fiber at a second radius that is larger than the first radius.

24. The spectroscopic diagnostic system of Paragraph 18 wherein the spectroscopic diagnostic system generates a circumferential image.

25. The spectroscopic diagnostic system of Paragraph 18 further comprising a controller that gates a collection time, the collection time being less than 2 seconds.

26. The spectroscopic diagnostic system of Paragraph 18 wherein the optical lens system has a length less than 10 mm.

27. The spectroscopic diagnostic system of Paragraph 18 wherein the optical lens systems has a length of less than 4 mm.

28. The spectroscopic diagnostic system of Paragraph 18 wherein the light source has a wavelength longer than 750 urn.

29. The spectroscopic diagnostic system of Paragraph 18 wherein the optical lens system delivers and collects radiation in a radial direction.

30. A spectroscopic catheter system for measuring comprising:

a fiber optic probe having a proximal end and a distal end;
at least one delivery optical fiber in the probe coupled at the proximal end to a light source and having a first filter at the distal end;
at least one collection optical fiber in the probe that collects Raman scattered radiation from tissue, the collection optical fiber being coupled at the proximal end to a detector and having a second filter at the distal end; and
an optical system at the distal end of the probe including a delivery waveguide coupled to the delivery optical fiber, a collection waveguide coupled to the collection optical fiber and one of a reflective and refractive optical element.

31. The spectroscopic catheter system of Paragraph 30 further comprising an inflatable balloon disposed around the fiber optic probe.

32. The spectroscopic catheter system of Paragraph 30 further comprising a channel for inflating the balloon.

33. The spectroscopic catheter system of Paragraph 30 wherein the delivery waveguide comprises a rod and the collection waveguide comprising a cylindrical tube, the tube being concentric about the rod.

34. The spectroscopic catheter system of Paragraph 30 wherein the delivery waveguide comprises a first cylindrical tube and the collection waveguide comprises a second cylindrical tube, the second cylindrical tube being concentric about the first cylindrical tube.

35. The spectroscopic catheter system of Paragraph 30 wherein the optical element comprises an elliptical axicon optically coupled to the delivery optical fiber and the collection optical fiber:

36. The spectroscopic catheter system of Paragraph 30 further comprising a sleeve that optically isolates the delivery waveguide from the collection waveguide.

37. The spectroscopic catheter system of Paragraph 30 further comprising a first plurality of collection fibers arranged concentrically about the delivery fiber at a first radius, and a second plurality of collection fibers arranged concentrically about the delivery fiber at a second radius that is larger than the first radius.

38. The spectroscopic catheter system of Paragraph 30 wherein the spectroscopic catheter system generates a circumferential image.

39. The spectroscopic catheter system of Paragraph 30 wherein the optical element comprises a ball lens optically coupled to the delivery optical fiber and the collection optical fiber.

40. The spectroscopic catheter system of Paragraph 30 further comprising a controller that gates a collection time, the collection time being less than 2 seconds.

41. The method for measuring a sample comprising:

providing a fiber optic probe having a proximal end, a distal· end, at least one delivery optical fiber in the probe coupled at the proximal end to a light source and having a first filter at the distal end, and at least one collection optical fiber in the probe that collects Raman scattered radiation from a sample, the collection optical fiber being coupled at the proximal end to a detector and having a second filter at the distal end; and

collecting light from the sample with an optical system at the distal end of the probe including a delivery waveguide coupled to the delivery optical fiber, and a collection waveguide coupled to the collection optical fiber.

42. The method of Paragraph 41 further comprising inflating a balloon disposed around the fiber optic probe.

43. The method of Paragraph 42 further comprising inflating the balloon through a channel in the probe.

44. The method of Paragraph 41 further comprising providing a delivery waveguide comprising a rod and providing a collection waveguide comprising a cylindrical tube, the tube being concentric about the rod.

45. The method of Paragraph 41 further comprising providing a first cylindrical tube and providing a collection waveguide that comprises a second cylindrical. tube, the second cylindrical tube being concentric about the first cylindrical tube.

46. The method of Paragraph 41 further comprising providing an optical element including an elliptical axicon optically coupled to the delivery optical fiber and the collection optical fiber.

47. The method of Paragraph 41 further comprising providing a sleeve that optically isolates the delivery waveguide from the collection waveguide.

48. The method of Paragraph 41 further comprising providing a first plurality of collection fibers arranged concentrically about the delivery fiber at a first radius, and a second plurality of collection fibers arranged concentrically about the delivery fiber at a second radius that is larger than the first radius.

49. The method of Paragraph 41 further comprising generating a circumferential Image.

50. The method of Paragraph 41 further comprising transmitting light with a ball lens that is optically coupled to the delivery optical fiber and the collection optical fiber.

51. The method of Paragraph 41 further comprising controlling a collection time, the collection time being less than 2 seconds.

52. The method of Paragraph 41 further comprising rotating the distal end of the probe to direct light radially in the plurality of directions.

53. The method of Paragraph 41 further comprising a method of processing Raman data from tissue.

54. The method of Paragraph 53 further comprising processing the data to diagnose cancerous tissue.

55. The method of Paragraph 41 further comprising performing real-time in vivo analysis of spectral data.

56. The method of Paragraph 41 further comprising detecting an arterial fibrous cap having a thickness of less than 65 microns.

57. The method of Paragraph 41 further comprising detecting a lipid pool, inflammatory cells, foam cells or a thrombosis.

58. The method of Paragraph 41 further comprising detecting with a probe having a diameter of 1.5 mm or less.

59. The method of Paragraph 41 further comprising inserting the probe into a cavity or artery, and rotating the probe while withdrawing the probe to scan the cavity or artery.

60. The method of Paragraph 41 further comprising diagnosing breast tissue.

61. The method of Paragraph 41 further comprising inserting the probe through a needle.

62. The method of Paragraph 41 further comprising providing a half ball lens on a mirror at the distal end of the probe.

63. A microscope system for measuring tissue, comprising:

a delivery path coupled at a proximal end to a light source and having a first filter;
a collection path that collects Raman scattered light from tissue, the collection path being coupled at the proximal end to a detector system and having a seconds filter, the detector system including a dispensing element and a detector, and
a data processor that processes Raman spectral data from the detector system.

64. The system of Paragraph 63 further comprising a charge coupled device sensor.

65. The system of Paragraph 63 wherein the data processor determines the presence of a plurality of tissue components.

66. The system of Paragraph 63 further comprising a CCD camera.

67. The system of Paragraph 63 further comprising a controller that controls a laser light source, a shutter and the detector.

68. The system of Paragraph 41 further comprising detecting Raman signals in a range of 400-2000 cm$^{-1}$.

The claims should not be read as limited to the described order or elements unless stated to that effect. Therefore, all embodiments that come within the scope and spirit of the following claims and equivalents thereto are claimed as the invention.

**Claims**

1.  A probe for measuring tissue comprising:

a fiber optic probe having a proximal end and a distal end;
a delivery optical fiber in the probe that delivers light onto tissue;
a collection optical fiber in the probe that collects Raman scattered radiation from tissue; and
an optical system on the distal end of the probe, the optical system being optically coupled to a distal end of the delivery optical fiber and a distal end of the collection optical fiber.

2.  The probe of claim 1 wherein the proximal end of the delivery optical fiber is coupled to a light source, and, optionally, wherein the light source has a wavelength longer than 750nm.

3.  The probe of claim 1 further comprising a delivery waveguide positioned between a distal end of the delivery optical fiber and at least one of a refractory or reflective optical elements.

4.  The probe of claim 3 further comprising a collection waveguide positioned between a distal end of the collection optical fiber and at least one of a refractory or reflective optical element, and, optionally, any of:

a) wherein the delivery waveguide comprises a rod and the collection waveguide comprises a cylindrical tube, the tube being concentric about the rod;
b) wherein the delivery waveguide comprises a first cylindrical tube and the collection waveguide comprises a second cylindrical tube, the second cylindrical tube being concentric about the first cylindrical tube;
c) wherein the lens system comprises an elliptical axicon optically coupled to the delivery optical fiber and the collection optical fiber; and

d) further comprising a sleeve that optically isolates the delivery waveguide from the collection waveguide.

**5.** The probe of claim 1 wherein the probe comprises a plurality of collection fibers concentrically arranged around a probe axis, and, optionally, wherein the collection fibers extend concentrically around a central lumen.

**6.** The probe of claim 1 further comprising a filter module at the distal end of the probe.

**7.** The probe of claim 6 and any of:

a) wherein the filter module comprises a rod within a tube;
b) wherein the filter module comprises a plurality of thin film layers;
c) wherein the filter module defines a spacing between the distal ends of the optical fibers and a distal optical element of the optical system, in which case, further optionally, wherein the spacing is 1 mm.

**8.** The probe of claim 7 wherein the filter module comprises a first filter that filters light exiting the delivery optical fiber and a second filter that filters light entering the collection optical fiber, and, optionally, either:

a) wherein the first filter and the second filter are positioned on one plane face of the filter module, or
b) further comprising a plurality of collection fibers that receive light through the second filter.

**9.** The probe of claim 6 wherein the filter module has a delivery piece and a collection piece, and, optionally, wherein the delivery piece is separated from the collection piece by a sleeve.

**10.** The probe of claim 1 wherein the optical system comprises at least a portion of a ball lens, and, optionally, wherein the optical system further comprises a reflector.

**11.** The probe of claim 1 wherein the optical system comprises a distal axicon, and, optionally, wherein the axicon has an elliptical surface that radially delivers and collects light.

**12.** The probe of claim 1 further comprising a plurality of delivery optical fibers, and, optionally, wherein the plurality of delivery optical fibers deliver light radially to different regions.

**13.** The probe of claim 1 and any of:

a) wherein the delivery optical fiber has a first filter at the distal end of the probe;
b) wherein the collection fiber has a second filter at the distal end of the probe;
c) wherein the collection optical fiber is coupled at the proximal end to a detector;
d) further comprising a first plurality of collection fibers arranged concentrically about the delivery fiber at a first radius, and a second plurality of collection fibers arranged concentrically about the delivery fiber at a second radius that is larger than the first radius;
e) further comprising a controller that gates a collection time, the collection time being less than 2 seconds;
f) further comprising a needle for breast tissue analysis;
g) the probe has a diameter less than 2mm;
h) wherein the probe further comprises a central lumen extending through the probe lumen;
i) further comprising a radially expanding element;
j) wherein the collection fiber is optically coupled to a radial reflection;
k) further comprising a parabolic lens at a distal end of the probe;
l) further comprising a central delivery fiber positioned between a plurality of collection optical fibers;
m) wherein at least a first filter or a second filter comprises a thin film formed on a surface of a filter module;
n) wherein the collection fiber has a larger numerical aperture than the delivery fiber;
o) wherein the optical system comprises a single distal lens that couples light from the delivery optical fiber to tissue and couples collected light to one or more collection optical fibers;
p) wherein the probe comprises a balloon;
q) wherein the probe directs light radially onto an inner wall of a coronary artery; and
r) wherein the probe has a plurality of collection fibers positioned concentrically around a central lumen to simultaneously collect light from different portions of a circumferential region.

**14.** A method of making a fiber optic probe comprising:

positioning a delivery optical fiber and a collection optical fiber within a tubular body;
attaching a distal collection filter and a distal delivery filter to a distal end of the probe; and
attaching a distal optical element at the distal end of the probe.

**15.** The method of claim 14 and any of:

a) further comprising forming a filter module having the distal collection filter and the distal delivery filter, in which case, optionally, any of:

i) further comprising forming the distal collection filter and the distal delivery filter on a plane face of the filter module;
ii) wherein the distal collection filter comprises one or more think film layers; and
iii) wherein the distal delivery filter comprises one or more thin films;

b) wherein the filter module has a light collection piece and a light delivery piece, in which case, optionally, any of:

i) wherein the collection piece is separated from the delivery piece by a sleeve;
ii) wherein the collection piece comprises a cylindrical tube;
iii) wherein the delivery piece comprises a rod;
iv) wherein the delivery piece comprises a tube coupled to a plurality of delivery optical fibers;
v) wherein the light collection piece couples light to a plurality of collection optical fibers
vi) wherein the collection piece comprises a collection waveguide; and
vii) wherein the delivery piece comprises a delivery waveguide.

c) wherein the distal optical element comprises a lens; and
d) wherein the distal optical element comprises a reflective surface.

Figure 1B

Figure 1A

Figure 2A

Figure 2B

Figure 2C

Figure 3

collection fibers 72

aluminum jacket 74

excitation fiber 76

long-pass filter tube 78

metal sleeve 80

short-pass filter rod 82

retaining sleeve 84

ball lens 86

1 mm

2 mm

Figure 4A

collection fibers 72

aluminum jacket 74

excitation fiber 76

long-pass filter tube 78

metal sleeve 80

short-pass filter rod 82

70

0.55

0.70

1.75 mm

Figure 4B

EP 2 327 978 A2

Figure 4D

collection fibers
102

aluminum jacket
104

excitation fiber
106

long-pass
filter tube
108

metal sleeve
110

short-pass
filter rod
102

0.55

0.70

1.75 mm

100

Figure 4C

collection fibers
102

aluminum jacket
104

excitation fiber
106

long-pass
filter tube
108

metal sleeve
110

short-pass
filter rod
112

retaining
sleeve
114

parabolic lens
116

2 mm

1 mm

Figure 5

EP 2 327 978 A2

Figure 6

EP 2 327 978 A2

Figure 7

EP 2 327 978 A2

Figure 8

EP 2 327 978 A2

250

Excitation Light Diffusing Through Tissue

Fiber face
252

Tissue
surface
254

1 mm

Figure 9

Figure 10A

Figure 10B

Figure 10C

EP 2 327 978 A2

Figure 11

EP 2 327 978 A2

Distribution of Excitation Light in Tissue

340

Figure 12

Collection Efficiency of the Raman Probe

360

Figure 13

Collection Efficiency of the Raman Probe

Figure 14

380

EP 2 327 978 A2

Figure 15

Figure 16

Figure 17

Figure 18

Figure 19

EP 2 327 978 A2

Figure 20

EP 2 327 978 A2

550

2 mm

552
Fiber bundle

554
Filters

556
Elliptical axicon

558
Central
channel

Figure 21A

Figure 21B

560

567
balloon

565

562

2 mm

552
Fiber bundle

554
Filters

556
Elliptical axicon

EP 2 327 978 A2

2 mm

552
Fiber bundle

554
Filters

556
Elliptical axicon

568

567
balloon

566
Central
channel

Figure 21C

EP 2 327 978 A2

EP 2 327 978 A2

Figure 21D

81

Figure 22

EP 2 327 978 A2

Figure 23

Figure 24A

Figure 24B

Figure 24C

Figure 25

Figure 26A

Figure 26B

Figure 27

EP 2 327 978 A2

Figure 28A

Figure 28B

Figure 29A

Figure 29B

EP 2 327 978 A2

Figure 30

Figure 31

EP 2 327 978 A2

Figure 32A

Figure 32B

Figure 33

EP 2 327 978 A2

EP 2 327 978 A2

920

Figure 34A

930

Figure 34B

Figure 35

EP 2 327 978 A2

Figure 36A

Figure 36B

Figure 36C

Figure 36D

EP 2 327 978 A2

Figure 36E

Figure 36F

EP 2 327 978 A2

Figure 36G

Figure 36H

Figure 37A

Figure 37B

Adventitial fat

triolein

~1260

Figure 37D

Smooth muscle cell

actin

~1240

Figure 37C

Figure 37E

Figure 37F

Figure 37G

Figure 37H

EP 2 327 978 A2

$$Fit = 0.348e^{-x^2/\,0.025} + 0.1134e^{-x^2/0.2} + 0.557\,4e^{-x^2}$$

Figure 38A

EP 2 327 978 A2

Figure 38B

EP 2 327 978 A2

Figure 38C

Figure 38D

Figure 38E

EP 2 327 978 A2

EP 2 327 978 A2

Figure 39 A

$$B_1(r) = 0.348e^{-r^2/0.025} + 0.113e^{-r^2/0.200} + 0.557e^{-r^2}$$

Figure 39B

Figure 39 C

1540

EP 2 327 978 A2

Figure 40B

Figure 40A

*1590*

Figure 41

1600

Figure 42

1620

Raman Probe

Figure 43

3, 1650

Figure 44

3, 1660

Figure 45

111

Figure 46 A

Figure 46B

~ 1690

Figure 47A

Figure 47B

Figure 48B

Figure 48A

*1730*

Figure 49A

0.4 mm

*1740*

Figure 49B

116

EP 2 327 978 A2

Figure 50A

Figure 50B

Figure 50 c

EP 2 327 978 A2

Figure 51A

Figure 51B

EP 2 327 978 A2

Figure 52A

Figure 52B

Figure 52C

Figure 53A

Figure 53B

$\int$ 1850

0.4 mm

0.1 mm

1860

Figure 53C

120

EP 2 327 978 A2

1840

Figure 54

EP 2 327 978 A2

3, 1930
Raw Data

3, 1940
Fluorescence Removed

3, 1950
Normalized and Processed

Artery with no blood, Artery with blood, Blood alone

Figure 55A

Figure 55B

Figure 55C

EP 2 327 978 A2

Figure 56

2300

Start Laser — 2302

↓

Open Shutter — 2304

↓

Adjust Laser Power — 2312

Acquire Teflon Spectrum — 2306

↓

2301

Close Shutter — 2308

↓

**No** ⟷ Max(Teflon) > Target or Power > Threshold — 2310

↓ **Yes**

Wait for Acquire Signal — 2314

↓

2303

Open Shutter — 2316

↓

Aquire Tissue Spectrum — 2318

↓

Close Shutter — 2320

↓

Process Tissue Spectrum — 2322

↓

Display Diagnoses — 2324

Real-time acquisition process.

FIGURE 57

124

2350

FIGURE 58

Figures 59A-D

Figure 60

EP 2 327 978 A2

Figure 61

Figure 62

127

Figure 63

Figure 64

Figure 65

Figure 66

Figure 67

Figure 68 A-C

Figure 69 A-C

EP 2 327 978 A2

Figure 70

Figures 71A-c

Figures 72 A-C

Figure 73

EP 2 327 978 A2

Figure 74

Figures 75A-B

134

Figure 76

Figure 77

Figures 78A -G

A.

B.

C.

D.

E.

F.

G.

high

low

IEL → ← Collagen

Figures 79 A - G

A.  B.

C.  D.

E.  F.

$\overline{20\ \mu m}$

High     Low

G.

1664

1094

Intensity (a.u.)

residual

500   1000   1500   2000
Raman shift (cm-1)

Figures 80A-G

Figures 81A-G

A.

B.

Location (pixel number)

Figures 82A - B

## REFERENCES CITED IN THE DESCRIPTION

**Patent documents cited in the description**

- US 17806202 A
- US 37019702 P
- US 5615673 A
- US 5304173 A
- WO 9215008 A
- WO 9629925 A

**Non-patent literature cited in the description**

- **Hendrik P. Buschman et al.** Raman microspectroscopy of human coronary atherosclerosis: Biochemical assessment of cellular and extracellular morphologic structures in-situ. *Cardiovascular Pathology,* 2001, vol. 10, 69-82
- **Hendrik P. Buschman et al.** Diagnosis of human coronary atherosclerosis by morphology-based Raman spectroscopy. *Cardiovascular Pathology,* 2001, vol. 10, 59-68